# DEMANDE DE BREVET EUROPEEN

(11) **EP 1 935 404 A1**
(43) Date de publication de la demande: **25.06.2008**
(21) Numéro de dépôt: 07119151.4
(22) Date de dépôt: 24.10.2007
(51) Int. Cl.: A61K 8/898, A61Q 5/06

(54) **Composition de coloration des fibres kératiniques comprenant un copolymère bloc polysiloxane/polyurée**

(30) Priorité: 25.10.2006 FR 0654535
(71) Demandeur: L'Oréal, 75008 Paris (FR)
(72) Inventeur: Brun, Gaëlle, 75015 Paris (FR); Vrignaud, Sabine, 93210 La-Plaine-Saint-Denis (FR)
(74) Mandataire: Fevrier, Murielle Françoise E.

(57) **Abrégé**

La présente invention a pour objet une composition comprenant au moins un copolymère bloc non ionique polysiloxane/polyurée, au moins une espèce coloré ou colorante, au moins un solvant organique non siliconé volatil.

Une telle composition permet notamment d'obtenir une coloration particulièrement rémanente aux agents exterieurs.

## Description

La présente invention a pour objet une composition cosmétique pour la coloration des fibres kératiniques mettant en oeuvre un copolymère bloc polysiloxane/polyurée.

Dans le domaine de la coloration des fibres kératiniques, il est déjà connu de colorer des fibres kératiniques par différentes techniques à partir de colorants directs ou de pigments pour des colorations non permanente ou de précurseurs de colorant pour des colorations permanentes.

La coloration non permanente ou coloration directe consiste à teindre les fibres kératiniques avec des compositions tinctoriales contenant des colorants directs. Ces colorants sont des molécules colorées et colorantes ayant une affinité pour les fibres kératiniques. Ils sont appliqués sur les fibres kératiniques pendant un temps nécessaire à l'obtention de la coloration désirée, puis rincés.

Les colorants classiques qui sont utilisés sont en particulier des colorants du type nitré benzénique, anthraquinonique, nitropyridinique, azoïque, xanthénique, acridinique, azinique, triarylméthane ou des colorants naturels.

Certains de ces colorants peuvent être utilisés dans des conditions éclaircissantes ce qui permet d'obtenir des colorations visibles sur des cheveux foncés.

Il est aussi connu de teindre les fibres kératiniques de façon permanente par la coloration d'oxydation. Cette technique de coloration consiste à appliquer sur les fibres kératiniques une composition contenant des précurseurs de colorant tels que des bases d'oxydation et des coupleurs. Ces précurseurs sous l'action d'un agent oxydant vont former dans le cheveu une ou plusieurs espèces colorées.

La variété des molécules mises en jeu au niveau des bases d'oxydation et des coupleurs, permet l'obtention d'une riche palette de couleurs et les colorations qui en résultent sont permanentes, puissantes, et résistantes aux agents extérieurs, notamment à la lumière, aux intempéries, aux lavages, à la transpiration et aux frottements.

Pour être visible sur cheveux foncés, ces deux techniques de coloration nécessitent généralement une décoloration préalable ou simultanée des fibres kératiniques. Cette étape de décoloration mise en oeuvre avec un agent oxydant tel que le peroxyde d'hydrogène ou de persels entraîne une dégradation non négligeable des fibres kératiniques ce qui altère leurs propriétés cosmétiques. Les cheveux ont alors tendance à devenir rêches, plus difficilement démêlables et plus fragiles.

Une autre méthode de coloration consiste à utiliser des pigments. En effet, l'utilisation de pigment à la surface des fibres kératiniques permet en général d'obtenir des colorations visibles sur cheveux foncés puisque le pigment en surface masque la couleur naturelle de la fibre. L'utilisation de pigment pour colorer des fibres kératiniques est par exemple décrite dans la demande de brevet FR 2 741 530, qui préconise l'utilisation pour la coloration des fibres kératiniques d'une composition comprenant au moins une dispersion de particules de polymère filmogène comportant au moins une fonction acide et au moins un pigment dispersé dans la phase continue de ladite dispersion. Le gainage obtenu n'est pas rémanent aux shampooings.

Il est par ailleurs connu d'effectuer une coloration des cheveux à partir d'une composition contenant un cyanoacrylate et des pigments, notamment dans les demandes de brevet EP 1649898. Cependant, le gainage obtenu ne donne pas une totale satisfaction face aux agents extérieurs tels que le shampoing et la transpiration. Par ailleurs, le gainage obtenu est sensible aux corps gras tels que le sébum.

D'autre part, les demandes de brevet EP 1266647 et WO 2005/060922 décrivent des compositions destinées au maquillage des lèvres, des cils ou du teint qui contient un copolymère polysiloxane contenant au moins un motif capable de former des liaisons hydrogène. A titre d'exemple de motifs capables de former des liaisons hydrogène, sont cités, entre autre, les polyurées. Ces documents n'envisagent cependant pas l'utilisation capillaire d'une telle composition pour le gainage des cheveux.

Ainsi, le but de la présente invention est de mettre au point une composition de coloration des fibres kératiniques qui permet d'obtenir une coloration rémanente aux diverses agressions que peuvent subir les cheveux, notamment une coloration rémanente aux shampoings et à la transpiration tout en conservant l'intégrité des fibres kératiniques.

Ce but est atteint avec la présente invention qui a pour objet une composition de coloration qui comprend au moins un copolymère non ionique bloc polysiloxane/polyurée, au moins une espèce coloré ou colorante et au moins un solvant organique non siliconé volatil.

L'invention a aussi pour objet un procédé de coloration des fibres kératiniques, notamment les cheveux qui comprend l'application sur les fibres de la composition de l'invention.

Par l'utilisation d'une telle composition, on obtient sur les fibres kératiniques une coloration dans des nuances variées rémanente aux shampooings tout en préservant les qualités physiques des fibres kératiniques. Une telle coloration est en particulier résistante aux agressions extérieures que peuvent subir les cheveux telles que les shampoings et la transpiration.

Par ailleurs, le gainage coloré ainsi formé se présente sous forme d'un dépôt homogène, lisse et possède une excellente adhésion sur cheveux.On a constaté de façon surprenante que les cheveux restaient parfaitement individualisés, pouvaient être coiffés sans problème et que les propriétés de coloration apportées à la fibre étaient rémanentes aux shampooings.

La composition cosmétique conforme à l'invention permet d'obtenir, notamment avec les pigments colorés des colorations visibles même sur des cheveux foncés sans qu'il soit nécessaire de décolorer les fibres kératiniques puisque l'espèce coloré ou colorante restant en surface permet de masquer la couleur naturelle de la fibre.

Dans la présente invention, le copolymère est un copolymère non ionique polysiloxane/polyurée, c'est-à-dire qu'il ne contient pas de groupement ionisé ou ionisable.

Dans le cadre de l'invention on entend par copolymère bloc, un copolymère constitué d'au moins deux séquences distinctes de chacun des polymères constituant le copolymère dans le squelette du copolymère. Par exemple, le copolymère de l'invention contient au moins une séquence (ou bloc) de polysiloxane et au moins une séquence (bloc) de polyurée dans le squellette du copolymère.

Le copolymère de l'invention peut comprendre en plus du polysiloxane/polyurée d'autres blocs de polymères différents. On citera en particulier les copolymères bloc polysiloxane/polyurée/polyuréthane. Selon un mode de réalisation particulier, le copolymère contient une quantité en poids de polysiloxane supérieure à 5%. Selon un mode de réalisation particulier, la quantité de polysiloxane est majoritaire dans le copolymère, de préférence supérieure à 90% en poids par rapport au poids total du copolymère.

Selon une variante, le copolymère contient uniquement un ou plusieurs blocs siloxane et un ou plusieurs blocs polyurée.

Le copolymère de l'invention peut comprendre en plus du polysiloxane/polyurée d'autres blocs de motifs différents. On citera en particulier les terpolymères blocs polysiloxane/polyurée/polyuréthane.

Selon un mode de réalisation particulier, le copolymère contient une quantité en poids de polysiloxane supérieure à 5%. Selon un mode de réalisation particulier, la quantité de polysiloxane est majoritaire dans le copolymère, de préférence supérieure à 90% en poids par rapport au poids total du copolymère.

Selon une variante, le copolymère contient uniquement un ou plusieurs blocs siloxane et un ou plusieurs blocs polyurée.

Selon l'invention, le copolymère peut répondre à la formule générale (1) : 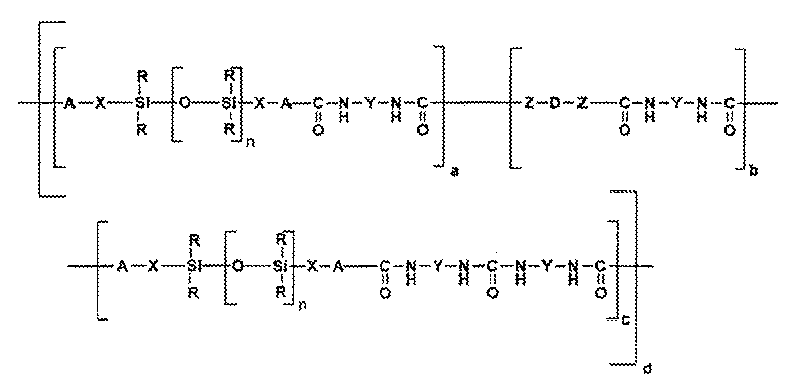 dans laquelle
- R: représente un radical hydrocarboné monovalent, le cas échéant substitué par le fluor ou le chlore, ayant 1 à 20 atomes de carbone,
- X: représente un radical alkylène ayant 1 à 20 atomes de carbone, dans lequel des unités méthylène non voisines peuvent être remplacées par des radicaux -O-,
- A: représente un atome d'oxygène ou un radical amino -NR'-,
- Z: représente un atome d'oxygène ou un radical amino -NR'-,
- R': représente hydrogène ou un radical alkyle ayant 1 à 10 atomes de carbone,
- Y: représente un radical hydrocarboné bivalent, le cas échéant substitué par le fluor ou le chlore, ayant 1 à 20 atomes de carbone,
- D: représente un radical alkylène, le cas échéant substitué par fluor, chlore, alkyle en C₁-C₆ ou ester d'alkyle en C₁-C₆, ayant 1 à 700 atomes de carbone, dans lequel des unités méthylène non voisines peuvent être remplacées par des radicaux -O-, -COO-, -OCO- ou -OCOO-,
- n: est un nombre allant de 1 à 4000,
- a: est un nombre d'au moins 1,
- b: est un nombre allant de 0 à 40,
- c: est un nombre allant de 0 à 30, et
- d: est un nombre supérieur à 0.
à la condition que A représente dans au moins un des motifs (a) un radical NH

De préférence, R représente un radical hydrocarboné monovalent avec 1 à 6 atomes de carbone par exemple méthyle, éthyle, vinyle et phényle. Selon un mode de réalisation particulier, R est un radical alkyle non substitué.

De préférence, X représente un radical alkylène avec 2 à 10 atomes de carbone. De préférence, le radical alkylène X n'est pas interrompu.

Selon un mode de réalisation particulier, le groupement A dans tous les motifs (b) et (c), lorsqu'ils sont présents, représente NH.

Selon un mode de réalisation particulièrement préféré, tous les groupements A représentent un radical NH.

De préférence, Z représente un atome d'oxygène ou un radical NH.

De préférence, Y représente un radical hydrocarboné comprenant de 3 à 13 atomes de carbone, qui est de préférence, non substitué. De préférence, Y représente un radical aralkylène, alkylène, linéaire ou cyclique.

De préférence, D représente un radical alkylène avec au moins 2, en particulier au moins 4 atomes de carbone et au maximum 12 atomes de carbone.

De préférence également, D représente un radical polyoxyalkylène, en particulier un radical polyoxyéthylène ou polyoxypropylène avec au moins 20, en particulier au moins 100 atomes de carbone et au maximum 800, en particulier au maximum 200 atomes de carbone.

De préférence, le radical D n'est pas substitué.

De préférence, n représente un nombre d'au moins 3, en particulier au moins 25 et de préférence, au maximum 800, en particulier au maximum 400, de manière particulièrement préférée, au maximum 250.

De préférence, a représente un nombre de plus de 50.

Lorsque b est différent de 0, b représente de préférence, un nombre d'au maximum 50, en particulier au maximum 25.

De préférence, c représente un nombre d'au maximum 10, en particulier au maximum 5.

Les copolymères de l'invention peuvent être obtenus selon les procédés de polymérisation décrits dans la demande de brevet US 2004/0254325 ou la demande WO 03/014194.

Le copolymère peut ainsi être obtenu par un procédé en deux étapes, tel que :
- dans la première étape, on fait réagir un silazane de formules générales (2) ou (2'): 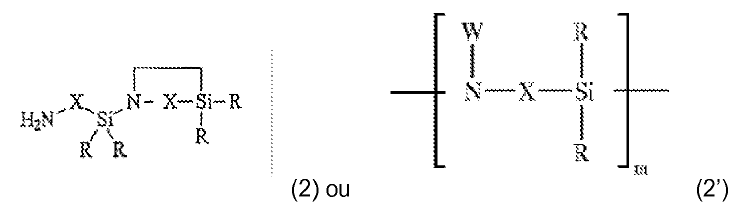 W représentant un atome d'hydrogène, un radical hydrocarboné substitué ou non, comprenant de préférence de 1 à 20 atomes de carbone ou un radical R₂Si-X-NH₂ ; avec un composé organique du silicium de la formule générale (3) :

   (HO)(R₂SiO)ₙ₋₁[H] (3)

   pour obtenir un aminoalkylpolydiorganosiloxane de la formule générale (4)

   H₂N-X-[SIR₂O]ₙSIR₂-X-NH₂ (4)
- dans une deuxième étape, l'aminoalkyl-polydiorganosiloxane de la formule générale (4) est polymérisé avec un diisocyanate de la formule générale (5):

   OCN-Y-NCO (5)

De manière générale, dans la première étape, on met en oeuvre les silazanes de formule générale (2) ou (2') et les réactifs contenant des groupes silanol en rapports équimolaires.

Pour la préparation des silicones à terminaison bisaminoalkyle très purs, de la formule générale (4), on utilise de préférence, un petit excès du composé silazane de la formule générale (2) ou (2'), qui peut être ensuite éliminé, dans une étape de procédé supplémentaire simple, comme par exemple, l'addition de faibles quantités d'eau.

Si b est au moins 1, on peut mettre en oeuvre au cours de la deuxième étape, jusqu'à 95% en poids, sur base de tous les composants mis en oeuvre, d'agents d'allongement de chaîne, qui sont choisis parmi les diamines, les composés hydroxy bloqués par un isocyanate, les composés dihydroxy ou leurs mélanges.

De préférence, les agents d'allongement de chaîne présentent la formule générale (6) :

HZ-D-ZH (6)

où D et Z présentent les significations précédentes. Si Z a la signification O, l'agent d'allongement de chaîne de la formule générale (6) peut également être mis à réagir avant la réaction dans la deuxième étape, avec le diisocyanate de la formule générale (5). Le cas échéant, on peut mettre en oeuvre de l'eau comme agent d'allongement de chaîne.

Des exemples de diisocyanates de la formule générale (5) à utiliser, sont des composés aliphatiques comme l'isophoronediisocyanate, l'hexaméthylène-1,6-diisocyanate, le tétraméthylène-1,4-düsocyanate et le méthylènedicyclohexyl-4,4'-diisocyanate ou des composés aromatiques comme le méthylènediphényl-4,4'-diisocyanate, le 2,4-toluènedüso-cyanate, le 2,5-toluènediisocyanate, le 2,6-toluènediiso-cyanate, le m-phénylènedüsocyanate, le p-phénylène-düsocyanate, le m-xylènediisocyanate, le tétraméthyl-m-xylènedüsocyanate ou des mélanges de ces isocyanates. Un exemple de composé disponible dans le commerce est un diisocyanate de la série DESMODUR® (H, 1, M, T, W) de Bayer AG, Allemagne. On préfère les diisocyanates aliphatiques, dans lesquels Y est un radical alkylène, parce que ceux-ci conduisent à des matériaux, qui présentent des stabilités aux U.V. améliorées.

Les alkylènes à terminaison α,ω-OH de la formule générale (6) sont de préférence, des polyalkylènes ou des polyoxyalkylènes. Ceux-ci sont de préférence, essentiellement exempts de contaminations de polyoxyalkylènes mono-, trifonctionnels ou de fonctionnalité supérieure. On peut mettre en oeuvre ici, des polyétherpolyols, polytétraméthylènediols, polyesterpolyols, polycaprolactone-diols, mais également des polyalkylènes à terminaison α,ω-OH à base de poly(acétate de vinyle), de copolymères poly(acétate de vinyle)-éthylène, de copolymères poly(chlorure de vinyle), de polyisobutyèneldiols. De préférence, on utilise des polyoxyalkylènes, de manière particulièrement préférée, des polypropylèneglycols. De tels composés sont disponibles dans le commerce comme matériaux de base entre autres, pour des mousses polyuréthanne et pour des utilisations comme revêtement, avec des masses moléculaires Mn de jusqu'à 10 000. Des exemples sont les polyétherpolyols et polyesterpolyols BAYCOLL® de BAYER AG, Allemagne ou les polyétherpolyols Acclaim® de Lyondell Inc., USA. On peut mettre en oeuvre également, des monomères α,ω-alkylènediols, comme l'éthylèneglycol, le propanediol, le butanediol ou l'hexanediol. D'autre part, par composés dihydroxylés dans le sens de l'invention, on entend également les bishydroxyalkyl-silicones, comme elles sont fournies par exemple par la société Goldschmidt sous les noms Tegomer H-Si 2111, 2311 et 2711.

La préparation des copolymères décrits ci-dessus de la formule générale (I) peut se faire en solution, mais également sous forme solide, de manière continue ou discontinue.

Si la quantité de segments uréthane ou urée est grande, on choisit un solvant ayant un paramètre élevé de solubilité, comme par exemple le diméthylacétamide. On peut également utiliser le THF. Selon un mode de réalisation particulier, la synthèse du copolymère est mise en oeuvre sans solvant.

La synthèse est de préférence réalisée en l'absence d'humidité et sous gaz protecteur, usuellement l'azote ou l'argon.

La réaction est réalisée de préférence en présence d'un catalyseur. Les catalyseurs appropriés pour la préparation sont des composés dialkylétain, comme par exemple le dilaurate de dibutyl-étain, le diacétate de dibutyl-étain, ou des amines tertiaires comme par exemple la N,N-diméthylcyclohexanamine, le 2-diméthylaminoéthanol, la 4-diméthylaminopyridine.

Selon un mode de réalisation particulier, le copolymère utile dans la présente invention ne contient pas de polyuréthane.

A titre d'exemple de copolymère, on peut citer le copolymère diméthylpolysiloxane/urée, de nom INCI polyureadimethicone.

Un tel polymère peut être obtenu notamment par copolymérisation d'un alpha,oméga-aminosilicone avec un di-isocyanate. Des polymères répondant à ces caractéristiques sont par exemple les produits commercialisés sous la référence Wacker-Belsil ® UD 60 Wacker-Belsil ® UD 80, Wacker-Belsil ® UD 140 et Wacker-Belsil ® UD 200 par la société Wacker.

Selon l'invention, la composition appliquée sur le cheveux contient au moins un solvant organique non siliconé volatil. Selon l'invention, la composition appliquée sur le cheveux contient au moins un solvant organique non siliconé volatil. Dans le cadre de l'invention, on entend par solvant volatile, un composé organique liquide à la température ambiante (20°C) et à la pression atmosphérique présentant une pression de vapeur à 20°C supérieure à 0,1 mmHg et de préférence comprise entre 0,1 et 300 mmHg, encore plus préférentiellement entre 0.5 et 200 mmHg.

A titre de solvant organique non siliconé volatil, on peut citer :
- les alcanols volatils en C₁-C₄ tels que l'éthanol, l'isopropanol;
- les alcanes volatils en C₅-C₇ tels que le n-pentane, l'hexane, le cyclopentane, le 2,3-diméthylbutane, le 2,2-diméthylbutane, le 2-méthylpentane, le 3-méthylpentane ;
- les esters d'acides en C₁-C₂₀ liquides et d'alcools en C₁-C₈ volatils tels que l'acétate de méthyle, l'acétate de n-butyle, l'acétate d'éthyle, l'acétate de propyle, l'acétate d'isopentyle, le 3-éthoxypopionate d'éthyle;
- les cétones liquides à température ambiante et volatiles telles que la méthyléthylcétone, la méthylisobutylcétone, la diisobutylcétone, l'isophorone, la cyclohexanone, l'acétone ;
- les polyols volatils tels que le propylène glycol ;
- les éthers volatils tels que le diméthoxyméthane, le diéthoxyéthane, le diéthyléther ;
- les éthers de glycol volatils comme le 2-butoxyéthanol, le butyle diglycol, le monoméhyléther de diéthylène glycol, le n-butyléther de propylène glycol, l'acétate de monométhyl éther de propylène glycol.
- les huiles hydrocarbonées volatiles telles que les huiles volatiles hydrocarbonées ayant de 8 à 16 atomes de carbone et leurs mélanges, et notamment les alcanes ramifiés en C8-C16 comme les iso-alcanes (appelées aussi isoparaffines) en C8-C16, l'isododécane, l'isodécane, et par exemple les huiles vendues sous les noms commerciaux d'Isopars ou de Permetyls, et leurs mélanges. On peut aussi citer les neopentanoate d'isohexyle ou d'isodecyle.
- les perfluoroalcanes volatils en C4-C10 tels que dodecafluoropentane, le tetradecafluorohexane, le decafluoropentane
- les perfluorocycloalkyles volatils tels que le perfluorométhylcyclopentane, le 1,3-perfluorodiméthylcyclohexane et le perfluorodecaline, vendus respectivement sous les dénominations de "Flutec PC1®", "Flutec PC3®" et "Flutec PC6®" par la Société F2 Chemicals, ainsi que le perfluorodiméthylcyclobutane et la perfluoromorpholine.
- les composés fluoroalkyles ou hétérofluoroalkyles volatils répondant à la formule suivante :

   CH₃-(CH₂)ₙ-[Z]ₜ-X-CF₃
dans laquelle t est 0 ou 1 ; n est 0, 1, 2 ou 3 ; X est un radical perfluoroalkyle divalent, linéaire ou ramifié, ayant de 2 à 5 atomes de carbone, et Z représente O, S, ou NR, R étant hydrogène, un radical -(CH₂)ₙ-CH₃ ou -(CF₂)ₘ-CF₃, m étant 2, 3, 4 ou 5. Parmi les composés fluoroalkyles ou hétérofluoroalkyles volatils, on peut notamment citer le méthoxynonafluorobutane vendu sous la dénomination de "MSX 4518®", "HFE-7100®" par la Société 3M et l'éthoxynonafluorobutane vendu sous la dénomination de "HFE-7200®" par la Société 3M.

De préférence, le solvant est choisi de telle manière que son point d'ébullition soit inférieur à 200°C.

De préférence le solvant organique non siliconé volatil est choisi parmi, l'éthanol, l'isopropanol, l'acétone, l'isododécane.

Le solvant organique non siliconé volatil est généralement présent dans la composition selon l'invention en une teneur allant de 0,1 % à 90 % en poids, par rapport au poids total de la composition, de préférence allant de 1 % à 80 % en poids, et préférentiellement allant de 5 % à 70 % en poids.

La composition de l'invention contient au moins une espèce colorée ou colorante telle que des pigments colorés, des colorants directs hydrophiles ou hydrophobes ou des précurseurs de colorants. Les pigments colorés et les colorants directs peuvent être fluorescents ou non.

Par espèce coloré, on entend dans le cadre de l'invention un composé coloré à l'état sec ou en solution, c'est-à-dire absorbant entre 250 et 750 nm et/ou réémettant par excitation une lumière visible. Par espèce colorante, on entend une espèce non colorée générant un ou plusieurs composés colorés par interaction avec un agent réactif, tel qu'un agent oxydant.

A titre de colorant hydrophile, on peut citer les colorants présentant une hydrophilicité définie par la valeur de logP inférieur ou égale à 2. Dans le cadre de l'invention, la valeur du logP représente de façon classique le coefficient de partage du colorant entre l'octanol et l'eau. La valeur du logP peut être calculée selon la méthode décrite dans l'article de Meylan et Howard « Atom / Fragment contribution method for estimating octanol-water partition coefficient », J. Pharm. Sci., 1995, 84, p83-92. Cette valeur peut aussi être calculée à partir de nombreux logiciels disponibles sur le marché qui détermine la valeur de logP en fonction de la structure d'une molécule.

A titre d'exemple, on peut citer le logiciel Epiwin de l'agence de l'environnement des Etats-Unis.

De façon avantageuse, LogP des colorants hydrohiles utiles dans la composition de l'invention est strictement inférieur à 2.

Parmi ces colorants hydrophiles, on peut citer les colorants directs nitrés benzéniques neutres ; acides ou cationiques ; les colorants directs azoïques neutres acides ou cationiques ; les colorants directs quinoniques et en particulier anthraquinoniques neutres, acides ou cationiques ; les colorants directs aziniques ; les colorants directs triarylméthaniques ; les colorants directs indoaminiques et les colorants directs naturels.

Ainsi, les colorants directs peuvent être choisis en fonction de la valeur LogP.

| **STRUCTURE** | **NOM** | **log P** |
|---|---|---|
| 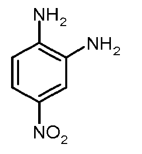 | 4-nitro-o-phénylène-diamine | 0.88 |
| 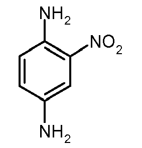 | 2-nitro-p-phénylène-diamine | 0.53 |
| 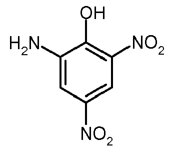 | acide picramique | 0.93 |
| 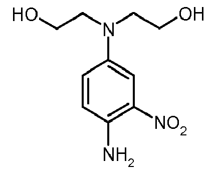 | HC Red 13 | 0.66 |
| 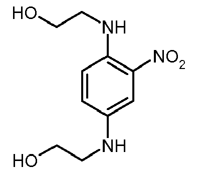 | N,N'-bis-(2-hydroxyéthyl)-2-nitro-p-phénylène-diamine | -0.44 |
| 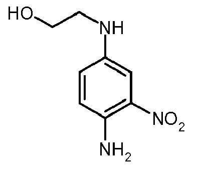 | HC Red 7 | 0.13 |
| 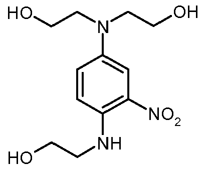 | HC Blue 2 | -0.32 |
| 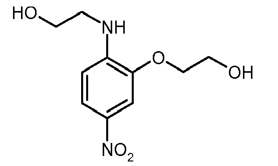 | HC Yellow 4 | 0.56 |
| 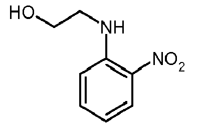 | HC Yellow 2 | 1.05 |
| 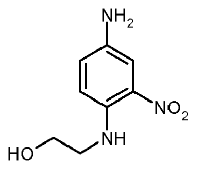 | HCRed3 | -0.42 |
| 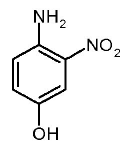 | 4-amino-3-nitrophénol | 1.19 |
| 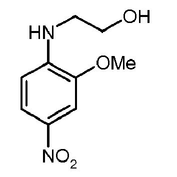 | 1-hydroxyéthyl-amino-5-nitroanisole | 1.13 |
| 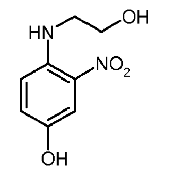 | 3-nitro-p-hydroxyéthylamino phénol | 0.21 |
| 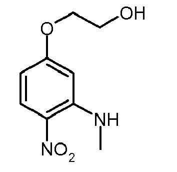 | 3-méthylamino-4-nitrophénoxyéthanol | 1.13 |
| 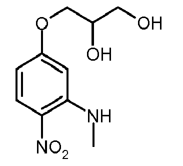 | 2-nitro-5-glycéryl méthylaniline | 0.89 |
| 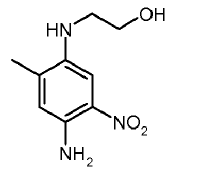 | HC Violet 1 | 0.67 |
| 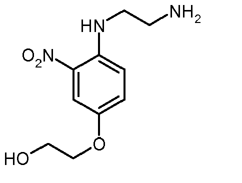 | HC Orange 2 | 0.15 |
| 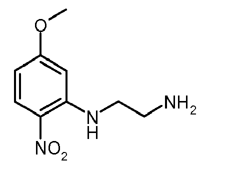 | HC Yellow 9 | 1.12 |
| 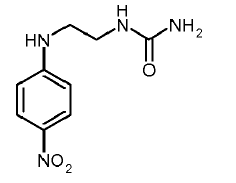 | 4-nitrophényl aminoéthylurée | 0.59 |
| 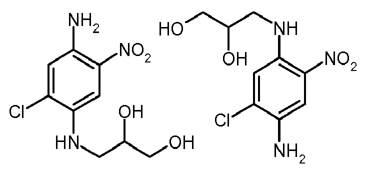 | HC Red 10 et HC Red 11 | 0.13 |
| 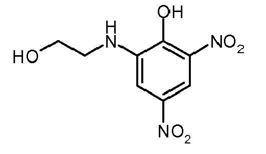 | Acide 2-hydroxyéthyl picramique | 0.38 |
| 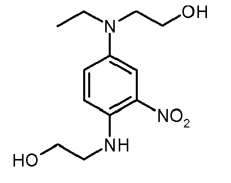 | HC Blue 12 | 1.15 |
| 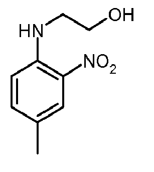 | 3 nitro 4 N beta hydroxyéthyl aminotoluène | 1.59 |
| 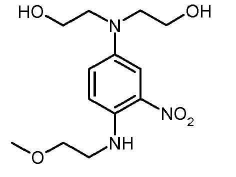 | 2 N béta méthoxyéthylamino 5 N,Nbis (hydroxyéthyl) amino nitrobenzène | 0.38 |
| 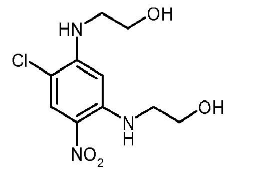 | HC Yellow 10 | 0.20 |
| 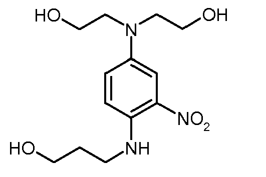 | HC Violet 2 | 0.17 |
| 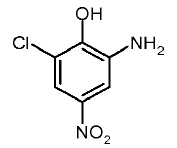 | 2-amino-6-chloro-4-nitrophénol | 1.53 |
| 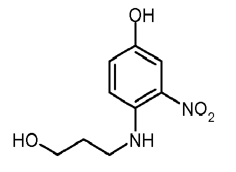 | 4-hydroxypropyl-amino-3-nitrophénol | 0.70 |
| 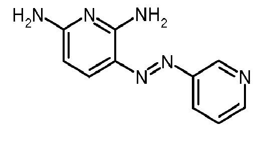 | 2,6-diamino-3-((pyridine-3-yl)-azo)pyridine | 1.58 |
| 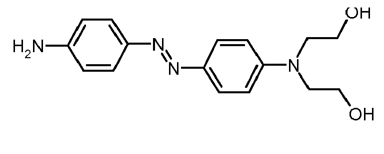 | Disperse Black 9 | 1.83 |
| 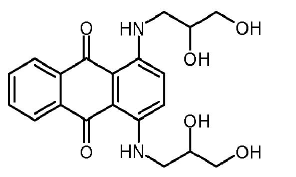 | HC Blue 14 | 0.62 |

La composition de l'invention peut aussi contenir des colorants hydrophobes. Dans le cadre de l'invention, l'hydrophobicité est définie par la valeur de logP qui doit être supérieure à 2.

A titre d'exemple, on peut citer :

| **Colorant** | **Structure chimique** | **logP** |
|---|---|---|
| Solvent Black 3 | 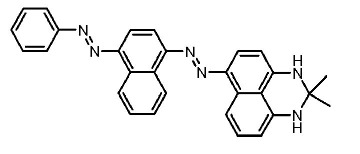 | 8.81 |
| Solvent Blue 104 | 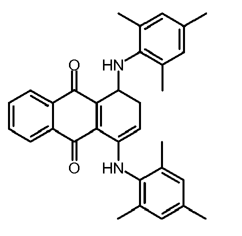 | 8.26 |
| Disperse Blue 134 | 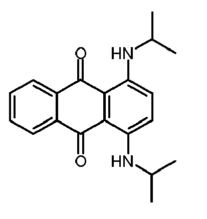 | 6.07 |
| Solvent Blue 14 | 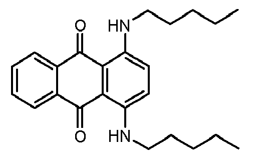 | 8.18 |
| Disperse Blue 14 | 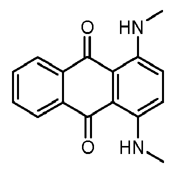 | 4.25 |
| Solvent Red 2 | 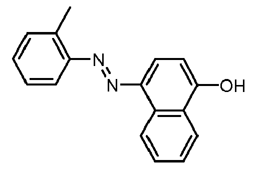 | 5.35 |
| Solvent Brown 5 | 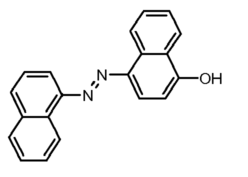 | 5.98 |
| Solvent Green 5 | 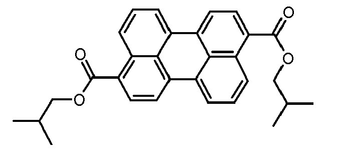 | 8.55 |
| Solvent Orange 2 | 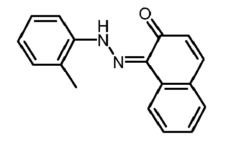 | 3.86 |
| Solvent Orange 1 | 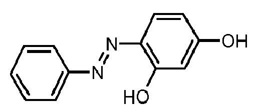 | 3.85 |
| Disperse Orange 24 | 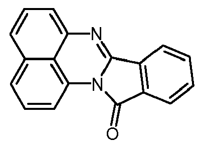 | 3.21 |
| Solvent Orange 63 | 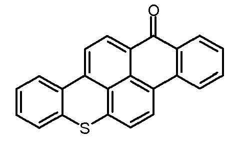 | 7.02 |
| Solvent Red 49 | 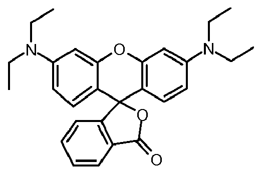 | 6.63 |
| Solvent Red 1 | 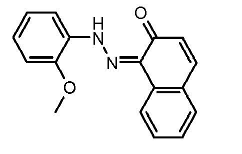 | 3.39 |
| Solvent Red 26 | 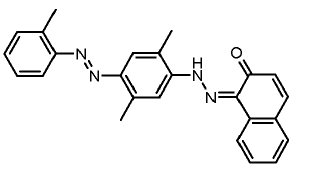 | 7.07 |
| Solvent Red 27 | 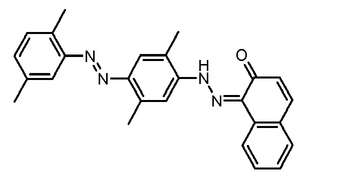 | 7.62 |
| Solvent Red 18 | 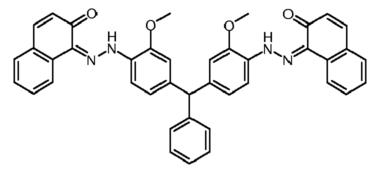 | 8.16 |
| Solvent Red 23 | 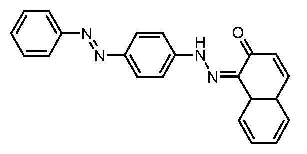 | 5.58 |
| Solvent Red 4 | 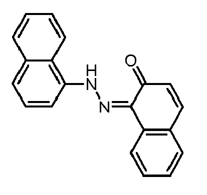 | 4.48 |
| Solvent Orange 7 | 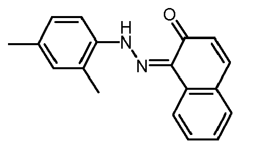 | 4.40 |
| Disperse Blue 72 | 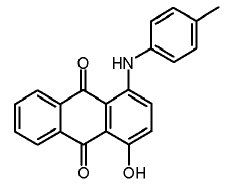 | 6.24 |
| Disperse Violet 26 | 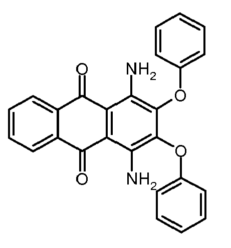 | 5.19 |
| Disperse Yellow 16 | 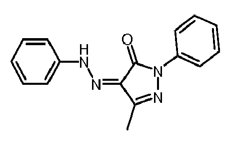 | 3.89 |
| Disperse Yellow 82 | 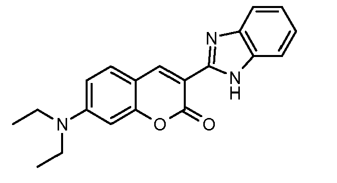 | 3.68 |
| Disperse Yellow 54 | 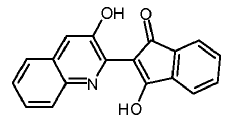 | 4.76 |
| Solvent Yellow 29 | 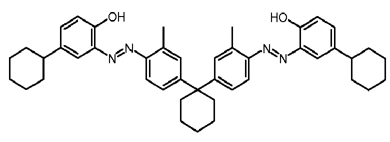 | 17.37 |
| Solvent Yellow 163 | 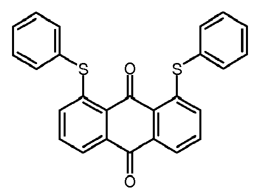 | 7.94 |
| Solvent Yellow 3 | 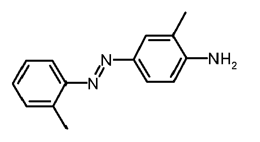 | 4.29 |
| Solvent Yellow 56 | 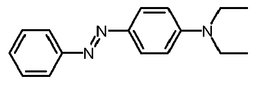 | 5.27 |
| Solvent Yellow 18 | 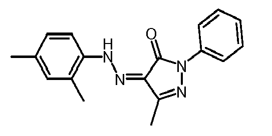 | 4.98 |
| Solvent Yellow 98 | 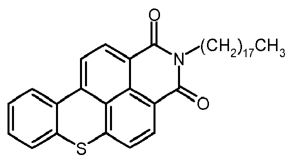 | 4.5 |
| Solvent Yellow 12 | 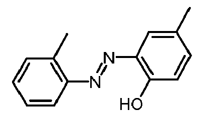 | 5.43 |
| Solvent Yellow 14 | 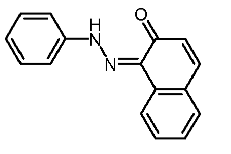 | 3.31 |
| Disperse Red 13 | 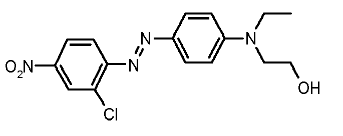 | 5.22 |
| Disperse Green 9 | 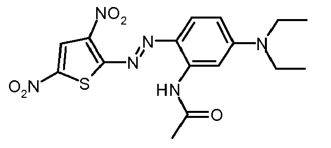 | 4.23 |
| Disperse Blue 148 | 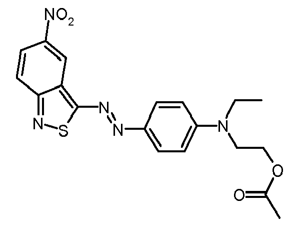 | 4.81 |
| Disperse Violet 63 | 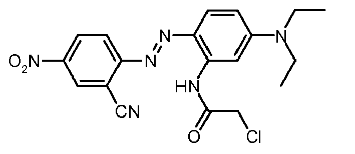 | 5.30 |
| Disperse Blue 60 | 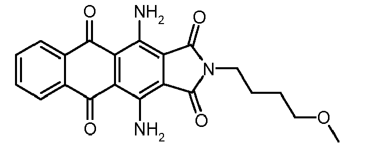 | 3.38 |
| Solvent Orange 15 | 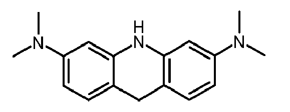 | 3.90 |

De préférence les colorants hydrophobes ont un logP supérieur à 4 et encore plus préférentiellement supérieur à 6.

L'utilisation de colorants fluorescents peut permettre d'obtenir sur cheveux foncés des colorations plus visibles qu'avec des colorants directs hydrophiles ou hydrophobes traditionnels. De plus ces colorants fluorescents lorsqu'ils sont appliqués sur des cheveux foncés peuvent également permettre de les éclaircir sans les dégrader. Cette technique décrite notamment dans le document FR 2830189 permet de respecter la qualité de la fibre kératinique lors du traitement mais les colorants fluorescents employés ne présente pas une bonne résistance aux shampoings. La présence du copolymère PDMS-polyurée permet d'améliorer cette rémanence aux shampooings.

On entend par ailleurs par composé fluorescent des colorants fluorescents et des azurants optiques. Ces composés fluorescents doivent eux aussi être solubles dans le milieu de la composition.

Les colorants fluorescents sont des composés fluorescents qui absorbent le rayonnement visible généralement entre 400 et 800 nm et qui sont capables de réémettre de la lumière dans le domaine du visible à une longueur d'onde supérieure. Par définition, ces colorants sont des espèces colorées puisqu'elles absorbent de la lumière visible.

Selon un mode de réalisation particulier, les colorants fluorescents utiles dans le cadre de l'invention réémettent de la lumière fluorescente orangée. Ils présentent notamment une longueur d'onde maximum de ré-émission comprise entre comprise entre 500 et 700 nm.

A titre d'exemple de colorants fluorescents, on peut citer les composés connus de la technique, décrits par exemple dans les ouvrages suivants : Ullmann's Encyclopedia of Industrial Chemistry Release 2004, 7th edition, chapitre « Fluorescent Dyes ».

Les azurants optiques utiles dans la présente invention aussi connus sous le nom de "brighteners", ou "fluorescent brighteners", ou "fluorescent brightening agents" ou "FWA", ou "fluorescent whitening agents", ou "whiteners", ou encore "fluorescent whiteners" en terminologie anglo-saxone, sont des composés transparents incolores car il n'absorbent pas dans la lumière visible, mais uniquement dans les Ultra Violets (longueurs d'onde allant de 200 à 400 nanomètres), et transforment l'énergie absorbée en lumière fluorescente de plus grande longueur d'onde émise dans la partie visible du spectre ; en général dans le bleu et/ou le vert, c'est-à-dire dans des longueurs d'onde allant de 400 à 550 nanomètres.

Les azurants optiques sont connus de la technique. Ils sont par exemple décrits dans Ullmann's Encyclopedia of Industrial Chemistry (2002) "Optical Brighteners" etKirk-Othmer Encyclopedia of Chemical Technology (1995); "Fluorescent Whitening Agents".

Les colorants fluorescents qui peuvent être utilisés dans le cadre de l'invention sont des composés connus de la technique. Ils sont par exemple décrits dans le document FR 2 830 189. A titre d'exemples de colorants fluorescents, on peut notamment citer :
- le Photosensitizing Dye NK-557 commercialisé par la société UBICHEM qui présente la structure suivante : 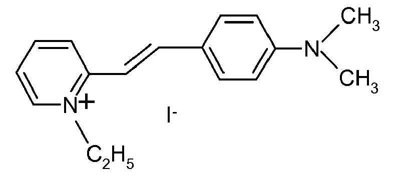 iodure de 2-[2-(4-diméthylamino)phényl éthényl]-1 éthyl-pyridinium ;
- le Jaune Brilliant B6GL commercialisé par la société SANDOZ et de structure suivante : 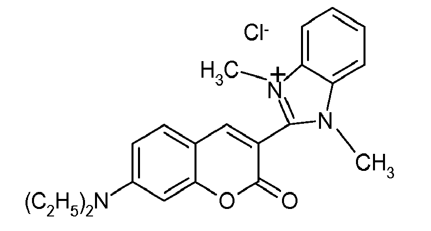
- le Basic Yellow 2, ou Auraminoe O commercialisé par les sociétés PROLABO, ALDRICH ou CARLO ERBA et de structure suivante : 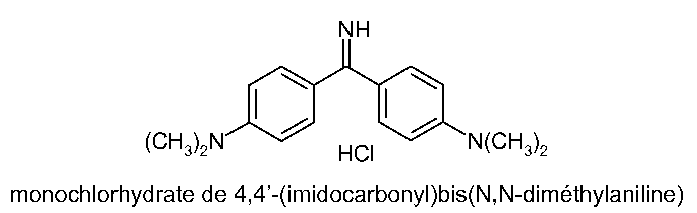 A titre d'azurants optiques et de colorants fluorescents utiles dans la présente invention on peut aussi citer :
• Les naphthalimides, par exemple le composé suivant 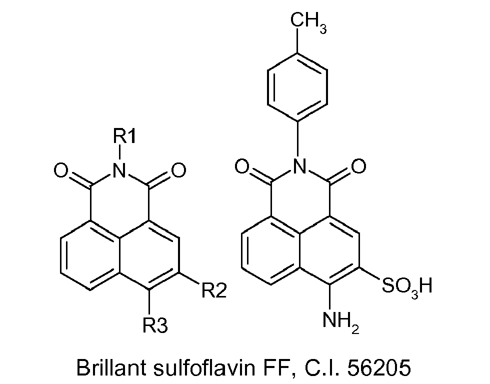 Dans laquelle R₁, R₂, R₃, indépendamment les uns des autres représentent un atome d'hydrogène ; un atome d'halogène ; un groupement aryle en C₆-C₃₀; un groupement hydroxy ; un groupement cyano ; un groupement nitro ; un groupement sulfo ; un groupement amino ; un groupement acylamino ; un groupement dialkyl(C₁-C₆)amino ; un groupement dihydroxyalkyl(C₁-C₆)amino; un groupement alkyl(C₁-C₆)hydroxyalkyl(C₁-C₆)amino ; un groupement alcoxy(C₁-C₆) ; un groupement alcoxy(C₁-C₆)carbonyle ; un groupement carboxyalcoxy en C₁-C₆; un groupement pipéridinosulfonyle ; un groupement pyrrolidino ; un groupement alkyle(C₁-C₆)halogénoalkyle(C₁-C₆)amino; un groupement benzoylalkyle(C₁-C₆) ;un groupement vinyle ; un groupement formyle ; un radical aryle en C₆-C₃₀ éventuellement substitué par un ou plusieurs groupements choisis parmi les groupements hydroxyle, alcoxy en C₁-C₆ linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C₁-C₆ un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C₁-C₆ linéaire, ramifié ou cyclique, aryle éventuellement substitué, carboxyle, sulfo, un atome d'halogène,ce radical alkyle pouvant être interrompu par un hétéroatome, tel que N, S ou O ; les substituants R₁, R₂, R₃ peuvent former avec les atomes de carbone auxquels ils sont rattachés un cycle aromatique ou non, en C₆-C₃₀
   ou hétérocyclique comportant au total de 5 à 30 chaînons et de 1 à 5 hétéroatomes ; ces cycles étant condensés ou non, insérant ou non un groupement carbonyle, et étant substitués ou non par un ou plusieurs groupements choisis parmi les groupements alkyle en C₁-C₄, alcoxy(C₁-C₄)alkyl(C₁-C₄), amino, dialkyl(C₁-C₄)amino, halogène, phényle, carboxy, trialkyl(C₁-C₄)ammonioalkyl(C₁-C₄).
• Les dérivés de coumarines tels que les composés correspondants aux formules suivantes 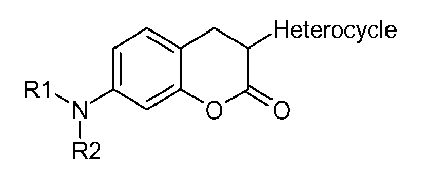 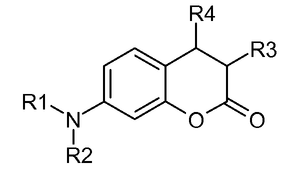 dans laquelle l'hétérocycle est choisi parmi les furane, tiophène, 2H-pyrrole, 2-pyrroline, pyrrolidine, 1,3-dioxolane, oxazole, thiazole, Imidazole, 2-imidazoline, Imidazoline, pyrazole, 2-pyrazoline, pyrazolidine, isoxazole, isothiazole, 1,2,3-oxadiazole, 1,2,3-triazole, 1,3,4-thiadiazole, 2H-pyran, 4H-pyran, pyridine, piperidine, 1,4-dioxane, morpholine, 1,4-dithiane, thiomorpholine, pyridazine, pyrimidine, pyrazine, piperazine, 1,3,5-Triazine, 1,3,5-trithiane, indolizine, indole, isoindole, 3H-indole, indoline, benzo[b]furan, benzo[b]thiophene, 1H-indazole, benzimidazole, benzothiazole, purine, 4H-quinolizine, quinoline, isoquinoline, cinnoline, phtalazine, quinazoline, quinoxaline, 1,8-naphtyridine, pteridine, quinuclidine, carbazole, acridine, phenazine, phenothiazine, phenoxazine, indene, naphtalene, azulene, fluorene, anthracene, norbornane, adamantane, et
   R₁, R₂, R₃, R₄ indépendamment les uns des autres représentent un atome d'hydrogène ; un atome d'halogène ; un groupement aryle en C⁶-C₃₀ ; un groupement hydroxy ; un groupement cyano ; un groupement nitro ; un groupement sulfo ; un groupement amino ; un groupement acylamino ; un groupement dialkyl(C₁₋C₆)amino ; un groupement dihydroxyalkyl(C₁-C₆)amino ; un groupement alkyl(C₁-C₆)hydroxyalkyl(C₁-C₆)amino ; un groupement alcoxy(C₁-C₆) ; un groupement alcoxy(C₁-C₆)carbonyle ; un groupement carboxyalcoxy en C₁-C₆ ; un groupement pipéridinosulfonyle ; un groupement pyrrolidino ; un groupement alkyle(C₁-C₆)halogénoalkyle(C₁-C₆)amino; un groupement benzoylalkyle(C₁-C₆) ; un groupement vinyle ; un groupement formyle ; un radical aryle en C₆-C₃₀ éventuellement substitué par un ou plusieurs groupements choisis parmi les groupements hydroxyle, alcoxy en C₁-C₆ linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C₁-C₆ un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C₁-C₆ linéaire, ramifié ou cyclique, aryle éventuellement substitué, carboxyle, sulfo, un atome d'halogène, ce radical alkyle pouvant être interrompu par un hétéroatome, tel qu'un atome d'azote, de soufre ou d'oxygène ; les substituants R₁, R₂, R₃ et R₄ peuvent former avec les atomes de carbone auxquels ils sont rattachés un cycle aromatique ou non, en C₆-C₃₀ ou hétérocyclique comportant au total de 5 à 30 chaînons et de 1 à 5 hétéroatomes ; ces cycles étant condensés ou non, insérant ou non un groupement carbonyle, et étant substitués ou non par un ou plusieurs groupements choisis parmi les groupements alkyle en C₁-C₄, alcoxy(C₁-C₄)alkyl(C₁-C₄), amino, dialkyl(C₁-C₄)amino, halogène, phényle, carboxy, trialkyl(C₁-C₄)ammonioalkyl(C₁-C₄);
   deux des substituants R₃ , R₄ peuvent former avec les atomes de carbone auxquels ils sont rattachés un noyau aromatique en C₆-C₃₀ ou hétérocyclique comportant au total de 5 à 30 chaînons et de 1 à 5 hétéroatomes ; ce cycle étant condensés ou non,ce cycle et l'éventuel cycle condensé étant substitués ou non par au moins un groupement choisi parmi les groupements alkyle en C₁-C₄, alcoxy(C₁-C₄)alkyl(C₁-C₄), amino, dialkyl(C₁-C₄)amino, halogène, phényle, carboxy, trialkyl(C₁-C₄)ammonioalkyl(C₁-C₄).
   . A titre d'exemple, on peut citer 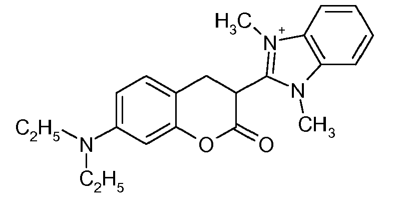 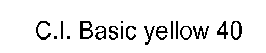
• Les dérivés de xanthènes tels que : 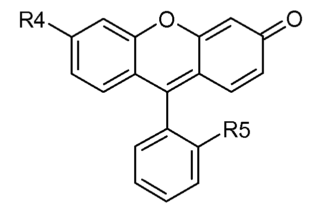 dans laquelle R₄ et R₅ indépendamment les uns des autres représentent un atome d'hydrogène ; un atome d'halogène ; un groupement aryle en C₆-C₃₀; un groupement hydroxy ; un groupement cyano ; un groupement nitro ; un groupement sulfo ; un groupement amino ; un groupement acylamino ; un groupement dialkyl(C₁-C₆)amino ; un groupement dihydroxyalkyl(C₁-C₆)amino; un groupement alkyl(C₁-C₆)hydroxyalkyl(C₁-C₆)amino ; un groupement alcoxy(C₁-C₆) ; un groupement alcoxy(C₁-C₆)carbonyle ; un groupement carboxyalcoxy en C₁-C₆; un groupement pipéridinosulfonyle ; un groupement pyrrolidino ; un groupement alkyle(C₁-C₆)halogénoalkyle(C₁-C₆)amino; un groupement benzoylalkyle(C₁-C₆) ; un groupement vinyle ; un groupement formyle ; un radical aryle en C₆-C₃₀ éventuellement substitué par un ou plusieurs groupements choisis parmi les groupements hydroxyle, alcoxy en C₁-C₆ linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C₁-C₆ un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C₁-C₆ linéaire, ramifié ou cyclique, aryle éventuellement substitué, carboxyle, sulfo, un atome d'halogène,ce radical alkyle pouvant être interrompu par un hétéroatome, tel que l'azote, le soufre et l'oxygène.
• Les rhodamines telles que 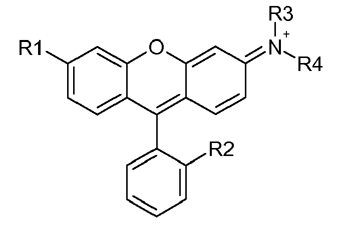 dans laquelle R₁, R₂, R₃ et R₄ sont tels que définis ci-dessus. A titre d'exemple, on peut citer 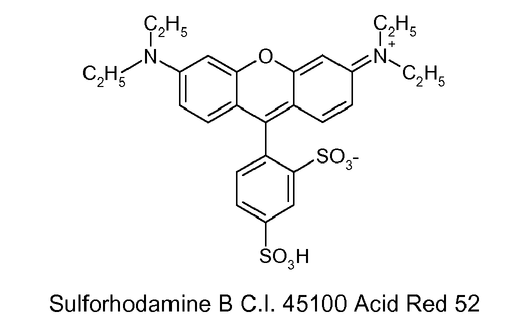
• Les derives de thioxanthènes tels que 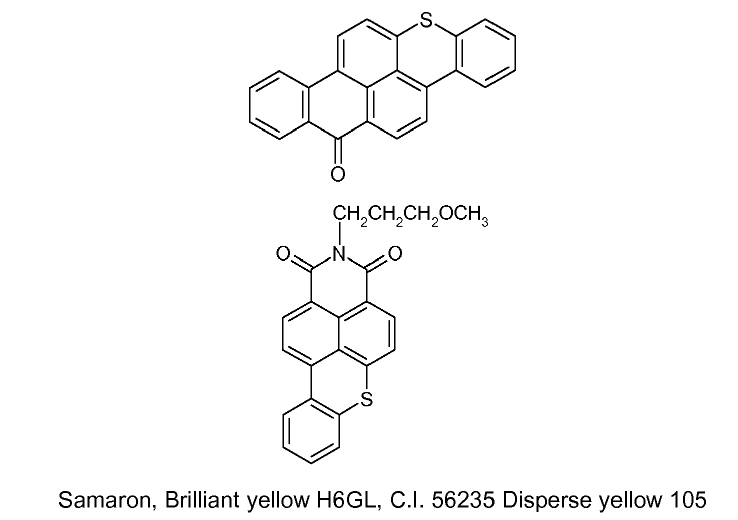
• Les dérivés de naphtolactames tels que : 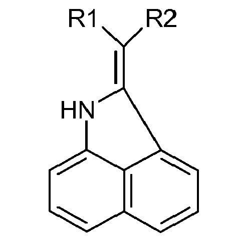 avec R₁ et R₂ définis comme précédemment
   On peut citer par exemple le composé suivant 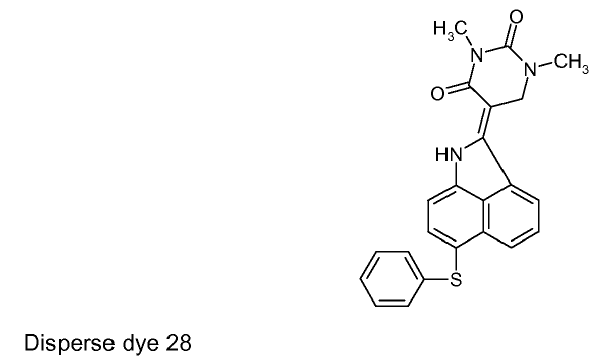
• Les dérivés AZALACTONES : 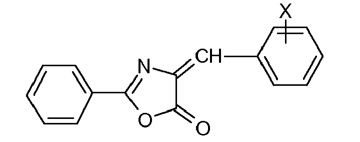 X ayant la même définition que R1 décrit précédemment.
   On peut citer par exemple le composé suivant 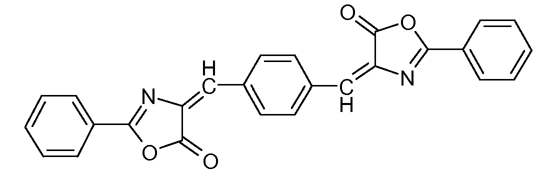
• Les dérivés méthiniques tels que 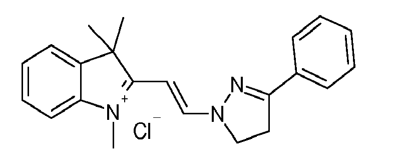 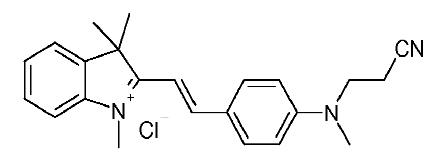
• Les dérivés oxazines et thiazines tels que 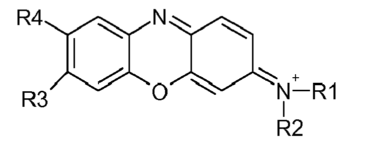 dans lesquelles R₁, R₂, R₃ et R₄ sont tels que définis ci-dessus. A titre d'exemple, on peut citer 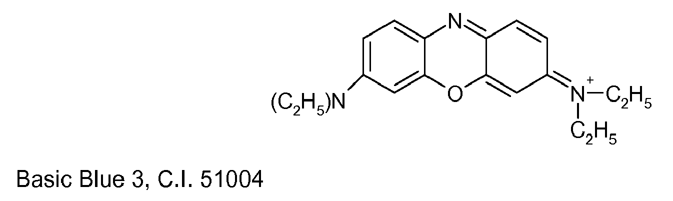
• Les dérives de 1,4-DISTYRYLBENZENES de formule : 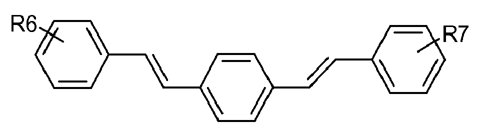 dans laquelle R6 et R7 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle; un groupement formyle; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, aryle éventuellement substitué, carboxyle, sulfo, un atome d'halogène,ce radical alkyle pouvant être interrompu par un hétéroatome, tel que N, S ou O.
• Les dérivés 4,4'-DISTYRYLBIPHENYLES de formule : 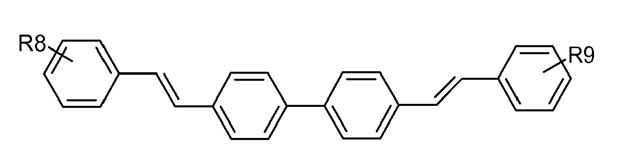 dans laquelle R8 et R9 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle; un groupement formyle; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène.
• Les dérivés TRIAZINYLAMINOSTILBENES de formule : 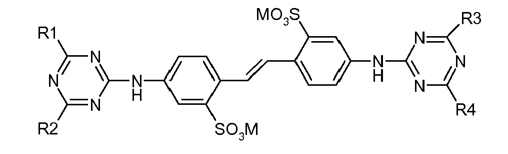 dans laquelle R1, R2, R3 et R4 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(Cl-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle; un groupement formyle; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique,, aryle éventuellement substitué, carboxyle, sulfo, un atome d'halogène,ce radical alkyle pouvant être interrompu par un hétéroatome, tel que l'azote, le soufre et l'oxygène ;
   et M est un cation monovalent ou divalent issu de la famille des alcalins ou des alcalino-terreux, comme par exemple les ions sodium, potassium et calcium.
• Les dérivés STILBAZOLIUM de formule : 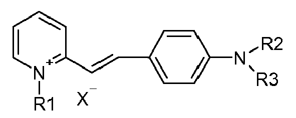 dans laquelle R1, R2 et R3 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle; un groupement formyle; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène ; deux des substituants R2, R3 peuvent former avec les atomes de carbone auxquels ils sont rattachés un noyau aromatique en C6-C30 ou hétérocyclique comportant au total de 5 à 30 chaînons et de 1 à 5 hétéroatomes ; ce cycle étant condensés ou non, ce cycle et l'éventuel cycle condensé étant substitués ou non par au moins un groupement choisi parmi les groupements alkyle en C1-C4, alcoxy(C1-C4)alkyl(Cl-C4), amino, dialkyl(C1-C4)amino, halogène, phényle, carboxy, trialkyl(C1-C4)ammonioalkyl(C1-C4), et
   X- est un anion organique ou minéral. On peut citer par exemple pour X- les ions Chlorure, Bromure, Iodure, méthosulfate, éthosulfate, mésylate, tosylate, acétate, les sels d'acide organique simple tel que les lactate, les oléate, les benzoate, les perchlorate, les triflate.
• Les Dimères de Stilbazolium de formules : 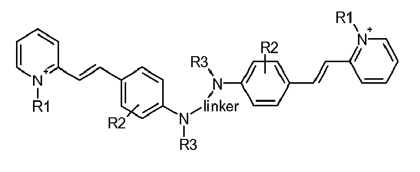 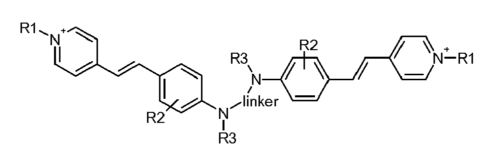 dans lesquelles R1, R2, R3 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène.
• Les Trimères et tétramères de Stilbazolium suivants 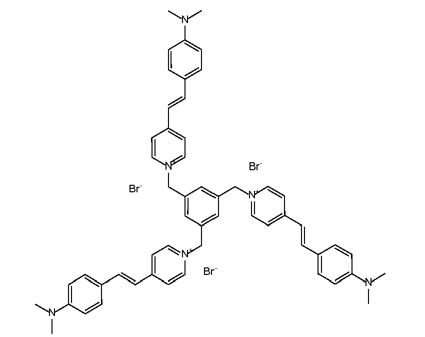 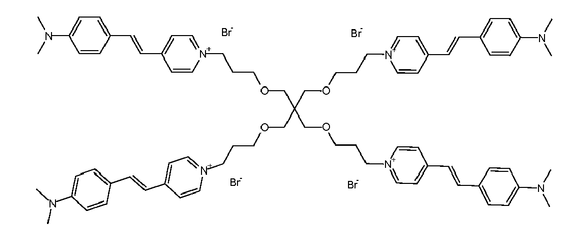
• Les dérivés Bis(BENZOXAZOLE) de formule : 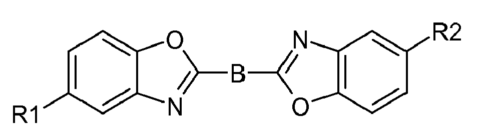 dans laquelle R1, R2 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène ; et
   B est choisi parmi 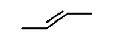 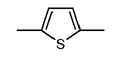 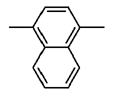 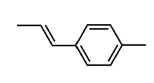
• Les Bis(BENZIMIDAZOLES) cationiques ou non
• Les 1,3-Diphenyl-2-PYRAZOLINES anioniques ou non, notamment 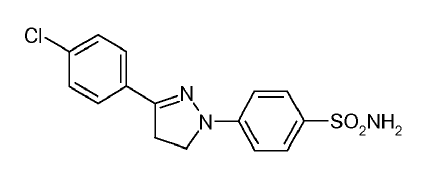
• Les DICETOPYRROLOPYRROLE de formule : 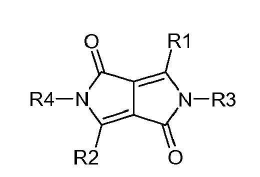 dans laquelle R1, R2, R3 et R4 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle; un groupement formyle; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle, amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène.
   On peut citer par exemple le composé suivant 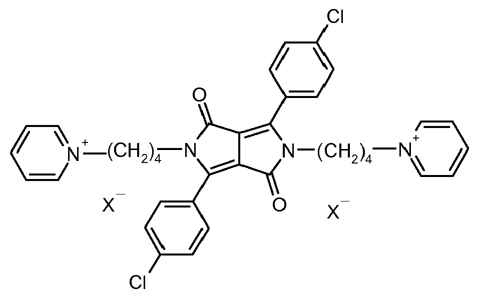 dans lequel X⁻ est un anion défini comme précédemment.
• Les dérivés de DICYANO PYRAZINES de formules : 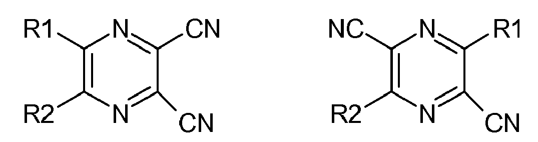 dans lesquelles R1, R2 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle; un groupement formyle; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène ;
   deux des substituants R1 et R2, peuvent former avec les atomes de carbone auxquels ils sont rattachés un cycle aromatique ou non, en C6-C30 ou hétérocyclique comportant au total de 5 à 30 chaînons et de 1 à 5 hétéroatomes ; ces cycles étant condensés ou non,insérant ou non un groupement carbonyle, et étant substitués ou non par un ou plusieurs groupements choisis parmi les groupements alkyle en C1-C4, alcoxy(C1-C4)alkyl(C1-C4), amino, dialkyl(C1-C4)amino, halogène, phényle, carboxy, trialkyl(C1-C4)ammonioalkyl(C1-C4) ;
   On peut citer les composés dans la publication suivante : « Selective topochemicalphotoreaction of crystallized 2,3-(-phenyletheny)-4,5-dicyanopyrazine" de Kim, Jae Hong; Matsuoka Masaru, Chem. Lett. (1999), (2), 143-144
   On peut notamment citer

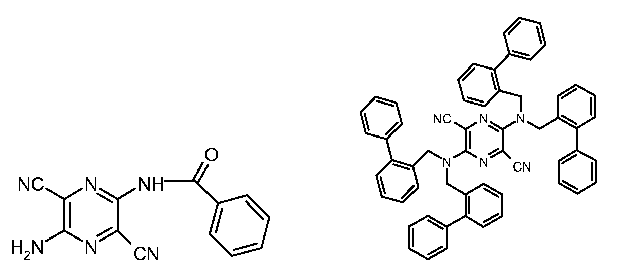

L'espèce colorée présente dans la composition de l'invention peut être un pigment.

Au sens de la présente invention, on entend par pigment toute entité organique et / ou minérale dont la solubilité dans l'eau est inférieure à 0,01 % à 20 °C, de préférence inférieure à 0,0001 %, et présentant une absorption entre 350 et 750 nm, de préférence une absorption avec un maximum.

Les pigments qui peuvent être utilisés sont notamment choisis parmi les pigments organiques et/ ou minéraux connus de la technique, notamment ceux qui sont décrits dans l'encyclopédie de technologie chimique de Kirk-Othmer et dans l'encyclopédie de chimie industrielle de Ullmann.

Ces pigments peuvent se présenter sous forme de poudre ou de pâte pigmentaire. Ils peuvent être enrobés ou non enrobés.

Les pigments peuvent par exemple être choisis parmi les pigments minéraux, les pigments organiques, les laques, les pigments à effets spéciaux tels que les nacres ou les paillettes, et leurs mélanges.

Le pigment peut être un pigment minéral. Par pigment minéral, on entend tout pigment qui répond à la définition de l'encyclopédie Ullmann dans le chapitre pigment inorganique. On peut citer, parmi les pigments minéraux utiles dans la présente invention, le dioxyde de titane, traité ou non traité en surface, les oxydes de zirconium ou de cérium, les oxydes de fer ou de chrome, le violet de manganèse, le bleu outremer, l'hydrate de chrome et le bleu ferrique. Par exemple, les pigments minéraux suivants peuvent être utilisés : Ta2O5, Ti3O5, Ti2O3, TiO, ZrO2 en mélange avec TiO2, ZrO2, Nb2O5, CeO2, ZnS.

Le pigment non traité en surface, appelé par la suite pigment, peut-être un pigment organique. Par pigment organique, on entend tout pigment qui répond à la définition de l'encyclopédie Ullmann dans le chapitre pigment organique. Le pigment organique peut notamment être choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

En particulier, les pigments organiques blancs ou colorés peuvent être choisis parmi le carmin, le noir de carbone, le noir d'aniline, le jaune azo, la quinacridone, le bleu de phtalocyanine, le rouge sorgho, les pigments bleus codifiés dans le Color Index sous les références Cl 42090, 69800, 69825, 73000, 74100, 74160, les pigments jaunes codifiés dans le Color Index sous les références Cl 11680, 11710, 15985, 19140, 20040, 21100, 21108, 47000, 47005, les pigments verts codifiés dans le Color Index sous les références Cl 61565, 61570, 74260, les pigments oranges codifiés dans le Color Index sous les réfénces Cl 11725, 15510, 45370, 71105, les pigments rouges codifiés dans le Color Index sous les références Cl 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

A titre d'exemple on peut aussi citer les pâtes pigmentaires de pigment organique telles que les produits vendus par la société HOECHST sous le nom :
- JAUNE COSMENYL lOG : Pigment YELLOW 3 (CI 11710) ;
- JAUNE COSMENYL G : Pigment YELLOW 1 (CI 11680) ;
- ORANGE COSMENYL GR : Pigment ORANGE 43 (CI 71105) ;
- ROUGE COSMENYL R : Pigment RED 4 (CI 12085) ;
- CARMIN COSMENYL FB : Pigment RED 5 (CI 12490) ;
- VIOLET COSMENYL RL : Pigment VIOLET 23 (CI 51319) ;
- BLEU COSMENYL A2R : Pigment BLUE 15.1 (CI 74160) ;
- VERT COSMENYL GG : Pigment GREEN 7 (CI 74260) ;
- NOIR COSMENYL R : Pigment BLACK 7 (CI 77266).

Les pigments conformes à l'invention peuvent aussi être sous forme de pigments composites tels qu'ils sont décrits dans le brevet EP 1 184 426. Ces pigments composites peuvent être composés notamment de particules comportant un noyau inorganique, au moins un liant assurant la fixation des pigments organiques sur le noyau, et au moins un pigment organique recouvrant au moins partiellement le noyau.

Le pigment organique peut aussi être une laque. Par laque, on entend les colorants adsorbés sur des particules insolubles, l'ensemble ainsi obtenu restant insoluble lors de l'utilisation.

Les substrats inorganiques sur lesquels sont adsorbés les colorants sont par exemple l'alumine, la silice, le borosilicate de calcium et de sodium ou le borosilicate de calcium et d'aluminium, et l'aluminium.

Parmi les colorants, on peut citer le carmin de cochenille. On peut également citer les colorants connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45 430), D & C Red 4 (CI 15 510), D & C Red 33 (CI 17 200), D & C Yellow 5 (CI 19 140), D & C Yellow 6 (CI 15 985), D & C Green (CI 61 570), D & C Yellow 1 O (CI 77 002), D & C Green 3 (CI 42 053), D & C Blue 1 (CI 42 090).

A titre d'exemples de laques, on peut citer le produit connu sous la dénomination suivante : D & C Red 7 (CI 15 850:1).

Le pigment peut aussi être un pigment à effets spéciaux. Par pigments à effets spéciaux, on entend les pigments qui créent d'une manière générale une apparence colorée (caractérisée par une certaine nuance, une certaine vivacité et une certaine clarté) non uniforme et changeante en fonction des conditions d'observation (lumière, température, angles d'observation...). Ils s'opposent par-là même aux pigments blancs ou colorés qui procurent une teinte uniforme opaque, semi-transparente ou transparente classique.

Il existe plusieurs types de pigments à effets spéciaux, ceux à faible indice de réfraction tels que les pigments fluorescents, photochromes ou thermochromes, et ceux à plus fort indice de réfraction tels que les nacres ou les paillettes.

A titre d'exemples de pigments à effets spéciaux, on peut citer les pigments nacrés blancs tels que le mica recouvert de titane, ou d'oxychlorure de bismuth, les pigments nacrés colorés tels que le mica recouvert de titane ou d'oxydes de fer, le mica recouvert de titane et notamment de bleu ferrique ou d'oxyde de chrome, le mica recouvert de titane et d'un pigment organique tel que défini précédemment ainsi que les pigments nacrés à base d'oxychlorure de bismuth. A titre de pigments nacrés, on peut citer les nacres Cellini commercialisée par Engelhard (Mica-TiO2-laque), Prestige commercialisée par Eckart (Mica-TiO2), Prestige Bronze commercialisée par Eckart (Mica-Fe2O3),Colorona commercialisée par Merck (Mica-TiO2-Fe2O3).

En plus des nacres sur un support mica, on peut envisager les pigments multicouches basés sur des substrats synthétiques comme l'alumine, la silice, le borosilicate de calcium et de sodium ou le borosilicate de calcium et d'aluminium, et l'aluminium.

On peut également citer les pigments à effet interférentiel non fixés sur un substrat comme les cristaux liquides (Helicones HC de Wacker), les paillettes holographiques interférentielles (Geometric Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques et les quantum dots, commercialisés par exemple par la société Quantum Dots Corporation.

Les quantum dots sont des nanoparticules semi conductrices luminescentes capables d'émettre, sous excitation lumineuse, un rayonnement présentant une longueur d'onde comprise entre 400 nm et 700 nm. Ces nanoparticules sont connues de la littérature. En particulier, elles peuvent être synthétisées selon les procédés décrits par exemple dans le US 6 225 198 ou US 5 990 479, dans les publications qui y sont citées, ainsi que dans les publications suivantes : Dabboussi B.O. et al "(CdSe)ZnS core-shell quantum dots : synthesis and characterisation of a size series of highly luminescent nanocristallites" Journal of phisical chemistry B, vol 101, 1997, pp 9463-9475. et Peng, Xiaogang et al, "Epitaxial Growth of highly Luminescent CdSe/CdS core/shell nanocrystals with photostability and electronic accessibility" Journal of the American Chemical Society, vol 119, N°30, pp 7019-7029.

La variété des pigments qui peuvent être utilisés dans la présente invention permet d'obtenir une riche palette de couleurs, ainsi que des effets optiques particuliers tels que des effets métalliques, interférentiels.

Selon un mode de réalisation particulier, les pigments sont des pigments colorés. On entend par pigment coloré des pigments autres que les pigments blancs.

La taille du pigment utilisé dans la composition cosmétique selon la présente invention est généralement comprise entre 10 nm et 200 µm, de préférence entre 20 nm et 80 µm, et plus préférentiellement entre 30 nm et 50 µm.

Les pigments peuvent être dispersées dans le produit grâce à un agent dispersant.

L'agent dispersant sert à protéger les particules dispersées contre leur agglomération ou floculation. Cet agent dispersant peut être un tensioactif, un oligomère, un polymère ou un mélange de plusieurs d'entre eux, portant une ou des fonctionnalités ayant une affinité forte pour la surface des particules à disperser. En particulier, ils peuvent s'accrocher physiquement ou chimiquement à la surface des pigments. Ces dispersants présentent, en outre, au moins un groupe fonctionnel compatible ou soluble dans le milieu continu. En particulier, on utilise les esters de l'acide hydroxy-12 stéarique en particulier et d'acide gras en C8 à C20 et de polyol comme le glycérol, la diglycérine, tel que le stéarate d'acide poly(12-hydroxystéarique) de poids moléculaire d'environ 750g/mole tel que celui vendu sous le nom de Solsperse 21 000 par la société Avecia, le polygycéryl-2 dipolyhydroxystéarate (nom CTFA) vendu sous la référence Dehymyls PGPH par la société Henkel ou encore l'acide polyhydroxystéarique tel que celui vendu sous la référence Arlacel P100 par la société Uniqema et leurs mélanges.

Comme autre dispersant utilisable dans les compositions de l'invention, on peut citer les dérivés ammonium quaternaire d'acides gras polycondensés comme le Solsperse 17 000 vendu par la société Avecia, les mélanges de poly diméthylsiloxane/oxypropylène tels que ceux vendus par la société Dow Corning sous les références DC2-5185, DC2-5225 C.

L'acide polydihydroxystéarique et les esters de l'acide hydroxy12-stéarique sont de préférence destinés à un milieu hydrocarboné ou fluoré, alors que les mélanges de polydiméthylsiloxane oxyéthylène/oxypropyléné sont de préférence destinés à un milieu siliconé.

Les pigments utilisés dans la composition cosmétique selon l'invention peuvent être traités en surface par un agent organique.

Ainsi les pigments préalablement traités en surface utiles dans le cadre de l'invention sont des pigments ou des charges qui ont subi totalement ou partiellement un traitement de surface de nature chimique, électronique, électro-chimique, mécano-chimique ou mécanique, avec un agent organique tel que ceux qui sont décrits notamment dans Cosmetics and Toiletries, Février 1990, Vol. 105, p. 53-64 avant d'être dispersés dans la composition conforme à l'invention. Ces agents organiques peuvent être par exemple choisis parmi les acides aminés ; les cires, par exemple la cire de carnauba et la cire d'abeille ; les acides gras, les alcools gras et leurs dérivés, tels que l'acide stéarique, l'acide hydroxystéarique, l'alcool stéarylique, l'alcool hydroxystéarylique, l'acide laurique et leurs dérivés ; les tensio-actifs anioniques ; les lécithines ; les sels de sodium, potassium, magnésium, fer, titane, zinc ou aluminium d'acides gras, par exemple le stéarate ou laurate d'aluminium ; les alcoxydes métalliques ; les polysaccharides, par exemple le chitosane, la cellulose et ses dérivés ; le polyéthylène ; les polymères (méth)acryliques, par exemple les polyméthylmethacrylates ; les polymères et copolymères contenant des motifs acrylates ; les protéines ; les alcanoamines ; les composés siliconés, par exemple les silicones, les polydiméthylsiloxanes, les alcoxysilanes, les alkylsilanes, les siloxysilicates ; les composés organiques fluorés, par exemple les perfluoroalkyle éthers; les composés fluoro-siliconés.

Les pigments traités en surface utiles dans la composition cosmétique selon l'invention peuvent aussi avoir été traités par un mélange de ces composés et / ou avoir subi plusieurs traitements de surface.

Les pigments traités en surface utiles dans le cadre de la présente invention peuvent être préparés selon des techniques de traitement de surface bien connues de l'homme de l'art ou trouvés tels quels dans le commerce.

De préférence, les pigments traités en surface sont recouverts par une couche organique.

L'agent organique avec lequel sont traités les pigments peut être déposé sur les pigments par évaporation de solvant, réaction chimique entre les molécules de l'agent de surface ou création d'une liaison covalente entre l'agent de surface et les pigments. Le traitement en surface peut ainsi être réalisé par exemple par réaction chimique d'un agent de surface avec la surface des pigments et création d'une liaison covalente entre l'agent de surface et les pigments ou les charges. Cette méthode est notamment décrite dans le brevet US 4 578 266.

De préférence, on utilisera un agent organique lié aux pigments de manière covalente.

L'agent pour le traitement de surface peut représenter de 0,1 à 50 % en poids du poids total des pigments traités en surface, de préférence de 0,5 à 30 % en poids, et encore plus préférentiellement de 1 à 10 % en poids.

De préférence, les traitements en surface des pigments sont choisis parmi les traitements suivants :
- un traitement PEG-Silicone comme le traitement de surface AQ commercialisé par LCW ;
- un traitement Chitosane comme le traitement de surface CTS commercialisé par LCW;
- un traitement Triéthoxycaprylylsilane comme le traitement de surface AS commercialisé par LCW ;
- un traitement Méthicone comme le traitement de surface SI commercialisé par LCW;
- un traitement Diméthicone comme le traitement de surface Covasil 3.05 commercialisé par LCW ;
- un traitement Diméthicone / Triméthylsiloxysilicate comme le traitement de surface Covasil 4.05 commercialisé par LCW ;
- un traitement Lauroyl Lysine comme le traitement de surface LL commercialisé par LCW ;
- un traitement Lauroyl Lysine Diméthicone comme le traitement de surface LL / SI commercialisé par LCW ;
- un traitement Myristate de Magnésium comme le traitement de surface MM commercialisé par LCW ;
- un traitement Dimyristate d'Aluminium comme le traitement de surface MI commercialisé par Miyoshi ;
- un traitement Perfluoropolyméthylisopropyl éther comme le traitement de surface FHC commercialisé par LCW ;
- un traitement Isostéaryl Sébacate comme le traitement de surface HS commercialisé par Miyoshi ;
- un traitement Disodium Stéaroyl Glutamate comme le traitement de surface NAI commercialisé par Miyoshi ;
- un traitement Diméthicone / Disodium Stéaroyl Glutamate comme le traitement de surface SA / NAI commercialisé par Miyoshi ;
- un traitement Phosphate de Perfluoroalkyle comme le traitement de surface PF commercialisé par Daito ;
- un traitement Copolymère acrylate / Diméthicone et Phosphate de Perfluoalkyle comme le traitement de surface FSA commercialisé par Daito ;
- un traitement Polyméthylhydrogène siloxane / Phosphate de Perfluoroalkyle comme le traitement de surface FS01 commercialisé par Daito ;
- un traitement Lauryl Lysine / Aluminium Tristéarate comme le traitement de surface LL-StAl commercialisé par Daito ;
- un traitement Octyltriéthylsilane comme le traitement de surface OTS commercialisé par Daito ;
- un traitement Octyltriéthylsilane / Phosphate de Perfluoroalkyle comme le traitement de surface FOTS commercialisé par Daito ;
- un traitement Copolymère Acrylate / Diméthicone comme le traitement de surface ASC commercialisé par Daito ;
- un traitement Isopropyl Titanium Triisostéarate comme le traitement de surface ITT commercialisé par Daito ;
- un traitement Cellulose Microcrystalline et Carboxyméthyl Cellulose comme le traitement de surface AC commercialisé par Daito ;
- un traitement Cellulose comme le traitement de surface C2 commercialisé par Daito ;
- un traitement copolymère Acrylate comme le traitement de surface APD commercialisé par Daito ;
- un traitement Phosphate de Perfluoroalkyle / Isopropyl Titanium Triisostéarate comme le traitement de surface PF + ITT commercialisé par Daito.

La composition conforme à la présente invention peut de plus comprendre un ou plusieurs pigments non traités en surface.

A titre d'espèces colorantes, on peut notamment citer les précurseurs de colorants tels que les bases d'oxydation et les coupleurs. Parmi les bases d'oxydation classiquement utilisées en teinture d'oxydation, on peut notamment citer les paraphénylènediamines, les bis-phénylalkylènediamines, les paraaminophénols, les ortho-aminophénols et les bases hétérocycliques. Parmi les coupleurs, on peut notamment citer les métaphénylènediamines, les méta-aminophénols, les métadiphénols, les naphtols et les coupleurs hétérocycliques tels que par exemple les dérivés indoliques, les dérivés indoliniques, les dérivés pyridiniques, les dérivés indazoliques, les dérivés de pyrazolo[1,5-b]-1,2,4-triazole, les dérivés de pyrazolo[3,2-c]-1,2,4-triazole, les dérivés de benzimidazole, les dérivés de benzothiazole, les dérivés de benzoxazole, les dérivés de 1,3-benzodioxole et les pyrazolones, et leurs sels d'addition avec un acide.

La concentration en espèce colorée ou colorante dans la composition est comprise entre 0.01 et 50%, de préférence entre 0.5 et 20%.

Selon un mode de réalisation particulier, l'espèce colorée est un pigment.

La composition de l'invention contient au moins un composé siliconé présentant une viscosité inférieure ou égale à 100 centistokes (cst), viscosité mesurée à 25°C. Un tel composé siliconé à faible viscosité peut être choisi parmi les silicones linéaires ou cycliques ayant de 2 à 7 atomes de silicium, ces silicones comportant éventuellement des groupes alkyle ou alkoxy ayant de 1 à 10 atomes de carbone, par exemple l'octaméthylcyclotétrasiloxane, le décaméthylcyclopentasiloxane, le dodécaméthylcyclohexasiloxane, l'heptaméthylhexyltri-siloxane, l'heptaméthyléthyltrisiloxane, l'heptaméthyloctyltrisiloxane, l'octaméthyltrisiloxane, le décaméthyltétrasiloxane, et leurs mélanges. Selon un mode de réalistaion particulier, le composé siliconé est choisi parmi le cyclopentadiméthylsiloxane et le dodécaméthylcyclohexasiloxane.

Selon un mode de réalisation particulier, le composé siliconé présente une viscosité inférieure à 50 centistokes.

Le composé siliconé de viscosité inférieure à 100 cst peut être présent dans la composition selon l'invention en une teneur allant de 0,1 % à 90 % en poids, par rapport au poids total de la composition, de préférence allant de 1 % à 80 % en poids, et préférentiellement allant de 5 % à 70 % en poids.

La composition de l'invention peut de plus contenir d'autres solvants organiques non volatils tels que :
- les alcools aromatiques non volatils tels que l'alcool benzylique, le phenoxyéthanol
- les esters d'acides en C1-C20 liquides et d'alcools en C1-C8 non volatils tels que le myristate d'isopropyle ;
- l'éthylène carbonate, le propylène carbonate; le butylène carbonate ;
- les polyols non volatils tels que le glycérol, l'éthylène glycol, le dipropylène glycol, le butylène glycol,
- les éthers de glycol non volatils comme le monoéhyléther de diéthylène glycol, le mono n-butyl éther de dipropylène glycol.
- les huiles hydrocarbonées non volatiles telles que l'isohexadécane.
- Les alcools gras liquides non volatils en C10-C30 tels que l'alcool oléique, les esters d'alcools gras en C10-C30 liquides tels que les benzoates d'alcool gras en C10-C30 et leurs mélanges ; l'huile de polybutène, l'isononanoate d'isononyle, le malate d'isostéaryle, le tétra-isostéarate de pentaérythrityle, le trimélate de tridécyle
- Les solvants perfluorés non volatils tels que le perfluoroperhydrophenanthrene, vendu sous la dénomination de "Flutec PC11®" par la Société F2 Chemicals

La composition peut aussi contenir des charges qui sont généralement des composés substantiellement non colorés solides à température ambiante et pression atmosphérique, et insoluble dans les différents ingrédients de la composition, même lorsque ces ingrédients sont portés à une température supérieure de la température ambiante.

Les charges peuvent être minérales ou organiques. Les charges peuvent être des particules de toute forme, notamment plaquettaires, sphériques ou oblongues, quelle que soit leur forme cristallographique (par exemple feuillet, cubique, hexagonale, orthorhombique). De plus ces particules peuvent être pleines, creuses ou poreuses, enrobées ou non.

Parmi les charges utilisables dans les compositions selon l'invention, on peut notamment citer des charges minérales telles que le talc ; le mica naturel ou synthétique ; la silice ; le kaolin ; le nitrure de bore, le carbonate de calcium précipité; le carbonate et l'hydro-carbonate de magnésium ; l'hydroxyapatite.

Ces charges minérales peuvent se présenter sous la forme de particules sphériques avec par exemple les microsphères de silice creuses telles que les 'SILICA BEADS SB 700/HA®' ou 'SILICA BEADS SB 700®' de la société MAPRECOS, les 'SUNSPHERES H-33®' et les 'SUNSPHERES H-51®' de la société ASAHI GLASS.

Avantageusement, la ou les particules inorganiques présentent une taille primaire moyenne en nombre comprise entre 0,1 et 30 µm, de préférence comprise entre 0,2 et 20 µm, et encore plus préférentiellement comprise entre 0,5 et 15 µm. Au sens de la présente invention, on entend par « taille primaire de particule », la dimension maximale qu'il est possible de mesurer entre deux points diamétralement opposés d'une particule individuelle. La taille des particules organiques peut être déterminée par microscopie électronique à transmission ou à partir de la mesure de la surface spécifique par la méthode BET ou à partir d'une granulométrie laser.

De préférence les charges inorganiques utilisées selon l'invention sont la silice, le talc, le nitrure de bore.

Parmi les charges utilisables dans les compositions selon l'invention, on peut notamment citer des charges organiques. Par charge organique, on entend une particule polymérique qui peut être issue de la polymérisation d'un ou de plusieurs monomères. Les polymères constituant ces particules organiques peuvent être réticulés ou non. Les monomères utilisés peuvent être en particulier des esters d'acide méthacrylique ou acrylique, tels que l'acrylate et méthacrylate de méthyle, le chlorure de vinylidène, l'acrylonitrile, le styrène et ses dérivés.

Avantageusement, la ou les particules organiques présentent une taille primaire moyenne en nombre comprise entre 1 et 30 µm, de préférence comprise entre 1 et 20 µm, et encore plus préférentiellement comprise entre 1 et 15 µm.

La ou les particules organiques utilisées dans la composition cosmétique selon l'invention peuvent être choisies parmi les poudres de polyamide, les poudres de polymères acryliques, notamment de polyméthacrylate de méthyle, les poudres de copolymères acryliques, notamment de polyméthacrylate de méthyle/diméthacrylate d'éthylène glycol, de polyméthacrylate d'allyle/diméthacrylate d'éthylène glycol, de copolymère diméthacrylate d'éthylène glycol/méthacrylate de lauryle, de polyacrylate/alkylacrylate, les poudres de polystyrène, les poudres de polyéthylène, notamment de polyéthylène/acide acrylique, les microbilles de résine de silicone.

A titre représentatif et non limitatif, on peut particulièrement citer en tant que particules organiques selon l'invention :
- les poudres de polyamides (Nylon ®), par exemples celles commercialisées sous les dénominations « ORGASOL ® 4000 » et « ORGASOL ® 2002 UD NAT COS 204 » par la société ATOCHEM,
- les poudres de polymères acryliques, notamment de polyméthacrylate de méthyle, comme par exemples celles commercialisées sous la dénomination « COVABEAD ® LH85 » « COVABEAD ® PMMA »par la société WACKHERR ou celles commercialisées sous la dénomination « MICROPEARL ® MHB » commercialisée par la société MATSUMOTO,
- les poudres de copolymères acryliques, notamment de polyméthacrylate de méthyle/diméthacrylate d'éthylène glycol, comme celles commercialisées sous la dénomination de « DOW CORNING 5640 MICROSPONGE ® SKIN OIL ADSORBER » par la société DOW CORNING, ou celles commercialisés sous la dénomination « GANZPEARL ® GMP-0820 » par la société GANZ CHEMICAL, de polyméthacrylate d'allyle/diméthacrylate d'éthylène glycol, comme celles commercialisées sous la dénomination « POLYPORE ® L200 » ou « POLYPORE ® E200 » commercialisés par la société AMCOL, de copolymère diméthacrylate d'éthylène glycol / méthacrylate de lauryle, comme celles commercialisées par « POLYTRAP ® 6603 » par la société DOW CORNING, de polyacrylate/éthylhexylacrylate comme celles commercialisées sous la dénomination « TECHPOLYMER ® ACX 806C » par la société SEKISUI,
- les poudres de polystyrène / dinvinylbenzène comme celles commercialisées sous la dénomination « TECHPOLYMER ® SBX8 » par la société SEKISUI,
- les poudres de polyéthylène, notamment de polyéthylène/acide acrylique commercialisées sous la dénomination « FLOBEADS ® » par la société SUMITOMO,
- les microbilles de résine de silicone, comme celles commercialisées sous les dénominations « TOSPEARL ® » par la société TOSHIBA SILICONE, en particulier les « TOSPEARL ® 240A » et « TOSPEARL ® 120A ».
- les microsphères de polymères acryliques telles que celles en copolymère d'acrylate réticulé 'POLYTRAP 6603 ADSORBER®' de la société RP SCHERRER
- les poudres de polyuréthane telle que la poudre de copolymère de diisocyanate d'hexaméthylène et de triméthylol hexyl lactone vendue sous la dénomination « PLASTIC POWDER D-400® » par la société Toshiki
- les microcapsules de polymères ou de copolymères d'acrylate ou de méthacrylate de méthyle, ou encore de copolymères de chlorure de vinylidène et d'acrylonitrile, comme l' « EXPANCEL® » de la société EXPANCEL
- les poudres d'organopolysiloxane réticulés élastomères telles que celles vendues sous la dénomination « TREFIL POWDER E-506C » par la société DOW CORNING
- les poudres polyfluorées notamment de polytétrafluoroéthylène, par exemple celle commercialisée sous la dénomination "MP 1400" par la Société DUPONT DE NEMOURS,

De préférence, les particules organiques utilisées dans la composition conforme à l'invention sont choisies parmi les poudres de polyamide et les poudres de polyméthylméthacrylate.

Les espèces colorées ou colorantes ainsi que les charges peuvent être présents dans la composition en quantité comprise entre 0,001 et 20 % en poids environ du poids total de la composition, notamment entre 0,1 et 10 %.

Afin d'obtenir un meilleur étalement de la composition de l'invention ainsi qu'un gainage amélioré, la composition de l'invention peut aussi contenir un ou plusieurs polysiloxanes présentant une viscosité supérieure à 100 cst, préférentiellement supérieure à 300 cst. La viscosité de ces polysiloxanes peut être mesurée selon la norme ASTM D-445. De tels polysiloxanes peuvent être des huiles, des gommes ou des résines de silicone, les silicones greffées, les silicones réticulées.

A titre de polysiloxanes de viscosité supérieure à 100 cst, on peut notamment citer les polydiméthylsiloxanes; les alkyldiméthicones; les polyphénylméthylsiloxanes tels que les phényldiméthicones, les phényltriméthicones, et les vinylméthylméthicones; ainsi que les silicones modifiées par des groupements aliphatiques et/ou aromatiques, éventuellement fluorés, ou par des groupements fonctionnels tels que des groupements hydroxyles, thiols et/ou amines.

De tels polysiloxanes peuvent choisis parmi les silicones de formule (I): 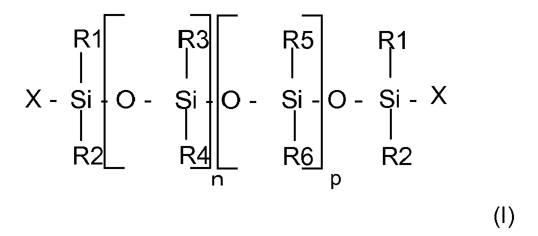 dans laquelle :
R₁, R₂, R₅ et R₆ sont, ensemble ou séparément, un radical alkyle ayant 1 à 6 atomes de carbone, R₃ et R₄ sont, ensemble ou séparément, un radical alkyle ayant de 1 à 6 atomes de carbone, un radical vinyle, un radical aryle, un radical amine, un radical hydroxyle, X est un radical alkyle ayant de 1 à 6 atomes de carbone, un radical hydroxyle, un radical vinyle, un radical amine, n et p étant des entiers choisis de manière à obtenir une viscosité supérieure à 300 cst.

A titre d'exemple, on peut citer les polydiméthylsiloxanes suivantes :
o les substituants R₁ à R₆ et X représentent un groupement méthyle, comme celle vendue sous la dénomination Baysilicone TP 3898 par la société Général Electric, et
celle vendue sous la dénomination AK 500000 par la société Waker,
o les substituants R₁ à R₆ et X représentent un groupement méthyle, p et n sont tels que le poids moléculaire est de 120 000 g/mol, comme celle vendue sous la dénomination Dow Corning 200 Fluid 60000 CS par la société Dow Corning.
o les substituants R₁ à R₆ et X représentent un groupement méthyle, p et n sont tels que le poids moléculaire est de 250 000 g/mol, comme celle vendue sous la dénomination Mirasil DM 500.000 par la société Rhodia et celle vendue sous la dénomination Dow Corning 200 Fluid 500.000 cst par la société Dow Corning.
o les substituants R₁ à R₆ représentent un groupement méthyle, le groupement X représente un groupement hydroxy, n et p sont tels que le poids moléculaire du polymère soit de 600 000 g/mol, comme celle vendue sous la dénomination SGM 36 par la société Dow Corning.
o Les diméthicones du type (polydiméthylsiloxane)(méthylvinylsiloxane) telle que la SE63 commercialisée par GE BAYER Silicones, les copolymères poly(diméthylsiloxane)(diphényl)(méthylvinylsiloxane), et leurs mélanges.

Dans le cas où le polysiloxane comprend un groupement fluoré, on peut choisir les copolymères de structure suivante : 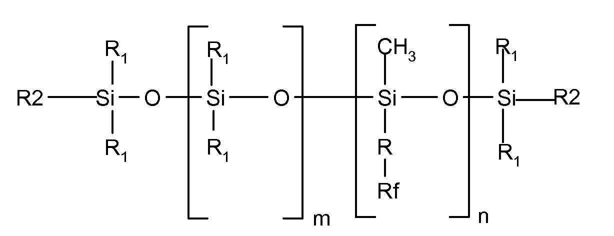 dans laquelle :
R représente un groupement divalent alkyle linéaire ou ramifié, ayant 1 à 6 atomes de carbone, de préférence un groupement divalent méthyle, éthyle, propyle ou butyle, Rf représente un radical fluoroalkyle, notamment un radical perfluoroalkyle, ayant 1 à 12 atomes de carbone, de préférence 1 à 9 atomes de carbone, R₁ représente, indépendamment l'un de l'autre, un radical alkyle en C₁-C₂₀, un radical hydroxyle, un radical phényle, R₂ représente R₁ ou R_{f}
   - m est choisi de 0 à 500, de préférence de 0 à 200, et n est choisi de 1 à 1000, de préférence de 1 à 500.

De préférence, les groupements R1 sont identiques et représentent un radical méthyle.

De tels polysiloxanes sont notamment ceux commercialisés par la société Shin Etsu sous les dénominations 'FL-5', 'FL-10', 'X22-821' et 'X22-822' ou encore 'FL-100', par la société Dow Corning sous le nom FS-1265 Fluid, par la société Phoenix Chemical sous la gamme Pecosil FS sous les dénominations Pecosil FSL-150, Pecosil FSL-300, Pecosil FSH-150, Pecosil FSH-300, Pecosil FSU-150, Pecosil FSU-300.

La masse moléculaire en poids du ou des polysiloxane peut être comprise entre 1000 et 1 500 000 g/mol, notamment entre 20 000 et 1 000 000 g/mol.

Le polysiloxane peut être sous forme de résine. Par le terme « résine », on entend une structure tridimensionnelle réticulée ou non. A titre d'exemple de résine de polysiloxane, on peut citer les silsesquioxanes et les siloxysilicates.

La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.

La lettre M représente l'unité monofonctionelle de formule (CH₃ₕSiO_{1/2}, l'atome de silicium étant relié à un seul atome d'oxygène dans le polymère comprenant cette unité.

La lettre D signifie une unité difonctionnelle (CH₃)₂SiO_{2/2} dans laquelle l'atome de silicium est relié à deux atomes d'oxygène

La lettre T représente une unité trifonctionnelle de formule (CH₃)SiO_{3/2}

Dans les motifs M, D, T définis précédemment, au moins un des groupes méthyles peut être substitués par un groupe R différent du groupe méthyle tel qu'un radical hydrocarboné (notamment alkyle) ayant de 2 à 10 atomes de carbone ou un groupe phényl ou bien encore un groupe hydroxyle.

Enfin, la lettre Q signifie une unité tetrafonctionnelle SiO_{4/2} dans laquelle l'atome de silicium est lié à quatre atomes d'oxygène eux mêmes liés au reste du polymère.

Divers résines de propriétés différentes peuvent être obtenues à partir de ces différentes unités, les propriétés des ces polymères variant en fonction du type de monomères (ou unités), du type et du nombre de radicaux subsitués, de la longueur de la chaîne polymérique, du degré de ramification et de la taille des chaines pendantes.

A titre d'exemple de ces résines silicones, on peut citer :
- les siloxysilicates qui peuvent être des triméthylsiloxysilicate de formule (XXI) :

   [(CH₃)₃-SI-O]ₓ-(SiO_{4/2})_{y} (XXI)

   (unités MQ) dans laquelle x et y sont des entiers allant de 50 à 80,
- les polysilesquioxanes de formule (CH₃SiO_{3/2})_{.x} (unités T) dans laquelle x est supérieur à 100 et dont au moins un des radicaux méthyle peut être substitué par un groupement R tel que défini plus haut,
- les polymethylsilsesquioxanes qui sont des polysilsesquioxanes dans lesquels aucun des radicaux méthyle n'est substitué par un autre groupement. De tels polymethylsilsesquioxanes sont décrits dans le document US 5,246,694 dont le contenu est incorporé par référence.
   A titre d'exemples de résines polymethylsilsesquioxanes commercialement disponibles, on peut citer celles qui sont commercialisés :
- par la société WACKER sous la référence RESIN MK tels que la BELSIL PMS MK : polymère comprenant des unités répétitives CH₃SiO_{3/2} (unités T), pouvant aussi comprendre jusqu'à 1 % en poids d'unités (CH₃)₂SiO_{2/2} (unités D) et présentant un poids moléculaire moyen d'environ 10000,
- par la société SHIN-ETSU sous les références KR-220L qui sont composé d'unités T de formule CH₃SiO_{3/2} et ont des groupes terminaux Si-OH (silanol), sous la référence KR-242A qui comprennent 98% d'unités T et 2% d'unités diméthyle D et ont des groupes terminaux Si-OH ou encore sous la référence KR-251 comprenant 88 % d'unités T et 12 % d'unités dimethyl D et ont des groupes terminaux Si-OH.

Comme résines siloxysilicates, on peut citer les résines trimethylsiloxysilicate (TMS) éventuellement sous forme de poudres. De telles résines sont commercialisées sous la référence SR1000 par la société GENERAL ELECTRIC ou sous la référence TMS 803 par la société WACKER. On peut encore citer les résines timéthylsiloxysilicate commercialisées dans un solvant tel que la cyclomethicone, vendues sous la dénomination "KF-7312J" par la société SHIN-ETSU, "DC 749", "DC 593" par la société DOW CORNING.

Dans un mode de réalisation de l'invention, les polysiloxanes utiles dans la composition de l'invention sont solubles ou dispersables dans la composition de l'invention. Dans un mode de réalisation, la résine de silicone est solide à 25°C.

La composition de l'invention peut aussi contenir un polymère siliconé greffé. Dans le cadre de l'invention, on entend par polymère siliconé greffé, un polymère comprenant une portion polysiloxane et une portion constituée d'une chaîne organique non-siliconée, l'une des deux portions constituant la chaîne principale du polymère l'autre étant greffée sur ladite chaîne principale.

Les polymères siliconés greffés utilisés dans la composition cosmétique selon l'invention sont choisis préférentiellement dans le groupe constitué par les polymères à squelette organique non siliconé greffé par des monomères contenant un polysiloxane, les polymères à squelette polysiloxanique greffé par des monomères organiques non siliconés et leurs mélanges.

Les monomères organiques non siliconés constituant la chaîne principale du polymère siliconé greffé peuvent être choisis parmi des monomères à insaturation éthylénique polymérisables par voie radicalaire, des monomères polymérisables par polycondensation tels que ceux formant des polyamides, des polyesters, des polyuréthanes, des monomères à ouverture de cycle tels que ceux du type oxazoline
ou caprolactone.

Les polymères à squelette organique non-siliconé greffé par des monomères contenant un polysiloxane, conformes à l'invention, peuvent être choisis parmi ceux décrits dans les brevets US 4,693,935, US 4,728,571 et US 4,972,037 et les demandes de brevet EP-A-0 412 704, EP-A-0 412 707,EP-A-0 640 105 et WO 95/00578. Il s'agit de copolymères obtenus par polymérisation radicalaire à partir de monomères à insaturation éthylénique et de macromères siliconés ayant un groupe vinylique terminal ou bien des copolymères obtenus par réaction d'une polyoléfine comprenant des groupes fonctionnalisés et d'un macromère polysiloxane ayant une fonction terminale réactive avec lesdits groupes fonctionnalisés.

Une famille particulière de polymères siliconés greffés convenant pour la réalisation de la présente invention est constituée par les copolymères greffés siliconés comprenant :
a) de 0,01 à 50 % en poids d'au moins un macromère polysiloxane (C) de formule générale :

   X(Y)ₙSi(R)₃₋ₘ Zₘ (VI)

   et
b) de 0 à 98% en poids d'au moins un monomère (A) lipophile de faible polarité lipophile à insaturation éthylénique, polymérisable par voie radicalaire ;
   et/ou
c) de 0 à 98% en poids d'au moins un monomère (B) hydrophile polaire à insaturation éthylénique, copolymérisable avec le ou les monomères du type (A) ;
où :
X désigne un groupe vinylique copolymérisable avec les monomères (A) et (B) ;
Y désigne un groupe de liaison divalent ;
R désigne un hydrogène, un alkyle ou un alcoxy en C₁-C₆, un aryle C₆-C₁₂ ;
Z désigne un motif polysiloxane monovalent ayant un poids moléculaire moyen en nombre d'au moins 500 ;
n est 0 ou 1 et m est un entier allant de 1 à 3 ; les pourcentages étant calculés par rapport au poids total des monomères (A), (B) et (C).

Ces polymères ont un poids moléculaire moyen en nombre de préférence allant de 10.000 à 2.000.000 et de préférence une température de transition vitreuse Tg ou une température de fusion cristalline Tm d'au moins -20°C.

On peut citer comme exemples de monomères lipophiles (A), les esters d'acide acrylique ou méthacrylique d'alcools en C₁-C₂₄ ; le styrène ; les macromères polystyrène ; l'acétate de vinyle ; le propionate de vinyle ; l'alpha-méthylstyrène ; le tertio-butylstyrène ; le butadiène ; le cyclohexadiène ; le cyclohexadiène ; l'éthylène ; le propylène ; le vinyltoluène ; les esters d'acide acrylique ou méthacrylique et de 1,1-dihydroperfluoroalcanols ou de leurs homologues ; les esters d'acide acrylique ou méthacrylique et de oméga-hydrydofluroalcanols ; les esters d'acide acrylique ou méthacrylique et de fluoroalkylsulfoamido-alcools ; les esters d'acide acrylique ou méthacrylique et d'alcools fluoroalkyliques ; les esters d'acide acrylique ou méthacrylique et de fluroéthers d'alcools; ou leurs mélanges. Les monomères (A) préférentiels sont choisis dans le groupe constitué par le méthacrylate de n-butyle, le méthacrylate d'isobutyle, le méthacrylate de stéaryle, l'acrylate de tertio-butyle, le méthacrylate de tertio-butyle, le méthacrylate de 2-éthylhexyle, le méthacrylate de méthyle, le 2-(N-méthyl perfluro-octane sulfonamido)-éthylacrylate ; le 2-(N-butylperflurooctane sulfonamido)-éthyl-acrylate, l'heptadacefluorooctylméthylamino-éthylmethacrylate ou leurs mélanges.

On peut citer comme exemples de monomères polaires (B), l'acide acrylique, l'acide méthacrylique, le N,N-diméthylacrylamide, le méthacrylate de diméthylaminoéthyle, le méthacrylate de diméthylaminoéthyle quaternisé, le (méth)acrylamide, le N-t-butyl-acrylamide, l'acide maléique, l'anhydride maléique et leurs demi-esters, les (méth) acrylates hydroxyalkylés, le chlorure de diallyldiméthylammonium, la vinyl-pyrrolidone, les éthers de vinyle, les maléimides, la vinylpyridine, le vinylimidazole, les composés polaires vinyliques hétérocycliques, le styrène sulfonate, l'alcool allylique, l'alcool vinylique, le vinyl caprolactame ou leurs mélanges. Les monomères (B) préférentiels sont choisis dans le groupe constitué par l'acide acrylique, le N,N-diméthylacrylamide, le méthacrylate de diméthylaminoéthyle, le méthacrylate de diméthylaminoéthyle quaternisé, la vinylpyrrolidone et leurs mélanges.

Les macromères polysiloxane (C) de formule (I) préférentiels sont choisis parmi ceux répondant à la formule générale suivante (VII): 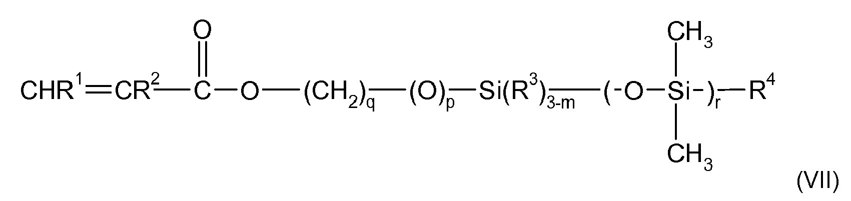 dans laquelle :
o R¹ est hydrogène ou -COOH et de préférence l'hydrogène ;
o R² est hydrogène, méthyle ou -CH₂COOH (de préférence méthyle) ;
o R³ est alkyle, alcoxy ou alkylamino en C₁-C₆, aryle en C₆-C₁₂ ou hydroxyle (de
préférence méthyle) ;
o R⁴ est alkyle, alcoxy ou alkylamino en C₁-C₆, aryle en C₆-C₁₂ ou hydroxyle (de
préférence méthyle) ;
o q est un entier de 2 à 6 (de préférence 3);
o p est 0 ou 1 ;
o r est un nombre entier de 5 à 700;
o m est un entier allant de 1 à 3 (de préférence 1) ;
de préférence p=0.

On utilise plus particulièrement les macromères polysiloxanes de formule : 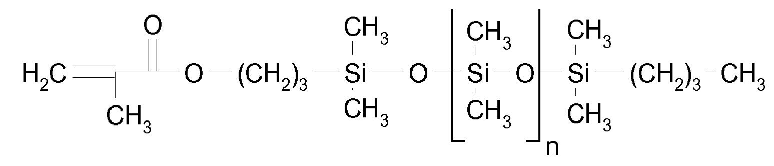 avec n étant un nombre allant de 5 à 700.

Le copolymère à squelette organique non-siliconé greffé par des monomères contenant un polysiloxane peut par exemple avoir la structure suivante :

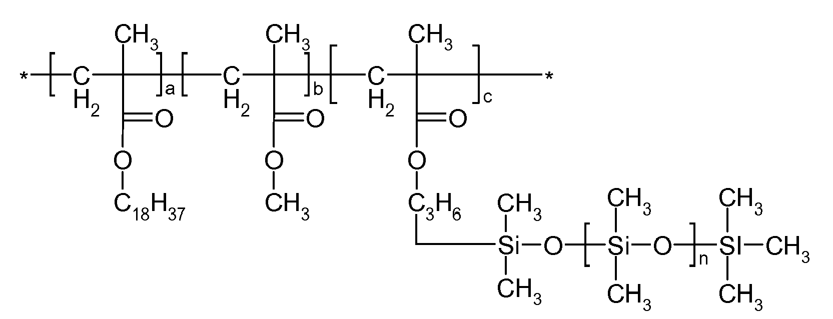

Un tel polymère est commercialisé sous le nom KP 561 commercialisés par Shin Etsu.

Le copolymère à squelette organique non-siliconé greffé par des monomères contenant un polysiloxane peut aussi avoir la structure suivante : 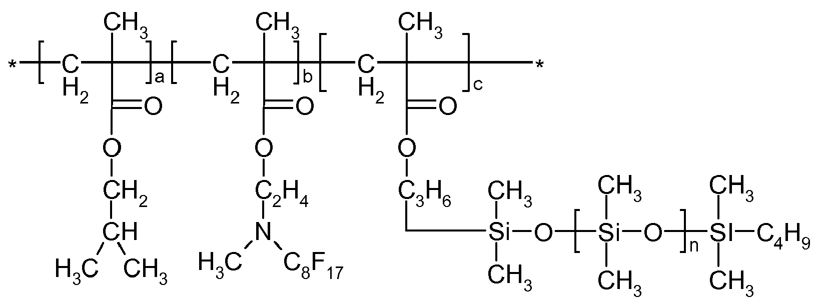 Un tel polymère, le Polysilicone 7, est commercialisé sous le nom SA70 par 3M.

D'autres copolymères à squelette organique non-siliconé greffé par des monomères contenant un polysiloxane peuvent aussi être le KP545, le KP574 et le KP575 commercialisés par Shin Etsu.

Un mode particulier de réalisation de l'invention consiste à utiliser un copolymère susceptible d'être obtenu par polymérisation radicalaire à partir du mélange de monomères constitué par :
a) 60% en poids d'acrylate de tertiobutyle ;
b) 20% en poids d'acide acrylique ;
c) 20% en poids de macromère siliconé de formule : 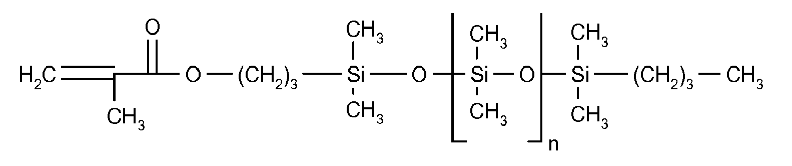 avec n étant un nombre allant de 5 à 700 ; les pourcentages en poids étant calculés par rapport au poids total des monomères.

Un autre mode particulier de réalisation de l'invention consiste à utiliser un copolymère susceptible d'être obtenu par polymérisation radicalaire à partir du mélange de monomères constitué par :
a) 80% en poids d'acrylate de tertiobutyle ;
b) 20% en poids de macromère siliconé de formule : 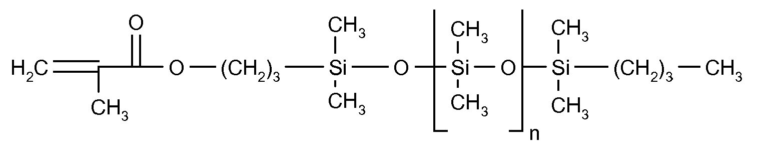
avec n étant un nombre allant de 5 à 700 ; les pourcentages en poids étant calculés par rapport au poids total des monomères.

Une autre famille particulière de polymères siliconés greffés à squelette organique non siliconé convenant pour la réalisation de la présente invention est constituée par les copolymères greffés siliconés susceptibles d'être obtenus par extrusion réactive d'un macromère polysiloxane ayant une fonction réactive terminale sur un polymère du type polyoléfine comportant des groupes réactifs susceptibles de réagir avec la fonction terminale du macromère polysiloxane pour former une liaison covalente permettant le greffage de la silicone sur la chaîne principale de la polyoléfine. Ces polymères sont décrits ainsi que leur procédé de préparation dans la demande de brevet WO 95/00578.

Les polyoléfines réactives sont choisies de préférence parmi les polyéthylènes ou les polymères de monomères dérivés de l'éthylène tels que propylène, styrène, alkyl styrène, butylène, butadiène, les (méth)acrylates, les esters de vinyle ou équivalents, comportant des fonctions réactives susceptibles de réagir avec la fonction terminale du macromère polysiloxane. Ils sont choisis plus particulièrement parmi les copolymères d'éthylène ou de dérivés d'éthylène et de monomères choisis parmi ceux comportant une fonction carboxylique tels que l'acide (méth)acrylique ; ceux comportant une fonction anhydride d'acide tels que l'anhydride de l'acide maléique ; ceux comportant une fonction chlorure d'acide tels que le chlorure de l'acide (méth) acrylique ; ceux comportant une fonction ester tels que les esters de l'acide (méth) acrylique ; ceux comportant une fonction isocyanate.

Les macromères siliconés sont choisis de préférence parmi les polysiloxanes comportant un groupe fonctionnalisé, au bout de la chaîne polysiloxanique ou à proximité de l'extrémité de ladite chaîne, choisi dans le groupe constitué par les alcools, les thiols, les époxy, les amines primaires et secondaires et plus particulièrement parmi ceux répondant à la formule générale :

T-(CH₂)ₛ-Si-[-(OSiR⁵R⁶)ₜ-R⁷]_{y} (VIII)

dans laquelle T est choisi dans le groupe constitué par NH2, NHR', une fonction époxy, OH, SH ; R⁵, R⁶, R⁷ et R', indépendamment, désignent un alkyle en C₁-C₆, phényle, benzyle, ou alkylphényle en C₆-C₁₂, hydrogène ; s est un nombre de 2 à 100 ; t est un nombre de 0 à 1000 et y est un nombre de 1 à 3. Ils ont un poids moléculaire moyen en nombre de préférence allant de 5.000 à 300.000, plus préférentiellement de 8.000 à 200.000 et plus particulièrement de 9000 à 40.000.

Selon la présente invention, le ou les polymères siliconés greffés, à squelette polysiloxanique greffé par des monomères organiques non siliconés comprennent une chaîne principale de silicone (ou polysiloxane (=Si-O-)ₙ) sur laquelle se trouve greffé, à l'intérieur de ladite chaîne ainsi qu'éventuellement à l'une au moins de ses extrémités, au moins un groupement organique ne comportant pas de silicone.

Selon un mode particulièrement préféré de réalisation de la présente invention, le polymère siliconé, à squelette polysiloxanique greffé par des monomères organiques non siliconés, mis en oeuvre comprend le résultat de la copolymérisation radicalaire entre d'une part au moins un monomère organique anionique non-siliconé présentant une insaturation éthylénique et/ou un monomère organique hydrophobe non-siliconé présentant une insaturation éthylénique et d'autre part une silicone présentant dans sa chaîne au moins un, et de préférence plusieurs, groupements fonctionnels capables de venir réagir sur lesdites insaturations éthyléniques desdits monomères non siliconés en formant une liaison covalente, en particulier des groupements thio-fonctionnels.

Selon la présente invention, lesdits monomères anioniques à insaturation éthylénique sont de préférence choisis, seuls ou en mélange, parmi les acides carboxyliques insaturés, linéaires ou ramifiés, éventuellement partiellement ou totalement neutralisés sous la forme d'un sel, ce ou ces acides carboxyliques insaturés pouvant être plus particulièrement l'acide acrylique, l'acide méthacrylique, l'acide maléique, l'anhydride maléique, l'acide itaconique, l'acide fumarique et l'acide crotonique. Les sels convenables sont notamment les sels d'alcalins, d'alcalino-terreux et d'ammonium. On notera que, de même, dans le polymère siliconé greffé final, le groupement organique à caractère anionique qui comprend le résultat de l'(homo)polymérisation radicalaire d'au moins un monomère anionique de type acide carboxylique insaturé peut être, après réaction, post-neutralisé avec une base (soude, ammoniaque,...) pour l'amener sous la forme d'un sel.

Selon la présente invention, les monomères hydrophobes à insaturation éthylénique sont de préférence choisis, seuls ou en mélange, parmi les esters d'acide acrylique alcanols et/ou les esters d'acide méthacrylique d'alcanols. Les alcanols sont de préférence en C₁-C₁₈ et plus particulièrement en C₁-C₁₂. Les monomères préférentiels sont choisis dans le groupe constitué par le (méth)acrylate d'isooctyle, le (méth)acrylate d'isononyle, le 2-éthylhexyl (méth)acrylate, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle ou leurs mélanges.

Une famille de polymères siliconés à squelette polysiloxanique greffé par des monomères organiques non siliconés convenant particulièrement bien à la mise en oeuvre de la présente invention est constituée par les polymères siliconés comportant dans leur structure des motifs de structure (IX ter) et des motifs de structure (IX) et/ou (IX bis) suivants : 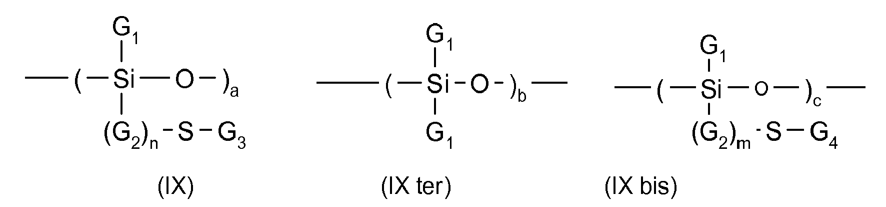 dans lequel les radicaux G₁, identiques ou différents, représentent l'hydrogène ou un radical alkyle en C₁-C₁₀ ou encore un radical phényle ; les radicaux G₂, identiques ou différents, représentent représente un groupe alkylène en C₁C₁₀ ; G₃ représente un reste polymérique résultant de l'(homo)polymérisation d'au moins un monomère anionique à insaturation éthylénique ; G₄ représente un reste polymérique résultant de l'(homo)polymérisation d'au moins un monomère d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

De préférence, le motif de formule (IX) ci-dessus présente au moins l'une, et encore plus préférentiellement l'ensemble, des caractéristiques suivantes :
- les radicaux G₁ désignent un radical alkyle, de préférence le radical méthyle;
- n est non nul, et les radicaux G₂ représentent un radical divalent en C₁-C₃, de préférence un radical propylène ;
- G₃ représente un radical polymérique résultant de l'(homo)polymérisation d'au moins un monomère du type acide carboxylique à insaturation éthylénique, de préférence l'acide acrylique et/ou l'acide méthacrylique ;
- G₄ représente un radical polymérique résultant de l'(homo)polymérisation d'au moins un monomère du type (méth)acrylate d'alkyle en C₁-C₁₀, de préférence le (méth)acrylate d'isobutyle ou de méthyle.

Des exemples de polymères siliconés répondant à la formule (VI) sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle. Comme composé répondant à cette définition on peut citer le poly dimethyl / methyl siloxane à groupements propyl thio-3 acrylate de methyle / methacrylate de methyle / acide methacrylique ou Polysilicone-8 commercialisé sous le nom VS80 par la société 3M.

D'autres exemples de polymères siliconés répondant à la formule (VI) sont notamment des polydiméthylsiloxanes (PDMS) sur lesquels sont greffés, par l'intermédiaire d'un chaînon de raccordement de type thiopropylène, des motifs polymères du type poly(méth)acrylate d'isobutyle.

De préférence, la masse moléculaire en nombre des polymères siliconés à squelette polysiloxanique greffé par des monomères organiques non siliconés de l'invention varie de 10 000 à 1 000 000 environ, et encore plus préférentiellement de 10 000 à 100 000 environ.

De préférence, les polymères siliconés greffés sont choisis dans le groupe constitué par le copolymère de méthacrylate d'alkyles greffé polydiméthylsiloxane, les copolymères de méthacrylate d'isobutyle, d'acide acrylique et de macromère siliconé et le poly diméthyl / méthyl siloxane à groupements propyl thio-3-acrylate de méthyle / méthacrylate de méthyle / acide méthacrylique.

La composition de l'invention peut aussi contenir une silicone réticulée telle qu'un organopolysiloxane élastomère réticulé, un composé siliconé de haut poids moléculaire présentant une structure tridimensionnelle, aux propriétés viscoélastiques d'un matériau solide souple. Ce matériau est capable de retrouver sa forme originelle suite à un étirement. Cet élastomère est formé de chaînes polymériques de haut poids moléculaire dont la mobilité est limitée par un réseau uniforme de points de réticulation. Ces composés ont la propriété d'absorber certains solvants, notamment siliconés, et ainsi de les épaissir, tout en conférant à la composition de très bonnes qualités cosmétiques, notamment d'étalement.

Ces organopolysiloxane peuvent ainsi se présenter sous forme sèche en poudre, ou sous forme gonflée, dans un solvant, le produit résultant étant généralement un gel. Ces produits peuvent également se présenter sous forme dispersée dans une solution aqueuse.
La synthèse de ces organopolysiloxane est décrite dans les brevets suivants :
- US 5266321 de Kobayashi Kose,
- US 4742142 de Toray Silicone,
- US 5654362 de Dow Corning Corp,
- la demande de brevet FR 2 864 784.
Les organopolysiloxanes élastomères utilisés dans la composition peuvent être partiellement ou totalement réticulés. Ils se présentent généralement sous forme de particules. En particulier, les particules d'organopolysiloxane élastomère ont une taille moyenne en nombre allant de 0,1 à 500 µm, de préférence de 3 à 200 µm et mieux de 3 à 50 µm. Ces particules peuvent avoir toute forme et par exemple être sphériques, plates ou amorphes.

L'organopolysiloxane réticulé élastomère peut être obtenu par réaction d'addition réticulation d'un diorganopolysiloxane contenant au moins un atome d'hydrogène lié à un atome de silicium et d'un diorganopolysiloxane ayant au moins deux groupements à insaturation éthylénique liés à des atomes de silicium distincts, notamment en présence de catalyseur platine ; ou par réaction de condensation réticulation, déshydrogénation entre un diorganopolysiloxane à terminaisons hydroxyle et un diorganopolysiloxane contenant au moins un atome d'hydrogène lié à un atome de silicium, notamment en présence d'un organoétain ; ou par réaction de condensation réticulation d'un diorganopolysiloxane à terminaisons hydroxyle et d'un organopolysilane hydrolysable ; ou par réticulation thermique d'organopolysiloxane, notamment en présence de catalyseur organopéroxyde ; ou par réticulation d'organopolysiloxane par radiations de haute énergie telles que rayons gamma, rayons ultraviolet, faisceau électronique.

De préférence, l'organopolysiloxane réticulé élastomère est obtenu par réaction d'addition réticulation d'un diorganopolysiloxane (X) contenant au moins un atome d'hydrogène lié à un atome de silicium, et d'un diorganopolysiloxane (XI) ayant au moins deux groupements à insaturation éthylénique liés chacun à un atome de silicium distinct, notamment en présence de catalyseur platine (XII), comme par exemple décrit dans la demande EP-A-295886.

Le composé (X) est en particulier un organopolysiloxane ayant au moins 2 atomes d'hydrogène liés à des atomes de silicium distincts dans chaque molécule. Le composé (X) peut présenter toute structure moléculaire, notamment une structure chaîne linéaire ou chaîne ramifiée ou une structure cyclique. Le composé (X) peut avoir une viscosité à 25 °C allant de 1 à 50 000 centistokes, notamment pour être bien miscible avec le composé (XI).

Les groupes organiques liés aux atomes de silicium du composé (X) peuvent être des groupes alkyles tels que méthyle, éthyle, propyle, butyle, octyle ; des groupes alkyles substitués tels que 2-phényléthyle, 2-phénylpropyle, 3,3,3-trifluoropropyle ; des groupes aryles tels que phényle, tolyle, xylyle ; des groupes aryles substitués tels que phényléthyle ; et des groupes hydrocarbonés monovalents substitués tels qu'un groupe époxy, un groupe ester carboxylate, ou un groupe mercapto. Le composé (X) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane.

Le composé (XI) est avantageusement un diorganopolysiloxane ayant au moins deux groupes alkényles inférieurs (par exemple en C2-C4) ; le groupe alkényle inférieur peut être choisi parmi les groupes vinyle, allyle, et propényle. Ces groupements alkényles inférieurs peuvent être situés en toute position de la molécule organopolysiloxane mais sont de préférence situés aux extrémités de la molécule organopolysiloxane.

L'organopolysiloxane (XI) peut avoir une structure à chaîne ramifiée, à chaîne linéaire, cyclique ou en réseau mais la structure en chaîne linéaire est préférée. Le composé (XI) peut avoir une viscosité allant de l'état liquide à l'état de gomme. De préférence, le composé (XI) a une viscosité d'au moins 100 centistokes à 25 °C. Outre les groupes alkényle précités, les autres groupes organiques liés aux atomes de silicium dans le composé (XI) peuvent être des groupes alkyles tels que méthyle, éthyle, propyle, butyle ou octyle ; des groupes alkyles substitués tels que 2-phényléthyle, 2-phénylpropyle ou 3,3,3-trifluoropropyle ; des groupes aryles tels que phényle, tolyle ou xylyle ; des groupes aryles substitués tels que phényléthyle ; et des groupes hydrocarbonés monovalents substitués tels qu'un groupe époxy, un groupe ester carboxylate, ou un groupe mercapto.

Les organopolysiloxanes (XI) peuvent être choisis parmi les méthylvinylpolysiloxanes, les copolymères méthylvinylsiloxane-diméthylsiloxane, les diméthylpolysiloxanes à terminaisons diméthylvinylsiloxy, les copolymères diméthylsiloxane-méthylphénylsiloxane à terminaisons diméthylvinylsiloxy, les copolymères diméthylsiloxane-diphénylsiloxane-méthylvinylsiloxane à terminaisons diméthylvinylsiloxy, les copolymères diméthylsiloxane-méthylvinylsiloxane à terminaisons triméthylsiloxy, les copolymères diméthylsiloxane-méthylphénylsiloxaneméthylvinylsiloxane à terminaisons triméthylsiloxy, les méthyl(3,3,3-trifluoropropyl) polysiloxane à terminaisons diméthylvinylsiloxy, et les copolymères diméthylsiloxane-méthyl(3,3,3-trifluoropropyl)siloxane à terminaisons diméthylvinylsiloxy. En particulier, l'organopolysiloxane élastomère peut être obtenu par réaction de diméthylpolysiloxane à terminaisons diméthylvinylsiloxy et de méthylhydrogénopolysiloxane à terminaisons triméthylsiloxy, en présence de catalyseur platine.

Avantageusement, la somme du nombre de groupements éthyléniques par molécule du composé (XI) et du nombre d'atomes d'hydrogène liés à des atomes de silicium par molécule du composé (X) est d'au moins 5.

Il est avantageux que le composé (X) soit ajouté en une quantité telle que le rapport moléculaire entre la quantité totale d'atomes d'hydrogène liés à des atomes de silicium dans le composé (X) et la quantité totale de tous les groupements à insaturation éthylénique dans le composé (XI) soit compris dans la gamme de 1,5/1 à 20/1.

Le composé (XII) est le catalyseur de la réaction de réticulation et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support. Le catalyseur (XII) est de préférence ajouté à raison de 0,1 à 1000 parties en poids, mieux de 1 à 100 parties en poids, en tant que métal platine propre pour 1000 parties en poids de la quantité totale des composés (X) et (XI).

L'organopolysiloxane réticulé obtenu peut être un composé non-émulsionnant ou un composé émulsionnant. Le terme "non émulsionnant" défini des organopolysiloxanes réticulés ne contenant pas de motifs polyoxyalkylène. Le terme "émulsionnant" signifie des composés organopoysiloxanes réticulés ayant au moins un motif polyoxyalkylène, notamment polyoxyéthylène ou polyoxypropylène.

Les particules d'organopolysiloxane réticulé peuvent être véhiculées sous forme de gel constitué d'un organopolysiloxane réticulé inclus dans au moins une huile hydrocarbonée et/ou une huile siliconée. Dans ces gels, les particules d'organopolysiloxane sont souvent des particules non-sphériques. Les particules d'organopolysiloxane réticulé peuvent également se présenter sous forme de poudre, notamment sous forme de poudre sphérique.

Des organopolysiloxanes réticulés non-émulsionnants sont notamment décrits dans les brevets US4970252, US4987169, US 5412004, US5654362, US5760116, dans la demande JP-A-61-194009.

Comme organopolysiloxanes réticulés non-émulsionnants, on peut utiliser ceux vendus sous les dénominations "KSG-6", "KSG-15", "KSG-16", "KSG-18", "KSG-31", "KSG-32", "KSG-33", "KSG-41", "KSG-42", "KSG-43", "KSG-44" « USG-103 » par la société Shin Etsu, "DC 9040", "DC9041", "DC 9509", "DC9505", "DC 9506" « DC 9045 » par la société Dow Corning, "GRANSIL" par la société Grant Industries, "SFE 839" par la société General Electric.

Avantageusement, les organopolysiloxanes réticulés émulsionnants comprennent les organopolysiloxanes modifiés polyoxyalkylène formé à partir de composés divinyliques, en particulier des polysiloxanes ayant au moins deux groupes vinyliques, réagissant avec des liaisons Si-H d'un polysiloxane. Des organopolysiloxanes réticulés émulsionnants sont notamment décrits dans les brevets US5236986, US5412004, US5837793, US5811487.

Comme organopolysiloxanes réticulés émulsionnants, on peut utiliser ceux commercialisés sous les dénominations "KSG-21", "KSG-20", "KSG-30", "X-226146" par la société Shin Etsu, et "DC901 0", "DC9011" par la société Dow Corning.

Les particules d'organopolysiloxane réticulé élastomère peuvent se présenter également sous forme de poudre d'organopolysiloxane réticulé élastomère enrobée de résine de silicone, notamment de résine silsesquioxane, comme décrit par exemple dans le brevet US5538793.

De tels élastomères sont vendus sous les dénomination "KSP-100", "KSP-101", "KSP-102", "KSP-103", KSP-104", "KSP-105" par la société Shin Etsu.

D'autres organopolysiloxanes réticulés élastomères sous forme de poudres peuvent être des poudres de silicone hybride fonctionnalisée par des groupes fluoroalkyle, notamment vendues sous la dénomination "KSP-200" par la société Shin Etsu ; ou des poudres de silicones hybrides fonctionnalisées par des groupes phényl, notamment vendues sous la dénomination "KSP-300" par la société Shin Etsu.

D'autres organopolysiloxanes réticulés peuvent se présenter sous forme de dispersions de poudres dans l'eau en présence ou non d'un émulsifiant comme par exemple les composés BY29-119, DC2-1997, EPSX001 B, EPSX002B, EPSX004A de Dow Corning.

Lorsqu'il est présent dans la composition de l'invention, le ou les polysiloxanes dont la viscosité est supérieure à 100 cst sont introduits en une quantité généralement comprise entre 0.1 % et 30% en poids, notamment entre 0.1% et 20% en poids et préférentiellement entre 0.1 et 10% en poids.

La composition de l'invention peut aussi comprendre un polymère non siliconé qui permet d'améliorer soit les propriétés intrinsèques de la composition soit le gainage obtenu lors de l'application sur le cheveu, soit les deux.

Un tel polymère peut être choisi parmi les polymères suivants:
- les polymères solubles dans un milieu liquide organique, en particulier les polymères liposolubles;
- les polymères dispersibles dans un milieu solvant organique, en particulier les polymères sous la forme de dispersions non aqueuses de particules de polymères de taille primaire inférieure à 1 µm, de préférence des dispersions dans les huiles siliconées ou hydrocarbonées;
- les polymères sous forme de dispersions aqueuses de particules de polymère de taille primaire inférieure à 1µm, souvent appelées «latex»; dans ce cas, la composition comprend une phase aqueuse ;
- les polymères hydrosolubles ; dans ce cas, la composition comprend une phase aqueuse ou bien le polymère est appliqué en pré ou post-traitement du copolymère PDMS-polyurée.

Le polymère utilisable dans la composition peut être anionique, cationique, non-ionique ou amphotère.

A titre de polymère anionique utilisable dans la composition cosmétique selon l'invention, on compte les polymères comportant des groupements dérivés d'acide carboxylique, sulfonique ou phosphorique et qui ont une masse moléculaire moyenne en nombre comprise entre environ 500 et 5 000 000.

Les groupements carboxyliques sont apportés par des monomères mono ou diacides carboxyliques insaturés tels que ceux répondant à la formule : 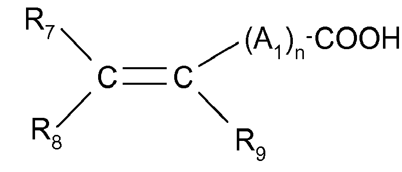 dans laquelle n est un nombre entier de 0 à 10, A₁ désigne un groupement méthylène, éventuellement relié à l'atome de carbone du groupement insaturé ou au groupement méthylène voisin lorsque n est supérieur à 1, par l'intermédiaire d'un hétéroatome tel qu'oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères anioniques à groupements carboxyliques préférés selon l'invention sont :
A) Les homo- ou copolymères d'acide acrylique ou méthacrylique ou leurs sels et en particulier les produits vendus sous les dénominations VERSICOL^{®} E ou K par la société ALLIED COLLOID et ULTRAHOLD^{®} par la société BASF, les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques ;
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevet luxembourgeois nos 75370 et 75371 ou proposés sous la dénomination QUADRAMER par la société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀, par exemple, de lauryle, tels que celui commercialisé par la société ISP sous la dénomination ACRYLIDONE^{®} LM et les terpolymères acide méthacrylique/acrylate d'éthyle/acrylate de tertiobutyle tels que le produit commercialisé sous la dénomination LUVIMER^{®} 100 P par la société BASF ;
   On peut aussi citer les copolymères acide méthacrylique/ acide acrylique/acrylate d'éthyle/méthacrylate de méthyle en dispersion aqueuse, commercialisé sous la dénomination AMERHOLD® DR 25 par la société AMERCHOL ;
C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-cyclique. De tels polymères sont décrits entre autres dans les brevets français nos 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch ;
D) Les copolymères d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US nos 2 047 398, 2 723 248, 2 102 113, le brevet GB n° 839 805. Des produits commerciaux sont notamment ceux vendus sous les dénominations GANTREZ^{®} AN ou ES par la société ISP ;
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,
      les fonctions anhydrides de ces copolymères étant éventuellement monoestérifiées ou monoamidifiées.
      Ces polymères sont par exemple décrits dans les brevets français nos 2 350 384 et 2 357 241 de la demanderesse ;
E) Les polyacrylamides comportant des groupements carboxylates.
   Les homopolymères et copolymères comprenant des groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi:
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone ;
- les sels de l'acide polystyrène-sulfonique tels que les sels de sodium vendus par exemple sous la dénomination Flexan^{®} 500 et Flexan^{®} 130 par National Starch. Ces composés sont décrits dans le brevet FR 2 198 719 ;
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER HSP 1180 par HENKEL.
- les polyesters sulfoniques. Par polyester sulfonique, on entend des copolyesters obtenus par polycondensation d'au moins un acide dicarboxylique ou d'un de ses esters, d'au moins un diol et d'au moins un composé difonctionnel sulfoaryldicarboxylique substitué sur le noyau aromatique par un groupe -S03M dans lequel M représente un atome d'hydrogène ou un ion métallique tel que Na+, Li+ ou K+.

Les polyesters sulfoniques dispersibles dans l'eau présentent généralement une masse moléculaire moyenne en poids comprise entre environ 1 000 et 60 000, et de préférence de 4 000 à 20 000, telle que déterminée par chromatographie par perméation de gel (ou GPC).

La température de transition vitreuse de ces polyesters sulfoniques est généralement comprise dans l'intervalle allant de 10 °C à 100 °C, de préférence de 25 à 60 °C.

Ils sont décrits plus en détail dans les demandes de brevet US 3 734 874, US 3 779 993, US 4 119 680, US 4 300 580, US 4 973 656, US 5 660 816, US 5 662 893, et US 5 674 479.

Les polyesters sulfoniques utilisés de préférence dans l'invention comprennent au moins des motifs dérivés d'acide isophtalique, de sel d'acide sulfoaryle-dicarboxylique et de diéthylèneglycol, et plus particulièrement les polyesters sulfoniques utilisés dans l'invention sont obtenus à partir d'acide isophtalique, de sel de sodium de l'acide sulfoisophtalique, de diéthylèneglycol et de 1,4-cyclohexanediméthanol.

A titre d'exemples de polyester sulfonique, on peut notamment citer ceux connus sous le nom INCl Diglycol/CHDM/Isophtalates/SIP, et vendus sous les dénominations commerciales EASTMAN AQ®, par la société Eastman Chemical, et plus particulièrement celui vendu sous la dénomination commerciale EASTMAN AQ 48®.

Comme autre polymère anionique utilisable selon l'invention, on peut citer le polymère anionique séquencé branché vendu sous la dénomination Fixate G-100 par la société NOVEON.

On peut également utiliser comme polymères, des polyuréthanes anioniques fonctionnalisés ou non, siliconés ou non.

Les polyuréthanes particulièrement visés sont ceux décrits dans les demandes EP 0 751 162, EP 0 637 600, EP 0 648 485 et FR 2 743 297 dont la demanderesse est titulaire, ainsi que dans les demandes EP 0 656 021 et WO 94/03510 de la société BASF, et EP 0 619 111 de la société National Starch.

Comme polyuréthanes convenant particulièrement bien dans la présente invention, on peut citer les produits commercialisés sous les dénominations LUVISET PUR^{®} et LUVISET^{®} Si PUR par la société BASF.

Parmi les polymères anioniques utilisables au sens de la présente invention, on compte aussi les copolymères à blocs ramifiés comprenant, comme monomères principaux, au moins un acrylate d'alkyle en C₁₋₂₀ et/ou au moins un N-mono- ou N,N-di-(alkyle en C₂-₁₂)(méth)acrylamide, et de l'acide acrylique et/ou de l'acide méthacrylique.

Ces copolymères séquencés (ou à blocs) ramifiés présentent une structure constituée de blocs hydrophobes sur lesquels sont fixés, notamment par l'intermédiaire de motifs bifonctionnels, un certain nombre de blocs plus hydrophiles. Ces copolymères présentent au moins deux températures de transition vitreuse.

Ces copolymères présentent la composition suivante :
o de 26 à 36 % en moles d'acide acrylique
o de 27,5 à 30,5 % en moles d'acrylate de n-butyle
o de 33,3 à 45,3 % en moles d'acide métacrylique
o de 0,48 à 0,92 % en moles de méthacrylate d'allyle

Les blocs les plus hydrophobes ont un poids moléculaire de 10 000 à 100 000 et les blocs les plus hydrophiles ont un poids moléculaire de 1000 à 100 000 daltons.

Les polymères ci-dessus sont sous forme anionique, c'est-à-dire qu'ils sont transformés en sels par neutralisation partielle ou totale des groupes acide (méth)acrylique. On peut citer à titre d'exemple d'agent de neutralisation préféré le 2-amino-2-méthyl-1-propanol ou l'hydroxyde de sodium.

Ils sont notamment décrits dans la demande de brevet WO 00/40628 et commercialisés par exemple sous les dénominations EX-SDR-26® et EX-SDR-45® par la société GOODRICH.

Les polymères anioniques sont de préférence choisis parmi les copolymères d'acide acrylique tels que les terpolymères acide acrylique/acrylate d'éthyle/N-tertiobutylacrylamide vendus notamment sous la dénomination ULTRAHOLD^{®} STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ^{®} par la société ISP, les copolymères d'acide méthacrylique et de méthacrylate de méthyle vendus sous la dénomination EUDRAGIT^{®} L par la société ROHM PHARMA, les copolymères d'acide méthacrylique et d'acrylate d'éthyle vendus sous la dénomination LUVIMER^{®} MAEX ou MAE par la société BASF et les copolymères acétate de vinyle/acide crotonique vendus sous la dénomination LUVISET CA 66 par la société BASF et les copolymères acétate de vinyle/acide crotonique greffés par du polyéthylèneglycol vendus sous la dénomination ARISTOFLEX^{®} A par la société BASF, le polymère vendu sous la dénomination Fixate G-100 par la société NOVEON.

Parmi les polymères anioniques cités ci-dessus, on préfère plus particulièrement utiliser dans le cadre de la présente invention, les copolymères méthylvinyléther/anhydride maléique monoestérifiés vendus sous la dénomination GANTREZ® ES 425 par la société ISP, les terpolymères acide acrylique/acrylate d'éthyle/N-tertiobutylacrylamide vendus sous la dénomination ULTRAHOLD® STRONG par la société BASF, les copolymères d'acide méthacrylique et de méthacrylate de méthyle vendus sous la dénomination EUDRAGIT® L par la société ROHM PHARMA, les terpolymères acétate de vinyle/tertio-butylbenzoate de vinyle/acide crotonique et les terpolymères acide crotonique/acétate de vinyle/néododécanoate de vinyle vendus sous la dénomination Résine 28-29-30 par la société NATIONAL STARCH, les copolymères d'acide méthacrylique et d'acrylate d'éthyle vendus sous la dénomination LUVIMER® MAEX OU MAE par la société BASF, les terpolymères vinylpyrrolidone/acide acrylique/méthacrylate de lauryle vendus sous la dénomination ACRYLIDONE® LM par la société ISP, le polymère vendu sous la dénomination Fixate G-100 par la société NOVEON.

A titre de polymère cationique, on peut citer les polymères contenant des groupements cationiques ou des groupements ionisables en groupements cationiques.

Les polymères cationiques les plus représentatifs sont choisis parmi ceux qui comprennent des motifs comportant des groupements amines primaires, secondaires, tertiaires et/ou quaternaires pouvant soit faire partie de la chaîne polymère soit être portés par un substituant latéral.

Les polymères cationiques utilisés ont de préférence une masse moléculaire comprise entre 1000 et 15.10⁶ environ.

Les polymères cationiques utilisés sont notamment ceux qui contiennent au moins 10 % en poids de motifs comportant des groupements amines ou des groupements ammonium quaternaires dont le taux de quaternisation, exprimé en équivalent cationique par gramme de polymère, est par exemple au moins égal à 0,05 méq cationique/g (méq : milliéquivalent).

Lorsque le polymère cationique porte des groupements amine ou ammonium quaternaire portés par un substituant latéral, la chaîne polymère est par exemple une chaîne acrylique, vinylique, peptidique.

Parmi les polymères cationiques on peut citer plus particulièrement les protéines quaternisées, les et les polymères du type polyamine, polyaminoamide et polyammonium quaternaire.

Les polymères peuvent être linéaires, ramifiés, à structure bloc, à structure peigne, à structure dendritique et/ou sous forme de latex dans l'eau ou dans une solution saline concentrée.

A titre d'exemple, on peut citer les protéines quaternisées, notamment des polypeptides modifiés chimiquement portant en bout de chaîne, ou greffés sur celle-ci, des groupements ammonium quaternaires. Parmi ces protéines on peut citer:
a/ les hydrolysats de collagène portant des groupements triéthylammonium tels que les produits vendus sous la dénomination "QUAT-PRO E" par la société MAYBROOK et dénommés dans le dictionnaire CTFA "Triéthonium Hydrolyzed Collagen Ethosulfate";
b/ les hydrolysats de collagène portant des groupements chlorure de triméthylammonium ou de triméthylstéarylammonium,vendus sous la dénomination de QUAT-PRO S par la société MAYBROOK et dénommés dans le dictionnaire CTFA "Steartrimonium Hydrolyzed Collagen";
c/ les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres, vendus par la société CRODA. le CROQUAT L , le CROQUAT M, le CROQUAT S, le CROTEIN O. ; ou les produits vendus par la Société INOLEX, sous la dénomination "LEXEIN QX 3000"; appelé dans le dictionnaire CTFA "Cocotrimonium Collagen Hydrolysate".

Parmi les protéines quaternisées, on peut également citer les protéines végétales quaternisées, telles que les protéines de blé, de maïs ou de soja ; comme protéines de blé quaternisées, on peut citer celles commercialisées par la société CRODA sous les dénominations "HYDROTRITICUM WQ ou QM, appelées dans le dictionnaire CTFA "cocodimonium hydrolysed wheat protein", "HYDROTRITICUM QL" appelée dans le dictionnaire CTFA
"Laurdimonium hydrolysed wheat protein", ou encore sous la dénomination
"HYDROTRITICUM QS" appelée dans le dictionnaire CTFA "Steardimonium hydrolysed wheat protein".

Les polysaccharides cationiques sont aussi des polymères cationiques pouvant être utilisés dans la composition de l'invention. A titre d'exemple, on peut citer les polysaccharides à ammonium quaternaire tels que ceux décrits dans les brevets américains 3.589.578 et 4.031.307 tel que les gommes de guar contenant des groupements cationiques trialkylammonium. De tels produits sont commercialisés notamment sous les dénominations commerciales de JAGUAR C13 S, JAGUAR C 15, JAGUAR C 17 par la société MEYHALL.

On peut aussi citer les les copolymères quaternaires de vinylpyrrolidone et de vinylimidazole ; les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyloxyéthyl triméthylammonium, de méthacrylamidopropyl triméthylammonium, de diméthyldiallylammonium.

Les produits commercialisés répondant à cette définition sont plus particulièrement les produits vendus sous la dénomination "CELQUAT L 200" et "CELQUAT H 100" par la Société National Starch.

D'autres exemples de polymères cationiques sont par exemple les polymères du type polyamine, polyaminoamide, polyammonium quaternaire,

Ces polymères utilisables conformément à la présente invention sont par exemple choisis parmi :
(1) les homopolymères ou copolymères dérivés d'esters ou d'amides acryliques
   ou méthacryliques et comportant au moins un des motifs de formules suivantes: 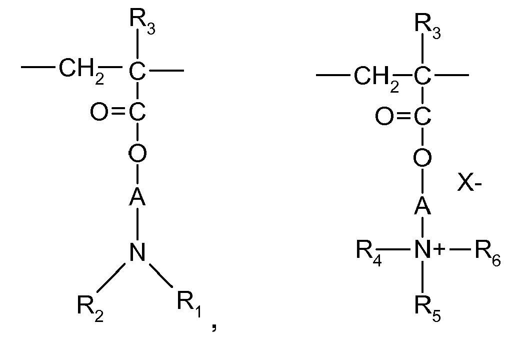 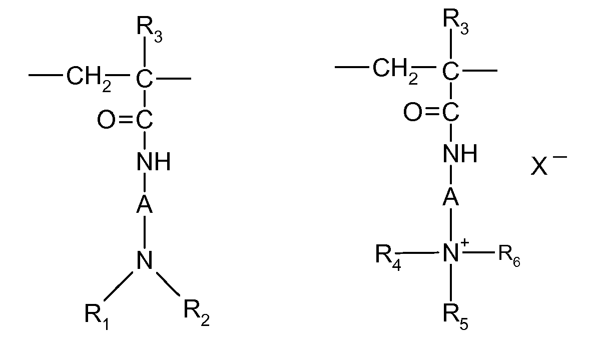 dans lesquelles:
   R3 , identiques ou différents, désignent un atome d'hydrogène ou un radical CH3;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R4, R5, R6, identiques ou différents, représentent un groupe alkyle ayant de 1 à 18 atomes de carbone ou un radical benzyle et de préférence un groupe alkyle ayant de 1 à 6 atomes de carbone;
   R1 et R2 , identiques ou différents, représentent hydrogène ou un groupe alkyle ayant de 1 à 6 atomes de carbone et de préférence méthyle ou éthyle;
   X désigne un anion dérivé d'un acide minéral ou organique tel que un anion méthosulfate ou un halogénure tel que chlorure ou bromure.
   Les copolymères de la famille (1) peuvent contenir en outre un ou plusieurs motifs dérivant de monomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un halogénure de méthyle tels que celui vendu sous la dénomination HERCOFLOC par la société HERCULES,
   - les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
   - le copolymères d'acrylamide et de méthosulfate de méthacryloyloxyéthyltriméthylammonium vendus sous la dénomination RETEN par la société HERCULES,
   - les copolymères vinylpyrrolidone / acrylate ou méthacrylate de dialkylaminoalkyle quaternisés ou non. Ces polymères sont décrits en détail dans les brevets français 2.077.143 et 2.393.573, et sont notamment commercialisés sous la dénomination "GAFQUAT" par la Société GAF CORPORATION comme par exemple "GAFQUAT 734 ou 755" ou bien le produit dénommé "COPOLYMERE 937".
   - les polymères à chaîne grasse et à motif vinylpyrrolidone, tels que les produits vendus sous la dénomination Stylèze W20 et Stylèze W10 par la société ISP,
   - les terpolymères méthacrylate de diméthylaminoéthyle/vinylcaprolactame/vinylpyrrolidone tel que le produit commercialisé sous la dénomination GAFFIX VC 713 par la société ISP, et
   - et les copolymères vinylpyrrolidone/méthacrylamide de diméthylaminopropyle quaternisés tels que les produits commercialisés sous la dénomination "GAFQUAT® HS 100" par la société ISP.
(2) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(3) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(4) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "CARTARETINE F, F4 ou F8" par la société SANDOZ.
(5) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "HERCOSETT 57" par la société Hercules Inc. par la société HERCULES dans le cas du copolymère d'acide adipique/époxypropyl/diéthylène-triamine.
(6) les cyclopolymères d'alkyl diallyl amine ou de dialkyl diallyl ammonium tels que les homopolymères ou copolymères comportant comme constituant principal de la chaîne des motifs répondant aux formules (1) ou (1') : 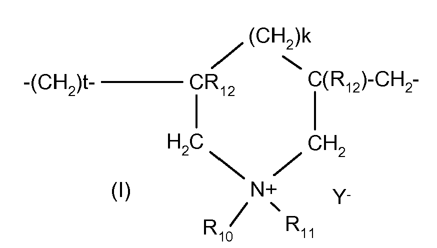 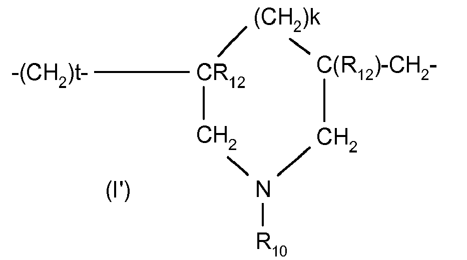 formules dans lesquelles k et t sont égaux à 0 ou 1, la somme k + t étant égale à 1 ; R12 désigne un atome d'hydrogène ou un radical méthyle ; R10 et R11, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4) ou R10 et R11 peuvent désigner conjointement avec l'atome d'azote auquel ils sont rattachés, des groupement hétérocycliques, tels que pipéridinyle ou morpholinyle ; Y-est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R10 et R11, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallylammonium vendu sous la dénomination "MERQUAT 100" par la société NALCO (et ses homologues de faibles masses molaires moyenne en poids) et les copolymères de chlorure de diallyldiméthylammonium et d'acrylamide .
(7) les polymères de diammonium quaternaire contenant des motifs récurrents répondant à la formule : 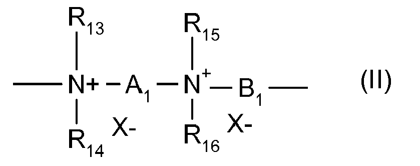 formule (11) dans laquelle :
   R13, R14, R15 et R16, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R13, R14, R15 et R16, ensemble ou séparément, constituent avec les atomes d'azote auxquels ils sont rattachés des hétérocycles contenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-OR17-D ou -CO-NH-R17-D où R17 est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X- désigne un anion dérivé d'un acide minéral ou organique;
   A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
      dans lequel D désigne :
      a) un reste de glycol de formule : -O-Z-O-, où Z désigne un radical hydrocarboné linéaire ou ramifié ou un groupement répondant à l'une des formules suivantes :
         - (CH2-CH2-O)x-CH2-CH2-
         - [CH2-CH(CH3)-O]y-CH2-CH(CH3)-
         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent -CH2-CH2-S-S-CH2-CH2- ;
      d) un groupement uréylène de formule : -NH-CO-NH- ;
         De préférence, X- est un anion tel que le chlorure ou le bromure.
         Ces polymères ont une masse molaire moyenne en nombre généralement comprise entre 1000 et 100000.
         Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
         On peut utiliser plus particulièrement les polymères qui sont constitués de motifs récurrents répondant à la formule : 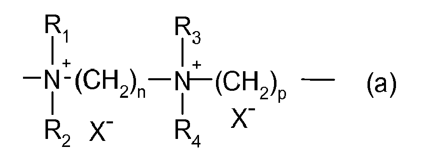 dans laquelle R1, R2, R3 et R4, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.
         Un composé de formule (a) particulièrement préféré est celui pour lequel R1, R2, R3 et R4, représentent un radical méthyle et n = 3, p = 6 et X = Cl, dénommé Hexadimethrine chloride selon la nomenclature INCI (CTFA).
(8) les polymères de polyammonium quaternaires comprenant des motifs de formule (III): 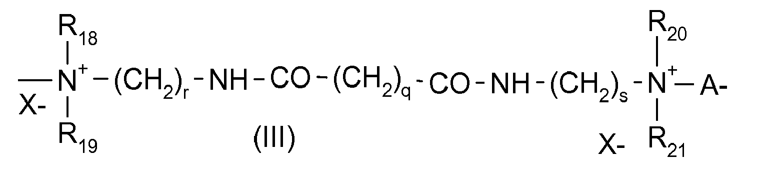 formule dans laquelle :
   R18, R19, R20 et R21, identiques ou différents, représentent un atome d'hydrogène ou un radical méthyle, éthyle, propyle, β-hydroxyéthyle, β-hydroxypropyle ou -CH2CH2(OCH2CH2)pOH,
   où p est égal à 0 ou à un nombre entier compris entre 1 et 6, sous réserve que R18, R19, R20 et R21 ne représentent pas simultanément un atome d'hydrogène,
   r et s, identiques ou différents, sont des nombres entiers compris entre 1 et 6,
   q est égal à 0 ou à un nombre entier compris entre 1 et 34,
   X- désigne un anion tel qu'un halogènure,
   A désigne un radical d'un dihalogénure ou représente de préférence -CH2-CH2-O-CH2-CH2-.
      De tels composés sont notamment décrits dans la demande de brevet EP-A-122 324.
      On peut par exemple citer parmi ceux-ci, les produits "Mirapol® A 15", "Mirapol® AD1", "Mirapol® AZ1" et "Mirapol® 175" vendus par la société Miranol.
(9) Les polymères quaternaires de vinylpyrrolidone et de vinylimidazole tels que par exemple les produits commercialisés sous les dénominations Luviquat® FC 905, FC 550 et FC 370 par la société B.A.S.F.
(10) Les polymères réticulés de sels de méthacryloyloxyalkyl(C1-C4) trialkyl(C1-C4)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide.
(11) Les polyalkylènes imines en particulier les polyéthylèneimines et leurs dérivés. Ces polymères sont notamment décrits dans les demandes EP1426035 et W02005/092274.
   Les polyéthylèneimines sont notamment décrites dans les documents : « KIRK-OTHMER ENCYCLOPEDIA OF CHEMICAL TECHNOLOGY », 3ème édition, vol.20, 1982, p.214-216, et « Polyethyleneimine Prospective Application » H.N. Feigenbaum, Cosmetics & Toiletries, 108, 1993, p.73.
   Les polyéthylèneimines (PEI) utilisables selon l'invention présentent généralement la formule suivante :

   -(CH₂-CH₂-NH)ₙ-

   où n est le nombre moyen d'unités éthylèneimine, n étant compris entre 5 et 10 000.
   Les homopolymères d'éthylèneimine peuvent être branchés.
   On peut citer en particulier la PEI-7 (n=7), la PEI-15 (n=15), la PEI-30 (n=30), la PEI-45 (n=45), la PEI-275 (n=275), la PEI-700 (n=700), la PEI-1000 (n=1000), la PEI-1400 (n=1400), la PEI-1500 (n=1500), la PEI-1750 (n=1750), et la PEI-2500 (n=2500).
   On citera par exemple les polyéthylèneimines de la gamme LUPASOL, notamment les produits commercialisés sous les dénominations LUPASOL G35, FG, PS, HF, P, et le POLYMIN SK de BASF.
   Les polyéthylènemimines (PEI) peuvent être modifiées par des greffons hydrophiles (par exemple polyéthylèneglycol (PEG), polyvinylacétate (PVA), polyacrylate) ou par des greffons hydrophobes (par exemple silicone et/ou chaînes grasses carbonées en C8-C30) comme décrit dans les brevets WO97/20879, WO97/23456, WO02/095122, WO02/15854, US 5 756 080, EP 0524612 ainsi que dans la publication H Petersen et al, Macromolecules, 2002, 35, p6867.
   Les PEI-PEG sont notamment vendues sous les dénominations Lupasol SC61 B, SC62J, LU158 et HEO1 par la société BASF.
   Les PEI à chaînes grasses sont notamment vendues sous les dénominations LUPASOL ESA 51685 ou LU157 par la société BASF.
   12) les polymères dérivés d'acides aminés
      Les polymères utilisables dans la composition selon l'invention peuvent également être choisis parmi les polymères comprenant au moins 2 unités d'un ou plusieurs acides aminés basiques.
      Le ou lesdits acides aminés basiques sont choisis de préférence parmi l'ornithine, l'aspargine, la glutamine, la lysine et l'arginine. Lesdits polymères contenant au moins 2 unités d'un ou plusieurs acides aminés basiques utilisables dans la composition selon 10 l'invention contiennent généralement 2 à 10000 unités d'acide aminé basique.
      Ces polymères peuvent être modifiés par des greffons hydrophiles (Polyéthylène glycol, PVA(polyacétate de vinyle), polyacrylate) ou hydrophobes (PDMS (polydiméthylsiloxane à chaînes grasses carbonée en C8-C30).
      On citera par exemple la publication suivante pour la synthèse de polylysine - PEG : GL Kenausis et al, Journal of Physical Chemistry B, 2000, 104, p3298.
      On citera également le brevet suivant WO 00/071601 : synthèse de poly(acides aminés basiques) alcoxylés, notamment les polylysine-polyéthylèneglycol.
      Les polylysines sont également décrites dans le brevet JP2003040724.
      On citera par exemple les Poly-epsilon-lysine et ses dérivés siliconés produits par CHISSO sous les noms « polylysine »
   13) les dendrimères aminés
      Par dendrimères contenant des amines primaires en position terminale, on entend des composés polymères étant constitués d'un coeur et de génération d'unités de base, monomères ou fuseaux, sur lesquels un groupe terminal T porteur d'une fonction amine primaire a été greffé.
      On peut citer par exemple les dendrimères polyamidoamines, tels que ceux commercialisés sous la référence commerciale STARBURST PAMAM par la société DENDRITECH (copolymères séquencés d'éthylène diamine et d'acrylate de méthyle), ou ceux commercialisés sous la référence commerciale ASTROMOLS (DAB) par la société DSM.
   14) Les polyallylamines
      Il s'agit des polyallylamines telles que celles produites par Nitto Boseki Co et de leurs dérivés tels que ceux décrits dans la demande W02005/092274.
      Les polymères utiles dans la composition de l'invention peuvent être choisis parmi les polymères amphotères choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes;
      B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-α,β-dicarboxylique dont l'un des groupements carboxyliques a été amené à réagir avec une polyamine comportant un ou plusieurs groupements amines primaires ou secondaires.

Les polymères amphotères répondant à la définition donnée ci-dessus plus particulièrement préférés sont choisis parmi les polymères suivants :
(1) les copolymères à motifs vinyliques acides et à motifs vinyliques basiques, tels que ceux résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique, tel que plus particulièrement les méthacrylate et acrylate de dialkylaminoalkyle, les dialkylaminoalkylméthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'atome d'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.
      Les acrylamides ou méthacrylamides N-substitués plus particulièrement préférés selon l'invention sont les composés dont les groupes alkyles comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
      Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléïque ou fumarique.
      Les comonomères basiques préférés sont des méthacrylates d'aminoéthyle, de butylaminoéthyle, de N,N'-diméthylaminoéthyle, de N-tertio-butylaminoéthyle.
      On utilise particulièrement les copolymères dont la dénomination CTFA (4ème Ed., 1991) est Octylacrylamide/acrylates/butylaminoethyl-methacrylate copolymer, tels que les produits vendus sous la dénomination AMPHOMER^{®} ou LOVOCRYL^{®} 47 par la société NATIONAL STARCH.
(3) les polyaminoamides réticulés et acylés partiellement ou totalement dérivant de polyaminoamides de formule générale : 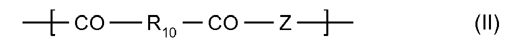 dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
   a) dans les proportions de 60 à 100 % en moles, le groupe 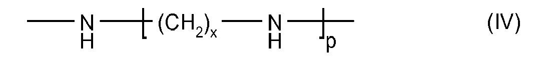 où x=2 et p=2 ou 3, ou bien x=3 et p=2
      ce groupe dérivant de la diéthylène-triamine, de la triéthylène-tétraamine ou de la dipropylène-triamine;
   b) dans les proportions de 0 à 40 % en moles, le groupe (IV) ci-dessus,
      dans lequel x=2 et p=1 et qui dérive de l'éthylène-diamine, ou le groupe dérivant de la pipérazine : 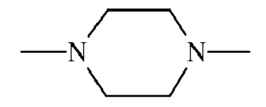
   c) dans les proportions de 0 à 20 % en moles, le groupe -NH-(CH₂)₆-NH- dérivant de l'hexaméthylènediamine,
      ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.
   Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
   Les alcane-sulfones utilisées dans l'acylation sont de préférence la propane- ou la butane-sulfone, les sels des agents d'acylation sont de préférence les sels de sodium
   ou de potassium.
(4) les polymères comportant des motifs zwittérioniques de formule : 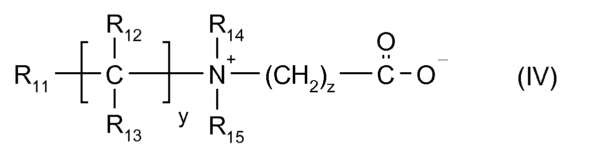 dans laquelle R₁₁ désigne un groupement insaturé polymérisable tel qu'un groupement acrylate, méthacrylate, acrylamide ou méthacrylamide, y et z représentent un nombre entier de 1 à 3, R₁₂ et R₁₃ représentent un atome d'hydrogène, un groupe méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de tels motifs peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.
   A titre d'exemple, on peut citer les copolymères méthacrylate de méthyle/diméthyl-carboxyméthylammonio-éthylméthacrylate, tel que le produit vendu sous la dénomination DIAFORMER Z301 par la société SANDOZ.
(5) les polymères dérivés du chitosane comportant des motifs monomères répondant aux formules suivantes : 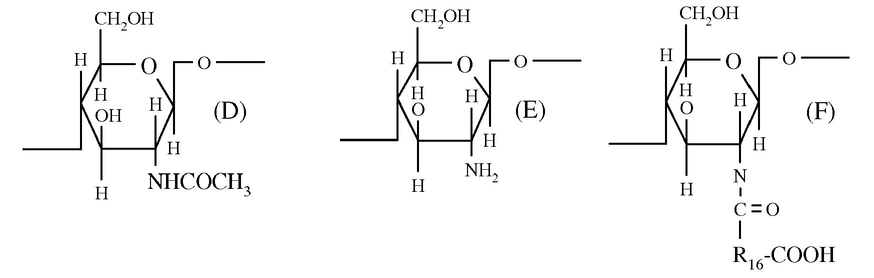 le motif (D) étant présent dans des proportions comprises entre 0 et 30%, le motif (E) dans des proportions comprises entre 5 et 50% et le motif (F) dans des proportions comprises entre 30 et 90%, étant entendu que dans ce motif (F), R₁₆ représente un groupe de formule : 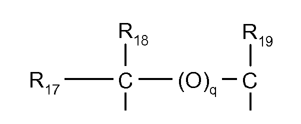 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères répondant à la formule générale (V) sont, par exemple, décrits dans le brevet français 1 400 366 et sont constitués de motifs: 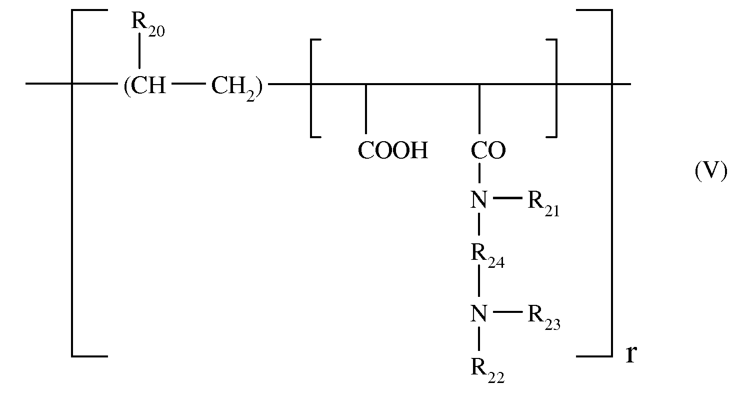 dans laquelle R₂₀ représente un atome d'hydrogène, un groupe CH₃O, CH₃CH₂O, phényle, R₂₁ désigne un atome d'hydrogène ou un groupe alkyle inférieur tel que méthyle, éthyle, R₂₂ désigne un atome d'hydrogène ou un groupe alkyle inférieur en C₁-C₆ tel que méthyle, éthyle, R₂₃ désigne un groupe alkyle inférieur en C₁-C₆ tel que méthyle, éthyle ou un groupe répondant à la formule : -R₂₄-N(R₂₂)₂, R₂₄ représentant un groupement -CH₂-CH₂-, -CH₂-CH₂-CH₂-, -CH₂-CH(CH₃)-, R₂₂ ayant les significations mentionnées ci-dessus.
(7) Les polymères dérivés de la N-carboxyalkylation du chitosane comme le N-carboxyméthyl-chitosane ou le N-carboxybutyl-chitosane, vendus sous la dénomination « EVALSAN » par la société JAN DEKKER.
(8) Les polymères amphotères du type -D-X-D-X choisis parmi:
   a) les polymères obtenus par action de l'acide chloracétique ou le chloracétate de sodium sur les composés comportant au moins un motif de formule :

      -D-X-D-X-D- (VI)

      où D désigne un groupe 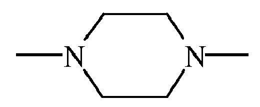 et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X-D-X- (VI')
   où D désigne un groupe 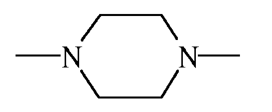 et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyles et comportant un ou plusieurs atomes d'azote, l'atome d'azote étant substitué par une chaîne alkyle interrompue éventuellement par un atome d'oxygène et comportant obligatoirement une ou plusieurs fonctions carboxyles ou une ou plusieurs fonctions hydroxyles et bétaïnisées par réaction avec l'acide chloracétique ou du chloracétate de soude.
(9) les copolymères alkyl(C₁-C₅)vinyléther/anhydride maléique modifiés partiellement par semiamidification avec une N,N-dialkylaminoalkylamine telle que la N,N-diméthylaminopropylamine ou par semiestérification avec un N,N-dialkylaminoalcanol. Ces copolymères peuvent également comporter d'autres comonomères vinyliques tels que le vinylcaprolactame.

Parmi les polymères amphotères cités ci-dessus les plus particulièrement préférés selon l'invention, on citera ceux de la famille (3) tels que les copolymères dont la dénomination CTFA est Octylacrylamide/acrylates/butylamino-ethylmethacrylate copolymer, tels que les produits vendus sous les dénominations AMPHOMER^{®}, AMPHOMER^{®} LV 71 ou LOVOCRYL^{®} 47 par la société NATIONAL STARCH et ceux de la famille (4) tels que les copolymères méthacrylate de méthyle/diméthyl-carboxy-méthylammonio-éthylméthacrylate de méthyle vendu par exemple sous la dénomination DIAFORMER Z301 par la société SANDOZ.

Les polymères utiles dans la composition de l'invention peuvent être des polymères non ioniques tels que :
- les polyalkyloxazolines ;
- les homopolymères d'acétate de vinyle ;
- les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une α-oléfine (ayant de 8 à 28 atomes de carbone), un ester acrylique, un ester maléique, un éthylène, un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).
   Ces copolymères peuvent être partiellement réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.
   Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, acétate de vinyle/maléate de dibutyle propionate, acétate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.
- les homopolymères et copolymères d'esters acryliques. Les homopolymères et les copolymères préférés sont obtenus à partir de monomères choisis parmi le groupe constitué par le (méth)acrylate d'isooctyle, le (méth)acrylate d'isononyle, le (méth)acrylate de 2-éthylhexyle, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle, ou des mélanges de ceux-ci. On citera, par exemple, le copolymère d'acrylate d'alkyle/acrylate de cycloalkyle commercialisé par PHOENIX CHEM. sous la dénomination GIOVAREZ AC-5099 ML les copolymères d'acrylates d'alkyle et de méthacrylates d'alkyle tels que les produits proposés par la société ROHM & HAAS sous les dénominations PRIMAL^{®} AC-261 K et EUDRAGIT^{®} NE 30 D, par la société BASF sous la dénomination 8845, par la société HOECHST sous la dénomination APPRETAN^{®} N9212 ;
- les copolymères d'acrylonitrile et d'un monomère non ionique choisis, par exemple, parmi le butadiène et les (méth)acrylates d'alkyle ; on peut citer les produits proposés sous la dénomination CJ 0601 B par la société ROHM & HAAS;
- les homopolymères de styrène ;
- les copolymères de styrène ou dérivés du styrène (par exemple le méthylstyrène, le chlorostyrène ou le chlorométhylstyrène). Le copolymère comprenant au moins un bloc styrène peut être un copolymère dibloc ou tribloc, voire un copolymère multibloc, en étoile ou radial. Le copolymère comprenant au moins un bloc styrène peut comprendre en outre, par exemple, un bloc alkylstyrène (AS), un bloc éthylène/butylène (EB) un bloc éthylène/ propylène (EP), un bloc butadiène (B), un bloc isoprène (1), un bloc acrylate (A), un bloc méthacrylate (MA) ou une association de ces blocs. Le copolymère comprenant au moins un bloc constitué de motifs styrène ou dérivés du styrène peut être un copolymère dibloc ou tribloc, et en particulier du type polystyrène/polyisoprène ou polystyrène/polybutadiène, tels que ceux commercialisés ou fabriqués sous la dénomination « Luvitol HSB » par BASF et ceux du type polystyrène/copoly(éthylène-propylène) ou de manière alternative du type polystyrène/copoly(éthylène/butylène), tels que ceux commercialisés ou fabriqués sous la marque de fabrique « Kraton » par Shell Chemical Co. ou Gelled Permethyl 99A par Penreco, peuvent être utilisés.

On peut citer par exemple le le Kraton G1650 (SEBS), le Kraton G1651 (SEBS), le Kraton G1652 (SEBS), le Kraton G1657X (SEBS), le Kraton G1701X (SEP), le Kraton G1702X (SEP), le Kraton G1726X (SEB), le Kraton D-1101 (SBS), le Kraton D-1102 (SBS), le Kraton D-1107 (SIS), le Gelled Permethyl 99A-750, le Gelled Permethyl 99A-753-58 (mélange de polymère bloc en étoile et de polymère tribloc), le Gelled Permethyl 99A-753-59 (mélange de polymère bloc en étoile et de polymère tribloc), le Versagel 5970 et le Versagel 5960 de chez Penreco (mélange de polymère en étoile et de polymère tribloc dans l'isododécane).

Des copolymères de styrène-méthacrylate peuvent également être utilisés tels que les polymère commercialisés sous les référenceq OS 129880, OS 129881 et OS 84383 de chez Lubrizol (copolymère de styrène-méthacrylate). On peut également citer les copolymères de styrène et de (méth)acrylate d'alkyle tels que les produits MOWILITH^{®} LDM 6911, MOWILITH^{®} DM 611 et MOWILITH^{®} LDM 6070 proposés par la société HOECHST, les produits RHODOPAS^{®} SD 215 et RHODOPAS^{®} DS 910 proposés par la société RHONE POULENC.
- les polyamides ;
- les homopolymères de vinyllactame différents des homopolymères de vinylpyrrolidone, tels que le polyvinylcaprolactame commercialisé sous la dénomination Luviskol^{®} PLUS par la société BASF ; et
- les copolymères de vinyllactame tels qu'un copolymère poly(vinylpyrrolidone/vinyllactame) vendu sous le nom commercial Luvitec^{®} VPC 55K65W par la société BASF, les copolymères poly(vinylpyrrolidone/acétate de vinyle) comme ceux commercialisés sous la dénomination PVPVA^{®} S630L par la société ISP, Luviskol^{®} VA 73, VA 64, VA 55, VA 37 et VA 28 par la société BASF ; et les terpolymères poly(vinylpyrrolidone/acétate de vinyle/propionate de vinyle) comme, par exemple, celui commercialisé sous la dénomination Luviskol^{®} VAP 343 par la société BASF.
- les copolymères de vinylpyrrolidone, tels que les copolymères d'un alkène en C₂ à C₃₀, tel qu'en C₃ à C₂₂, et des associations de ceux-ci, peuvent être utilisés. Comme exemples de copolymères de VP pouvant être utilisés dans l'invention, on peut citer le copolymère de VP/laurate de vinyle, de VP/stéarate de vinyle, la polyvinylpyrrolidone (PVP) butylée, de VP/hexadécène, de VP/Eicosène, de VP/triacontène ou de VP/acide acrylique/méthacrylate de lauryle. De tels copolymères sont par exemple ceux commercialisés par la société ISP sous le nom Ganex V 220 ou Ganex V 216.
- les polymères portant des groupements fluorés appartenant tels que les polyperfluoroéthers, en particulier les Fomblin décrits dans le brevet US 5 948 393, les copolymères de (méth)acrylate d'alkyle/(méth)acrylate de perfluoroalkyle décrits dans les brevets EP 0 815 836 et US 5 849 318.
- les polyuréthanes non-ioniques. Ce polyuréthane peut être non-associatif ou associatif

Par polyuréthane non associatif, on entend au sens de la présente invention, des polycondensats comprenant au moins une séquence polyuréthane et ne comprenant pas, dans leur structure, de chaîne alkyle ou alcényle, terminale ou pendante, comportant plus de 10 atomes de carbone. Ils sont décrits en particulier dans les brevets EP 0 751 162, EP 0 637 600, FR 2 743 297 et EP 0 648 485 dont la Demanderesse est titulaire, ainsi que les brevets EP 0 656 021 ou WO 94/03510 de la Société BASF et EP 0 619 111 de la Société National Starch.

Les polyuréthanes non associatifs utilisés conformément à l'invention peuvent être solubles dans le milieu aqueux cosmétiquement acceptable, ou encore former une dispersion dans ce milieu. La dispersion peut comprendre alors au moins 0,05 % de tensioactif permettant la mise en dispersion et le maintien en dispersion du polyuréthane non associatif.

Selon l'invention, on peut utiliser tout type de tensioactif dans ladite dispersion, mais de préférence un tensioactif non ionique. La taille moyenne des particules du polyuréthane non associatif dans la dispersion est de préférence comprise entre 0,1 et 1 micromètre.

A titre d'exemple, le polyuréthane non associatif peut être formé par un arrangement de blocs, cet arrangement étant obtenu notamment à partir de :
(1) au moins un composé qui contient deux ou plus de deux atomes d'hydrogène actifs par molécule ;
(2) au moins un diol ou un mélange de diols contenant des fonctions acides ou leurs sels ; et
(3) au moins un di- ou polyisocyanate.

Avantageusement, les composés (1) sont choisis dans le groupe comprenant les diols, les diamines, les polyesterols, les polyétherols ou leurs mélanges.

Les composés (1) qui sont préférés sont les polyéthylèneglycols et les polypropylèneglycols linéaires, en particulier ceux qui sont obtenus par réaction de l'oxyde d'éthylène ou de propylène avec l'eau ou du diéthylène ou du dipropylèneglycol en présence d'hydroxyde de sodium en tant que catalyseur. Ces polyalkylèneglycols ont généralement une masse moléculaire comprise entre environ 600 et 20 000.

D'autres composés organiques préférés sont ceux qui ont des groupes mercapto, amino, carboxyle ou hydroxyle. Parmi ceux-ci, on cite plus particulièrement les composés polyhydroxylés tels que les polyéther-diols, les polyester-diols, les polyacétal-diols, les polyamide-diols, les polyester-polyamide-diols, les poly(alkylène éther)-diols, les polythioéther-diols et les polycarbonate-diols.

Les polyéther-diols préférés sont, par exemple, les produits de condensation d'oxyde d'éthylène, d'oxyde de propylène ou de tétrahydrofurane, leurs produits de copolymérisation ou de condensation, greffés ou blocs, tels que les mélanges de condensats d'oxyde d'éthylène et de propylène, et les produits de polymérisation d'oléfines, sous haute pression, avec les condensats d'oxyde d'alkylène. Des polyéther-diols appropriés sont par exemple préparés par condensation d'oxydes d'alkylène et d'alcools polyhydriques, tels que l'éthylèneglycol, le 1,2-propylèneglycol et le 1,4-butanediol.

Les polyester-diols, polyester-amides, polyamide-diols sont de préférence saturés et sont obtenus, par exemple, à partir de la réaction d'acides polycarboxyliques saturés ou insaturés avec des alcools polyhydriques, des diamines ou des polyamines. Pour préparer ces composés, on peut utiliser, par exemple, l'acide adipique, l'acide succinique, l'acide phtalique, l'acide téréphtalique et l'acide maléique. Des alcools polyhydriques appropriés pour préparer les polyesters incluent par exemple l'éthylèneglycol, le 1,2-propylèneglycol, le 1,4-butanediol, le néopentylglycol et l'hexanediol. On peut aussi utiliser des aminoalcools, par exemple l'éthanolamine. Des diamines appropriées pour préparer les amide-polyesters sont l'éthylène-diamine et l'hexaméthylène-diamine.

Des polyacétals appropriés peuvent être préparés, par exemple, à partir de 1,4-butanediol ou d'hexanediol et de formaldéhyde. Des polythioéthers appropriés peuvent être préparés, par exemple, par réaction de condensation entre des thioglycols seuls ou en combinaison avec d'autres glycols tels que l'éthylèneglycol, le 1,2-propylèneglycol ou avec d'autres composés polyhydroxylés. Les composés polyhydroxylés contenant déjà des groupements uréthannes, des polyols naturels, qui peuvent être davantage modifiés, par exemple, l'huile de ricin et les carbohydrates peuvent également être utilisés.

Plus préférentiellement, le composé du groupe (1) est un polyestérol, notamment un polyester-diol formé par la réaction d'au moins un (di)-polyol (1a) et d'au moins un acide (1b). Le (di)-polyol (1a) est en particulier choisi dans le groupe comprenant le néopentylglycol, le 1,4-butanediol, l'hexanediol, l'éthylèneglycol, le diéthylèneglycol, le propylèneglycol, le butylèneglycol, le néopentylglycol et (di)-polyéthylèneglycol. L'acide (1 b) est en particulier choisi dans le groupe comprenant l'acide phtalique, l'acide isophtalique, l'acide adipique et l'acide (poly)-lactique.

En tant que composé (2), on peut notamment utiliser un acide hydroxycarboxylique tel que l'acide diméthylol-propanoïque (DMPA) ou un acide 2,2-dihydroxyméthyl-carboxylique. En général, le composé (2) est utile en tant que bloc de couplage. En tant que composés (2), on préfère ceux comprenant au moins un poly(acide carboxylique α,α-dihydroxylé).

Les composés (2) particulièrement préférés conformément à l'invention sont ceux choisis dans le groupe comprenant l'acide 2,2-di-(hydroxyméthyl)acétique, l'acide 2,2-dihydroxyméthylpropionique, l'acide 2,2-dihydroxyméthylbutyrique, l'acide 2,2-dihydroxyméthylpentanoïque.

Le di- ou polyisocyanate (3) peut être choisi en particulier dans le groupe comprenant l'hexaméthylène-diisocyanate, l'isophorone-diisocyanate (IDPI), le toluylène-diisocyanate, le diphénylméthane-4,4'-diisocyanate (DPMD) et le dicyclohexylméthane-4,4'-diisocyanate (DCMD), le méthylène-di-p-phényl-diisocyanate, le méthylène-bis(4-cyclohexylisocyanate), les toluène-diisocyanates, le 1,5-naphtalène-diisocyanate, le 4,4'-diphénylméthane-diisocyanate, le 2,2'-diméthyl-4,4'-diphénylméthane-diisocyanate, le 1,3-phénylène-diisocyanate, le 1,4-phénylène diisocyanate, des mélanges de 2,4- et de 2,6-toluène-diisocyanates, le 2,2'-dichloro-4,4'-diisocyanato-diphénylméthane, le 2,4-dibromo-1,5-diisocyanato naphtalène, le butane-1,4-diisocyanate, l'hexane-1,6-diisocyanate, et le cyclohexane-1,4-diisocyanate.

Le polyuréthane non associatif peut être formé à l'aide d'un composé supplémentaire (4) servant en général à allonger sa chaîne. Ces composés (4) peuvent être choisis dans le groupe comprenant notamment les glycols saturés ou insaturés tels que l'éthylèneglycol, le diéthylèneglycol, le néopentylglycol, le triéthylène glycol ; les aminoalcools tels que l'éthanolamine, la propanolamine, la butanolamine ; les amines primaires hétérocycliques, aromatiques, cycloaliphatiques, et aliphatiques ; les diamines ; les acides carboxyliques tels que les acides carboxyliques aliphatiques, aromatiques et hétérocycliques comme les acides oxalique, succinique, glutarique, adipique, sébacique et téréphtalique ; et les acides aminocarboxyliques. Les composés (4) préférés sont les diols aliphatiques.

Les polyuréthanes non associatifs utilisés selon l'invention peuvent également être encore formés à partir de composés supplémentaires (5) ayant un squelette siliconé tels que les polysiloxanes, les polyalkylsiloxanes ou les polyarylsiloxanes, notamment les polyéthylsiloxanes, les polyméthylsiloxanes et les polyphénylsiloxanes, comportant éventuellement des chaînes hydrocarbonées greffées sur les atomes de silicium.

Les polyuréthanes non associatifs utilisés avantageusement comprennent un motif répétitif de base répondant à la formule générale (VI) :

-O-B-O-CO-NH-R-NH-CO- (VI)

dans laquelle :
- B est un groupe hydrocarboné divalent en C1 à C30, ce groupe étant substitué
ou non par un groupement comportant une ou plusieurs fonctions acides carboxyliques et/ou une ou plusieurs fonctions acides sulfoniques, lesdites fonctions acides carboxyliques et/ou sulfoniques étant sous forme libre ou bien neutralisées partiellement ou totalement par une base minérale ou organique, et
- R est un groupe divalent choisi parmi les groupes hydrocarbonés aliphatiques en C1 à C20, cycloaliphatiques en C3 à C20 et aromatiques en C6 à C20, comme par exemple les groupes alkylène en C1 à C20, arylène en C6 à C20, cycloalkylène en C3 à C20, ou leurs combinaisons, ces groupes étant substitués ou non.
   Le groupe R est avantageusement choisi parmi les groupes répondant aux formules suivantes :

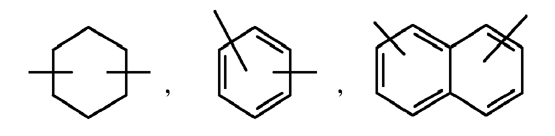

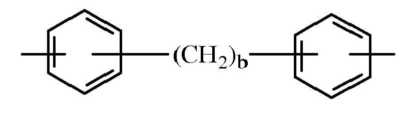

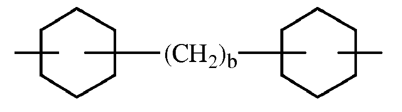

   -(CH₂)_{c}-
dans lesquelles b est un nombre entier compris entre 0 et 3, et c un nombre entier compris entre 1 et 20, de préférence compris entre 2 et 12.

En particulier, le groupe R est choisi parmi les groupes hexaméthylène, 4,4'-biphénylèneméthane, 2,4- et/ou 2,6-tolylène, 1,5-naphtylène, p-phénylène, méthylène-4,4-bis-cyclohexyle et le groupe divalent dérivé de l'isophorone.

Le polyuréthane non associatif utilisé dans la présente invention peut avantageusement comprendre en outre au moins une séquence polysiloxane dont le motif répétitif de base répond par exemple à la formule générale (VII) :

-O-P-O-CO-NH-R-NH-CO- (VII)

dans laquelle :
- P est un segment polysiloxanique, et
- R est un groupe divalent choisi parmi les groupes hydrocarbonés aliphatiques en C1 à C20, cycloaliphatiques en C3 à C20 et aromatiques en C6 à C20, comme par exemple les groupes alkylène en C1 à C20, arylène en C6 à C20, cycloalkylène en C3 à C20, ou leurs combinaisons, ces groupes étant substitués ou non.

Avantageusement, le segment polysiloxanique P répond à la formule générale (VIII) ci-après : 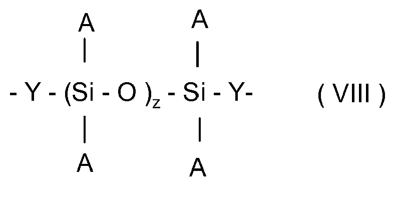 dans laquelle:
- les groupes A, qui peuvent être identiques ou différents, sont choisis parmi d'une part les groupes hydrocarbonés monovalents en C1 à C20 exempts ou substantiellement exempts d'insaturation éthylénique et, d'autre part, les groupes aromatiques,
- Y représente un groupe hydrocarboné divalent, et
- z représente un nombre entier, choisi de telle sorte que la masse moléculaire moyenne en poids du segment polysiloxane soit comprise entre 300 et 10 000.

De préférence, le groupe divalent Y est choisi parmi les groupes alkylène de formule -(CH2)a-, dans laquelle a représente un nombre entier pouvant être compris entre 1 et 10.

Les groupes A peuvent être choisis parmi les groupes alkyle en C1-8, en particulier les groupes méthyle, éthyle, propyle, isopropyle, butyle, pentyle, hexyle, octyle ; les groupes cycloalkyle en C3-8, en particulier le groupe cyclohexyle ; les groupes aryle en C6-10, notamment phényle ; les groupes arylalkyle en C7-10, notamment benzyle et phényléthyle, ainsi que les groupes tolyle et xylyle.

A titre d'exemples de polyuréthane non associatif, on peut notamment citer le copolymère acide diméthylolpropionique/ isophorone-diisocyanate/néopentylglycol/polyesterdiols (connu aussi sous le nom de polyuréthane-1, appellation INCI) vendu sous la marque Luviset® PUR par la société BASF, et le copolymère acide diméthylolpropionique/isophorone-diisocyanate/néopentylglycol /polyesterdiols/diamine siliconée (connu aussi sous le nom de polyuréthane-6, appellation INCI) vendu sous la marque Luviset® Si PUR A par la société BASF.

Ces polyuréthanes contenus dans la composition selon l'invention sont mis en oeuvre sous forme non neutralisée, donc non-ionique.

Par polyuréthane associatif, on entend un polyuréthane possédant au moins une chaîne alkyle terminale ou pendante comportant au moins 10 atomes de carbone. Ce type de polymère est susceptible d'interagir avec lui-même ou avec des composés particuliers tels que des tensioactifs pour conduire à un épaississement du milieu.

A titre d'exemple de polyuréthane associatif non-ionique, on peut notamment citer un copolymère acrylique soluble ou gonflable dans l'eau. Il est caractérisé par le fait qu'il comprend :
a) environ 40 à 99,5% en poids, de préférence 30 à 65% en poids, d'un monomère à insaturation monoéthylénique non tensioactif et
b) environ 0,5 à 60% en poids, de préférence 10 à 50% en poids, d'un monomère uréthane non-ionique qui est le produit de réaction d'un tensioactif non-ionique monohydrique avec un monoisocyanate à insaturation monoéthylénique.

Le copolymère doit contenir une proportion importante indiquée ci-dessus d'un monomère a) à insaturation monoéthylénique qui n'a pas de propriété tensioactive. Les monomères préférés sont ceux qui donnent des polymères insolubles dans l'eau lorsqu'ils sont homopolymérisés et sont illustrés par les acrylates et méthacrylates d'alkyle en C₁-C₄ comme l'acrylate de méthyle, l'acrylate d'éthyle, l'acrylate de butyle ou les méthacrylates correspondants. Les monomères plus particulièrement préférés sont les (méth)acrylates de méthyle et d'éthyle. D'autres monomères pouvant être utilisés sont le styrène, le vinyltoluène, l'acétate de vinyle, l'acrylonitrile et le chlorure de vinylidène. Les monomères non réactifs sont préférés, ces monomères étant ceux dans lesquels le groupe éthylénique unique est le seul groupe réactif dans les conditions de la polymérisation. Cependant, des monomères qui contiennent des groupes réactifs sous l'action de la chaleur peuvent être utilisés dans certaines situations, comme l'acrylate d'hydroxyéthyle.

Les tensioactifs non-ioniques monohydriques utilisés pour obtenir le monomère uréthane non-ionique b) sont bien connus et sont généralement des composés hydrophobes alcoxylés contenant un oxyde d'alkylène formant la partie hydrophile de la molécule. Les composés hydrophobes sont généralement constitués par un alcool aliphatique ou un alkylphénol dans lesquels une chaîne carbonée contenant au moins six atomes de carbone constitue la partie hydrophobe du tensioactif.
Les tensioactifs non-ioniques monohydriques préférés ont pour formule : 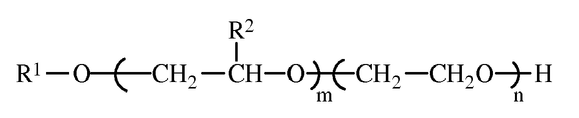 dans laquelle R¹ est un groupe alkyle en C₆-C₃₀ ou aralkyle en C₈-C₃₀, R² est un groupe alkyle en C₁-C₄, n est un nombre moyen allant d'environ 5 à 150 et m est un nombre moyen allant d'environ 0 à 50, à la condition que n soit au moins aussi grand que m et que n + m = 5-150.

A titre de groupes alkyle en C₆-C₃₀ préférés, on peut citer les radicaux dodécyle et alkyle en C₁₈-C₂₆. A titre de groupes aralkyle, on peut citer plus particulièrement les groupes alkyl (C₈-C₁₃) phényle. Le groupe R² préféré est le groupe méthyle.

Le monoisocyanate à insaturation monoéthylénique utilisé pour former le monomère uréthane non-ionique b) peut être choisi parmi des composés très variés. On peut utiliser un composé contenant toute insaturation copolymérisable telle qu'une insaturation acrylique ou méthacrylique. On peut aussi utiliser une insaturation allylique conférée par l'alcool allylique. Les monoisocyanates monoéthyléniques préférés sont l'α,α-diméthyl-m-isopropényl-benzylisocyanate et le méthylstyrène-isopropylisocyanate.

Le copolymère acrylique défini ci-dessus est obtenu par copolymérisation en émulsion aqueuse des composants a) et b) qui est usuelle et décrite dans la demande de brevet EP-A-0 173 109.

Les polyuréthanes associatifs non ioniques utilisés dans la présente invention sont notamment des polyuréthanes-polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

De préférence, les polyuréthane-polyéthers comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyuréthane-polyéthers peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyuréthane-polyéthers non-ioniques à chaîne grasse peuvent être des copolymères triblocs dont la séquence hydrophile est une chaîne polyoxyéthoxylée comportant de 50 à 1000 groupements éthoxylés. Les polyuréthane-polyéthers non-ioniques comportent une liaison uréthanne entre les séquences hydrophiles, d'où l'origine du nom.

Par extension figurent aussi parmi les polyuréthane-polyéthers non-ioniques à chaîne grasse, ceux dont les séquences hydrophiles sont liées aux séquences lipophiles par d'autres liaisons chimiques.

A titre d'exemples de polyuréthane-polyéthers non-ioniques à chaîne grasse utilisables dans l'invention, on peut mentionner aussi le Rhéolate 205 à fonction urée vendu par la société RHEOX ou encore les Rhéolates 208, 204 ou 212,ainsi que l'Acrysol® RM 184.

On peut également citer le produit ELFACOS T210 à chaîne alkyle en C12-14 et le produit ELFACOS® T212 à chaîne alkyle en C18 de chez AKZO.

Le produit DW 1206B de chez ROHM & HAAS à chaîne alkyle en C20 et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Rhéolate® 255, le Rhéolate® 278 et le Rhéolate® 244 vendus par la société RHEOX. On peut aussi utiliser le produit DW 1206F et le DW 1206J proposés par la société ROHM & HAAS.

Les polyuréthane-polyéthers utilisables selon l'invention sont en particulier ceux décrits dans l'article de G. Fonnum, J. Bakke et Fk. Hansen - Colloid Polym. Sci 271, 380.389 (1993).

Comme exemples préférés de polyuréthane associatif non ionique, on peut citer les polyuréthane-polyéthers susceptibles d'être obtenus par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyuréthane-polyéthers sont vendus notamment par la société ROHM & HAAS sous les appellations Aculyn® 46 et Aculyn® 44. L'ACULYN® 46 est un polycondensat de polyéthylèneglycol à 150 ou 180 moles d'oxyde d'éthylène, d'alcool stéarylique et de méthylène-bis(4-cyclohexylisocyanate) (SMDI), à 15% en poids dans une matrice de maltodextrine (4%) et d'eau (81%); l'ACULYN® 44 est un polycondensat de polyéthylèneglycol à 150 ou 180 moles d'oxyde d'éthylène, d'alcool décylique et de méthylène-bis(4-cyclohexylisocyanate) (SMDI), à 35% en poids dans un mélange de propylèneglycol (39%) et d'eau (26%).

Les groupes alkyles des polymères non ioniques mentionnés ci-dessus ont, de préférence, de 1 à 6 atomes de carbone.

Selon un mode de réalisation particulier, le polymère est choisi parmi les polymères anioniques, non ioniques et amphotères, de préférence filmogènes et :ou gélifiants.

Par polymère "filmogène", on entend un polymère apte à former à lui seul ou en présence d'un agent auxiliaire de filmification, un film macroscopiquement continu sur un support, notamment sur les matières kératiniques, et de préférence un film cohésif et mieux encore, un film dont la cohésion et les propriétés mécaniques sont telles que ledit film peut être isolé dudit support.

Les compositions conformes à l'invention peuvent également contenir au moins un agent utilisé habituellement en cosmétique, choisi, par exemple, parmi des agents réducteurs, des corps gras, des plastifiants, des adoucissants, des agents anti-mousse, des agents hydratants, des filtres UV, des colloïdes minéraux, des peptisants, des solubilisants, des parfums, des tensio-actifs anioniques, cationiques, non ioniques ou amphotères, des protéines, des vitamines, des agents nacrants, des propulseurs, et des épaississants minéraux ou organiques tels que le benzylidène-sorbitol et les N-acylaminoacides, les cires oxyéthylénées ou non, les paraffines, les acides gras en C10-C30 tels que l'acide stéarique, l'acide laurique, les amides gras en C10-C30 tel que le diéthanolamide laurique.

Les additifs ci dessus sont en général présents en quantité comprise pour chacun d'eux entre 0,01 et 20 % en poids par rapport au poids de la composition.

Bien entendu, l'homme de l'art veillera à choisir ce ou ces éventuels additifs de manière telle que les propriétés avantageuses attachées intrinsèquement à la formation du gainage conforme à l'invention ne soient pas, ou substantiellement pas, altérées

La composition selon l'invention peut se présenter notamment sous forme de crème, de mousse, de stick, de dispersion de vésicules notamment de lipides ioniques ou non, de lotion biphase ou multiphase, de spray, d'aérosol, de poudre, de pâte.

La composition peut être une composition anhydre, c'est-à-dire une composition contenant moins de 2 % en poids d'eau, voire moins de 0,5 % d'eau, notamment exempte d'eau, l'eau n'étant pas ajoutée lors de la préparation de la composition mais correspondant à l'eau résiduelle apportée par les ingrédients mélangés.

La composition décrite ci-dessus peut être mise en oeuvre sur cheveux secs ou humides. Après application de la composition de l'invention avec ou sans additifs tels que décrits précédemment, les cheveux sont séchés au casque ou au sèche-cheveux. Les additifs présents peuvent être appliqués sur les cheveux simultanément avec la composition de l'invention ou séparément.

Il est possible d'effectuer ensuite un lavage des cheveux, ce lavage n'étant pas obligatoire.

### EXEMPLES

### EXEMPLE 1

La composition suivante est réalisée:

| | |
|---|---|
| Isopropanol | 50g |
| Cyclopentadiméthylsiloxane commercialisé par Dow Corning sous le nom de DC245 Fluid | 30g |
| Nacre mica enrobé d'oxyde de fer brun commercialisée par Eckart sous le nom Prestige Bronze | 10g |
| Copolymère diméthylpolysiloxane/urée commercialisé sous la référence Wacker-Belsil® UD 60 par Wacker | 10g |

0.5g de la composition est appliqué sur une mèche de 1g de cheveux propre et secs. Après 5 minutes de pause, la mèche est séchée au sèche-cheveux pendant 2 minutes. On obtient une mèche colorée dont les cheveux sont individualisés et dont la couleur est rémanente au shampooing.

### EXEMPLE 2

La composition suivante est réalisée :

| | |
|---|---|
| Isopropanol | 45g |
| Cyclopentadiméthylsiloxane commercialisé par Dow Corning sous le nom de DC245 Fluid | 30g |
| Polydiméthylsiloxane commercialisée par Dow Corning sous la référence Dow Corning 200 Fluid 60000 cs | 5g |
| Nacre mica enrobé d'oxyde de fer brun commercialisée par Eckart sous le nom Prestige Bronze | 10g |
| Copolymère diméthylpolysiloxane/urée commercialisé sous la référence Wacker-Belsil® UD 60 par Wacker | 10g |

0.5g de la composition est appliqué sur une mèche de 1g de cheveux propre et secs. Après 5 minutes de pause, la mèche est séchée au sèche-cheveux pendant 2 minutes. On obtient une mèche colorée dont les cheveux sont individualisés et dont la couleur est rémanente au shampooing.

### EXEMPLE 3

La composition suivante est réalisée :

| | |
|---|---|
| Isopropanol | 50g |
| Cyclopentadiméthylsiloxane commercialisé par Dow Corning sous le nom de DC245 Fluid | 39g |
| Disperse Red 13 | 1g |
| Copolymère diméthylpolysiloxane/urée commercialisé sous la référence Wacker-Belsil® UD 60 par Wacker | 10g |

0.3g de la composition est appliqué sur une mèche de 1 g de cheveux propres et secs. Après 5 minutes de pause, la mèche est séchée au sèche-cheveux pendant 2 minutes. On obtient une mèche colorée dont les cheveux sont individualisés et dont la couleur est rémanente au shampooing.

### EXEMPLE 4

La composition suivante est réalisée :

| | |
|---|---|
| Isopropanol | 45g |
| Cyclopentadiméthylsiloxane commercialisé par Dow Corning sous le nom de DC245 Fluid | 40g |
| 3,6-bis(bis[biphenyl]methyl)amino)-2,5-pyrazine dicarbonitrile * | 5g |
| Copolymère diméthylpolysiloxane/urée commercialisé sous la référence Wacker-Belsil® UD 60 par Wacker | 10g |

0.3g de la composition est appliqué sur une mèche de 1 g de cheveux propres et secs. Après 5 minutes de pause, la mèche est séchée au sèche-cheveux pendant 2 minutes. On obtient une mèche colorée dont les cheveux sont individualisés et dont la couleur est rémanente au shampooing.
* cité dans les publications suivantes : Natu R.Patel, Journal of Heterocyclic Chemistry, Vol3, N°4, 1966, pp512-517 ; Kazuko Shirai, Journal of the Society of Dyes and Colorisis, Vol114, n°12, Dec 1998, pp368-374.

### EXEMPLE 5

La composition suivante est réalisée :

| | |
|---|---|
| Isopropanol | 50g |
| Cyclopentadiméthylsiloxane commercialisé par Dow Corning sous le nom de DC245 Fluid | 39g |
| 5,6-dihydroxyindole | 1g |
| Copolymère diméthylpolysiloxane/urée commercialisé sous la référence Wacker-Belsil® UD 60 par Wacker | 10g |

0.3g de la composition est appliqué sur une mèche de 1 g de cheveux propres et secs. Après 5 minutes de pause, la mèche est séchée au sèche-cheveux pendant 2 minutes. On obtient une mèche colorée dont les cheveux sont individualisés et dont la couleur est rémanente au shampooing.

### EXEMPLE 6

La composition suivante est réalisée :

| | |
|---|---|
| Isopropanol | 45g |
| Cyclopentadiméthylsiloxane commercialisé par Dow Corning sous le nom de DC245 Fluid | 30g |
| Polydiméthylsiloxane commercialisée par Dow Corning sous la référence Dow Corning 200 Fluid 60000 cs | 5g |
| Nacre mica enrobé d'oxyde de fer brun commercialisée par Eckart sous le nom Prestige Bronze | 10g |
| Copolymère diméthylpolysiloxane/urée commercialisé sous la référence Wacker-Belsil® UD 140 par Wacker | 10g |

0.5g de la composition est appliqué sur une mèche de 1g de cheveux propre et secs. Après 5 minutes de pause, la mèche est séchée au sèche-cheveux pendant 2 minutes. On obtient une mèche colorée dont les cheveux sont individualisés et dont la couleur est rémanente au shampooing.

## Revendications

1. Composition cosmétique comprenant au moins un copolymère non ionique bloc polysiloxane/polyurée, au moins une espèce coloré ou colorante, au moins un solvant organique non siliconé volatil.

2. Composition selon la revendication 1 comprenant au moins un composé siliconé présentant une viscosité inférieure à 100 mm²/s (100 cst) à 25°C..

3. Composition selon l'une quelconque des revendications 1 à 2 dans laquelle le copolymère contient au moins un bloc polyuréthane.

4. Composition selon l'une quelconque des revendications 1 à 2 dans laquelle le copolymère contient uniquement des blocs polysiloxane et des blocs polyurée.

5. Composition selon l'une quelconque des revendications précédentes dans laquelle le bloc polysiloxane du copolymère contient une quantité en poids de polysiloxane supérieure à 5 %.

6. Composition selon la revendication 5 dans laquelle la quantité de polysiloxane dans le copolymère est majoritaire dans le copolymère, de préférence supérieure à 90 % en poids par rapport au poids total du copolymère.

7. Composition selon l'une quelconque des revendications précédentes dans laquelle le copolymère polysiloxane/polyurée correspond à la formule générale (1) : 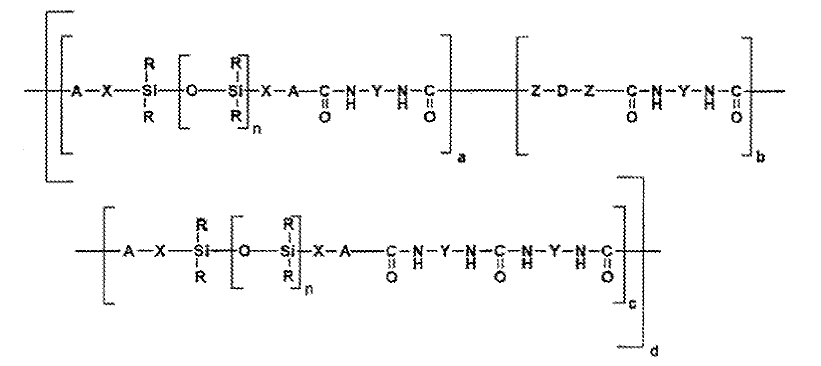 dans laquelle
R représente un radical hydrocarboné monovalent de 1 à 20 atomes de carbone, pouvant être substitué par un ou plusieurs atomes de fluor ou de chlore,
X représente un radical alkylène ayant 1 à 20 atomes de carbone, dans lequel des unités méthylène non voisines peuvent être remplacées par des radicaux -O-,
A représente un atome d'oxygène ou un radical amino -NR'-,
Z représente un atome d'oxygène ou un radical amino -NR'-,
R' représente hydrogène ou un radical alkyle ayant 1 à 10 atomes de carbone,
Y représente un radical hydrocarboné bivalent, le cas échéant substitué par le fluor ou le chlore, ayant 1 à 20 atomes de carbone,
D représente un radical alkylène, le cas échéant substitué par fluor, chlore, alkyle en C₁-C₆ ou ester alkylique en C₁-C₆, ayant 1 à 700 atomes de carbone, dans lequel des unités méthylène non voisines peuvent être remplacées par des radicaux -O-, -COO-, -OCO- ou -OCOO-,
n est un nombre allant de 1 à 4000,
a est un nombre d'au moins 1,
b est un nombre allant de 0 à 40,
c est un nombre allant de 0 à 30, et
d est un nombre supérieur à 0.
à la condition que A dans au moins un des motifs (a) représente un radical -NH-.

8. Composition selon la revendication 7, **caractérisée en ce que**, dans la formule (I), R est méthyle.

9. Composition selon la revendication 7 ou 8 dans laquelle dans la formule (I), X est propylène.

10. Composition selon l'une quelconque des revendications 7 à 9 dans laquelle dans la formule (1), Z est un radical amino ou un atome d'oxygène.

11. Composition selon l'une quelconque des revendications 7 à 10 dans laquelle dans la formule (I), Y est un radical aralkylène ou un radical alkylène linéaire ou cyclique.

12. Composition selon l'une quelconque des revendications 7 à 11 dans laquelle dans la formule (1), A représente un radical-NH- dans tous les motifs (a).

13. Composition selon la revendication 12 dans laquelle A représente NH dans tous les motifs (a), (b) et (c).

14. Composition selon l'une quelconque des revendications précédentes dans laquelle le copolymère est susceptible d'être obtenu par un procédé, qui comprend les deux étapes suivantes :
- dans la première étape, on fait réagir un silazane cyclique de la formule générale (2) : 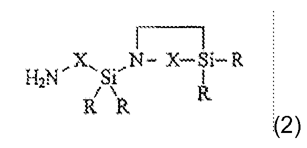 ou
un silazane de formule (2') 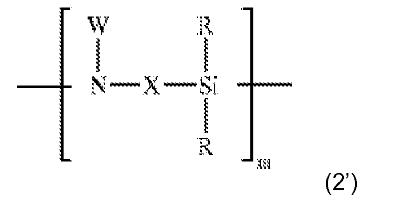 W représentant un atome d'hydrogène, n radical hydrocarboné substitué ou non, comprenant de préférence de 1 à 20 atomes de carbone ou un radical R₂Si-X-NH₂ ;
avec un composé organique du silicium de la formule générale (3) :
(HO)(R₂SiO)ₙ₋₁[H] (3)
en un aminoalkylpolydiorganosiloxane de la formule générale (4)
H₂N-X-[SiR₂O]ₙSiR₂-X-NH₂ (4)
- et dans une deuxième étape, l'aminoalkyl-polydiorganosiloxane de la formule générale (4) est polymérisé avec un diisocyanate de la formule générale (5):
OCN-Y-NCO (5)
X, Y, R et n étant définis dans la revendication 7.

15. Composition selon l'une quelconque des revendications précédentes dans laquelle le copolymère répond au nom INCI polyureadimethicone.

16. Composition selon l'une quelconque des revendications précédentes dans laquelle le solvant organique non siliconé volatil est choisi parmi l'éthanol, l'isopropanol, l'acétone, l'isododécane.

17. Composition selon l'une des revendications 2 à 16 dans laquelle le composé siliconé présentant une viscosité inférieure à 100 cst est choisi parmi l'octaméthylcyclotétrasiloxane, le décaméthylcyclopentasiloxane, le dodécaméthylcyclohexasiloxane, l'heptaméthylhexyltri-siloxane, l'heptaméthyléthyltrisiloxane, l'heptaméthyloctyltrisiloxane, l'octaméthyltrisiloxane, le décaméthyltétrasiloxane, et leurs mélanges.

18. Composition selon l'une quelconque des revendications précédentes dans laquelle l'espèce coloré ou colorante est choisie parmi des pigments colorés, des précurseurs de colorants ou des colorants directs hydrophiles ou hydrophobes.

19. Composition selon l'une quelconque des revendications précédentes dans laquelle l'espèce coloré est choisi parmi les pigments organiques, les pigments minéraux, les nacres.

20. Composition selon l'une des revendications précédentes **caractérisée en ce qu'**elle comprend en outre un polysiloxane présentant une viscosité supérieure à 100 cst.

21. Composition selon l'une des revendications précédentes, **caractérisée en ce qu'**elle comprend en outre une charge.

22. Composition selon l'une quelconque des revendications comprenant de plus un polymère non siliconé.

23. Composition selon l'une quelconque des revendications 12 à 18 dans laquelle le polymère non siliconé est un polymère anionique ou non ionique ou amphotère.

24. Procédé de coloration des fibres kératiniques qui comprend l'application sur les cheveux d'une composition telle que défini à l'une quelconque des revendications 1 à 23.
